# EUROPEAN PATENT APPLICATION

(11) **EP 4 039 702 A1**
(43) Date of publication of application: **10.08.2022**
(21) Application number: 20865483.0
(22) Date of filing: 15.09.2020
(51) Int. Cl.: C07K 16/28, C12N 15/13, C12N 5/10, A61K 39/395, A61P 35/00

(54) **ANTI-B7-H3 ANTIBODY AND APPLICATION THEREOF**

(30) Priority: 16.09.2019 CN 201910872843
(71) Applicant: Nanjing Sanhome Pharmaceutical Co., Ltd., Nanjing Jiangsu 210038 (CN)
(72) Inventor: WANG, Yong, Nanjing, Jiangsu 210038 (CN); ZHAO, Liwen, Nanjing, Jiangsu 210038 (CN); XIAO, Yang, Nanjing, Jiangsu 210038 (CN); XU, Yao, Nanjing, Jiangsu 210038 (CN); LUO, Cheng, Nanjing, Jiangsu 210038 (CN); YANG, Yang, Nanjing, Jiangsu 210038 (CN); WEI, Aiqi, Nanjing, Jiangsu 210038 (CN)
(74) Representative: ABG Intellectual Property Law, S.L.
(86) International application number: PCT/CN2020/115262
(87) International publication number: WO 2021/052307

(57) **Abstract**

Provided is an anti-B7-H3 antibody or antigen-binding fragment thereof, a pharmaceutical composition containing the anti-B7-H3 antibody or antigen-binding fragment thereof, and an application thereof. The anti-B7-H3 antibody or antigen-binding fragment thereof of the present invention has high affinity and stability, and can be used for preparing antitumor drugs.

## Description

### FIELD

The present disclosure relates to the technical field of antibody drugs, and in particular, to an anti-B7-H3 antibody or an antigen-binding fragment thereof, a pharmaceutical composition comprising the anti-B7-H3 antibody or the antigen-binding fragment thereof, and uses thereof.

### BACKGROUND

B7-H3 (CD276), a member of the B7 superfamily, is a transmembrane glycoprotein, and its extracellular domain has two types of structure, one is monovalent 2Ig-B7-H3, the other is bivalent 4Ig-B7-H3 composed of two repeating units. As a novel immune checkpoint, B7-H3 has been reported in the literature to inhibit T cell proliferation and cytokine release (Suh W K, et al. The B7 family member B7-H3 preferentially down-regulates T helper type 1-mediated immune responses[J]. Nature Immunology, 2003, 4(9):899) and inhibit the lytic activity of NK cells against patient-derived tumor cells with high B7-H3 expression (Castriconi R, et al. Identification of 4Ig-B7-H3 as a neuroblastoma-associated molecule that exerts a protective role from an NK cell-mediated lysis[J]. Proceedings of the National Academy of Sciences, 2004, 101(34): 12640-12645) by interacting with a receptor with unknown structure. Although the B7-H3 receptor is unknown, an increasing number of reports have been published in recent years on the negative regulation by the interaction of B7-H3 with its receptor in tumor immunity. Tumor cells may express B7-H3 to circumvent the immune surveillance of CD8+ T cells. In mouse models, the use of anti-mouse B7-H3 antibodies can enhance the immune function of CD8+ T cells (Yonesaka K, et al. B7-H3 negatively modulates CTL-mediated cancer immunity [J]. Clinical Cancer Research, 2018, clincanres.2852.2017). In the study of Lee Y, *et al.,* gene knockout of mouse B7-H3 or the use of anti-mouse B7-H3 antibody can significantly inhibit tumor growth, and this inhibition depends on the function of CD8+ T and NK cells (Lee Y, et al. Inhibition of the B7-H3 immune checkpoint limits tumor growth by enhancing cytotoxic lymphocyte function[J]. Cell Research, 2017, 27(8): 1034-1045). Studies in patients with lung cancer have found that patients with high B7-H3 expression have fewer tumor-infiltrating lymphocytes and are more susceptible to lymph node metastasis (Altan M, et al. B7-H3 expression in NSCLC and its association with B7-H4, PD-L1 and Tumor Infiltrating Lymphocytes[J]. Clinical Cancer Research, 2017, 23(17):5202).

B7-H3 is abnormally expressed in various tumor cells, tumor stroma, tumor blood vessels, and tumor-infiltrating macrophages and DC cells. In tissue samples from patients with prostate cancer, pancreatic cancer, hepatocellular carcinoma, head and neck cancer, kidney cancer, etc., the expression rate of B7-H3 exceeds 90%, while it is rarely expressed in normal tissues (Seaman S, et al. Eradication of Tumors through Simultaneous Ablation of CD276/B7-H3-Positive Tumor Cells and Tumor Vasculature.[J]. Cancer Cell, 2017, 31(4):501-515). Based on the tumor-specific expression trend of B7-H3, the antibody drugs against B7-H3 target under clinical research are all developed with the help of the tumor targeting of B7-H3. Enoblituzumab, a humanized anti-B7-H3 monoclonal antibody, kills tumors by targeting the tumor-mediated ADCC effect of the Fc region of the antibody. Burtomab is a murine-derived anti-B7-H3 antibody-drug conjugate carrying radioactive iodine, and is used in the clinical treatment of metastatic neuroblastoma, which significantly improves the survival of children with cancer.

Based on the high expression characteristics of B7-H3 in the tumor environment and its inhibitory effect on immune cells, B7-H3 may be an effective target for the development of anti-tumor drugs. It is necessary to provide an anti-B7-H3 antibody or an antigen-binding fragment thereof with higher affinity and stability, which can block the immunosuppression of B7-H3 on tumors, so that enhance the killing activity of CD8+ T cells and NK cells on tumors, and synergize with the ADCC effect of the Fc fragment of the antibody to play an anti-tumor effect, giving more excellent anti-tumor activity.

### SUMMARY

One aspect of the present disclosure provides an anti-B7-H3 antibody or antigen-binding fragment thereof, comprising a heavy chain variable region and/or a light chain variable region, wherein the heavy chain variable region comprises complementarity determining region 1 of the heavy chain variable region (HCDR1), complementarity determining region 2 of the heavy chain variable region (HCDR2), and/or complementarity determining region 3 of the heavy chain variable region (HCDR3); and the light chain variable region comprises complementarity determining region 1 of the light chain variable region (LCDR1), complementarity determining region 2 of the light chain variable region (LCDR2), and/or complementarity determining region 3 of the light chain variable region (LCDR3).

In some embodiments, the present disclosure provides an anti-B7-H3 antibody or antigen-binding fragment thereof, comprising a heavy chain variable region and a light chain variable region, wherein
(1) the heavy chain variable region comprises HCDR1, HCDR2 and HCDR3, selected from the group consisting of:
   (a1) amino acid sequences as set forth in SEQ ID NOs: 1, 2 and 3,
   (a2) amino acid sequences as set forth in SEQ ID NOs: 7, 8 and 9,
   (a3) amino acid sequences as set forth in SEQ ID NOs: 13, 14 and 15,
   (a4) amino acid sequences as set forth in SEQ ID NOs: 20, 21 and 22, and
   (a5) CDRs having at least 85% sequence identity to the amino acid sequences set forth in (a1), (a2), (a3), or (a4); and
(2) the light chain variable region comprises LCDR1, LCDR2 and LCDR3, selected from the group consisting of:
   (a6) amino acid sequences as set forth in SEQ ID NOs: 4, 5 and 6,
   (a7) amino acid sequences as set forth in SEQ ID NOs: 10, 11 and 12,
   (a8) amino acid sequences as set forth in SEQ ID NOs: 16, 18 and 19,
   (a9) amino acid sequences as set forth in SEQ ID NOs: 17, 18 and 19,
   (a10) amino acid sequences as set forth in SEQ ID NOs: 23, 24 and 25, and
   (a11) CDRs having at least 85% sequence identity to the amino acid sequences set forth in (a6), (a7), (a8), (a9), or (a10).

In a specific embodiment, the present disclosure provides an anti-B7-H3 antibody or antigen-binding fragment thereof, comprising a heavy chain variable region and a light chain variable region, wherein the heavy chain variable region comprises HCDR1, HCDR2, and HCDR3 set forth in SEQ ID NOs: 1, 2 and 3 respectively, or the CDRs having at least 85% sequence identity to the amino acid sequences set forth in SEQ ID NOs: 1, 2 and 3; and the light chain variable region comprises LCDR1, LCDR2 and LCDR3 set forth in SEQ ID NOs: 4, 5 and 6 respectively, or the CDRs having at least 85% sequence identity to the amino acid sequences set forth in SEQ ID NOs: 4, 5 and 6.

In a specific embodiment, the present disclosure provides an anti-B7-H3 antibody or antigen-binding fragment thereof, comprising a heavy chain variable region and a light chain variable region, wherein the heavy chain variable region comprises HCDR1, HCDR2, and HCDR3 set forth in SEQ ID NOs: 7, 8 and 9 respectively, or the CDRs having at least 85% sequence identity to the amino acid sequences set forth in SEQ ID NOs: 7, 8 and 9; and the light chain variable region comprises LCDR1, LCDR2 and LCDR3 set forth in SEQ ID NOs: 10, 11 and 12 respectively, or the CDRs having at least 85% sequence identity to the amino acid sequences set forth in SEQ ID NOs: 10, 11 and 12.

In a specific embodiment, the present disclosure provides an anti-B7-H3 antibody or antigen-binding fragment thereof, comprising a heavy chain variable region and a light chain variable region, wherein the heavy chain variable region comprises HCDR1, HCDR2, and HCDR3 set forth in SEQ ID NOs: 13, 14 and 15 respectively, or the CDRs having at least 85% sequence identity to the amino acid sequences set forth in SEQ ID NOs: 13, 14 and 15; and the light chain variable region comprises LCDR1, LCDR2 and LCDR3 set forth in SEQ ID NOs: 16, 18 and 19 respectively, or the CDRs having at least 85% sequence identity to the amino acid sequences set forth in SEQ ID NOs: 16, 18 and 19.

In a specific embodiment, the present disclosure provides an anti-B7-H3 antibody or antigen-binding fragment thereof, comprising a heavy chain variable region and a light chain variable region, wherein the heavy chain variable region comprises HCDR1, HCDR2, and HCDR3 set forth in SEQ ID NOs: 13, 14 and 15 respectively, or the CDRs having at least 85% sequence identity to the amino acid sequences set forth in SEQ ID NOs: 13, 14 and 15; and the light chain variable region comprises LCDR1, LCDR2 and LCDR3 set forth in SEQ ID NOs: 17, 18 and 19 respectively, or the CDRs having at least 85% sequence identity to the amino acid sequences set forth in SEQ ID NOs: 17, 18 and 19.

In a specific embodiment, the present disclosure provides an anti-B7-H3 antibody or antigen-binding fragment thereof, comprising a heavy chain variable region and a light chain variable region, wherein the heavy chain variable region comprises HCDR1, HCDR2, and HCDR3 set forth in SEQ ID NOs: 20, 21 and 22 respectively, or the CDRs having at least 85% sequence identity to the amino acid sequences set forth in SEQ ID NOs: 20, 21 and 22; and the light chain variable region comprises LCDR1, LCDR2 and LCDR3 set forth in SEQ ID NOs: 23, 24 and 25 respectively, or the CDRs having at least 85% sequence identity to the amino acid sequences set forth in SEQ ID NOs: 23, 24 and 25.

In some specific embodiments, the anti-B7-H3 antibody or antigen-binding fragment thereof according to the present disclosure is a monoclonal antibody or antigen-binding fragment thereof.

In some specific embodiments, the anti-B7-H3 antibody or antigen-binding fragment thereof according to the present disclosure is a murine-derived antibody or antigen-binding fragment thereof, a chimeric antibody or antigen-binding fragment thereof, or a humanized antibody or antigen-binding fragment thereof.

In some specific embodiments, the present disclosure provides an anti-B7-H3 antibody or antigen-binding fragment thereof, comprising a heavy chain variable region and a light chain variable region, wherein
(1) the amino acid sequence of the heavy chain variable region is selected from the group consisting of:
   (b1) amino acid sequences as set forth in SEQ ID NO: 26, SEQ ID NO: 28, SEQ ID NO: 30 and SEQ ID NO: 35,
   (b2) amino acid sequences derived from the amino acid sequences set forth in (b1) by substitution, deletion or addition of one or more amino acids and functionally identical or similar to the amino acid sequences set forth in (b1), and
   (b3) amino acid sequences having at least 80% sequence identity to the amino acid sequences set forth in (b1); and
(2) the amino acid sequence of the light chain variable region is selected from the group consisting of:
   (b4) amino acid sequences as set forth in SEQ ID NO: 27, SEQ ID NO: 29, SEQ ID NO: 31, SEQ ID NO: 32 and SEQ ID NO: 36,
   (b5) amino acid sequences derived from the amino acid sequences set forth in (b4) by substitution, deletion or addition of one or more amino acids and functionally identical or similar to the amino acid sequences set forth in (b4), and
   (b6) amino acid sequences having at least 80% sequence identity to the amino acid sequences set forth in (b4).

In some specific embodiments, the present disclosure provides an anti-B7-H3 antibody or antigen-binding fragment thereof, wherein the amino acid sequence of the heavy chain variable region is selected from the group consisting of SEQ ID NO: 26, the amino acid sequence derived from SEQ ID NO: 26 by substitution, deletion or addition of one or more amino acids and functionally identical to SEQ ID NO: 26, and the amino acid sequence having at least 85% sequence identity to SEQ ID NO: 26; and the amino acid sequence of the light chain variable region is selected from the group consisting of SEQ ID NO: 27, the amino acid sequence derived from SEQ ID NO: 27 by substitution, deletion or addition of one or more amino acids and functionally identical to SEQ ID NO: 27, and the amino acid sequence having at least 85% sequence identity to SEQ ID NO: 27.

In some specific embodiments, the present disclosure provides an anti-B7-H3 antibody or antigen-binding fragment thereof, wherein the amino acid sequence of the heavy chain variable region is selected from the group consisting of SEQ ID NO: 28, the amino acid sequence derived from SEQ ID NO: 28 by substitution, deletion or addition of one or more amino acids and functionally identical to SEQ ID NO: 28, and the amino acid sequence having at least 85% sequence identity to SEQ ID NO: 28; and the amino acid sequence of the light chain variable region is selected from the group consisting of SEQ ID NO: 29, the amino acid sequence derived from SEQ ID NO: 29 by substitution, deletion or addition of one or more amino acids and functionally identical to SEQ ID NO: 29, and the amino acid sequence having at least 85% sequence identity to SEQ ID NO: 29.

In some specific embodiments, the present disclosure provides an anti-B7-H3 antibody or antigen-binding fragment thereof, wherein the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30; and the amino acid sequence of the light chain variable region is selected from the group consisting of SEQ ID NO: 31, the amino acid sequence derived from SEQ ID NO: 31 by substitution, deletion or addition of one or more amino acids and functionally identical to SEQ ID NO: 31, and the amino acid sequence having at least 85% sequence identity to SEQ ID NO: 31.

In some specific embodiments, the present disclosure provides an anti-B7-H3 antibody or antigen-binding fragment thereof, wherein the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30; and the amino acid sequence of the light chain variable region is selected from the group consisting of SEQ ID NO: 32, the amino acid sequence derived from SEQ ID NO: 32 by substitution, deletion or addition of one or more amino acids and functionally identical to SEQ ID NO: 32, and the amino acid sequence having at least 85% sequence identity to SEQ ID NO: 32.

In some specific embodiments, the present disclosure provides an anti-B7-H3 antibody or antigen-binding fragment thereof, wherein the amino acid sequence of the heavy chain variable region is selected from the group consisting of SEQ ID NO: 35, the amino acid sequence derived from SEQ ID NO: 35 by substitution, deletion or addition of one or more amino acids and functionally identical to SEQ ID NO: 35, and the amino acid sequence having at least 85% sequence identity to SEQ ID NO: 35; and the amino acid sequence of the light chain variable region is selected from the group consisting of SEQ ID NO: 36, the amino acid sequence derived from SEQ ID NO: 36 by substitution, deletion or addition of one or more amino acids and functionally identical to SEQ ID NO: 36, and the amino acid sequence having at least 85% sequence identity to SEQ ID NO: 36.

In some specific embodiments, the present disclosure provides an anti-B7-H3 antibody or antigen-binding fragment thereof, wherein the amino acid sequence of the heavy chain variable region is selected from the group consisting of SEQ ID NO: 26, the amino acid sequence derived from SEQ ID NO: 26 by substitution, deletion or addition of one or more amino acids and functionally identical to SEQ ID NO: 26, and the amino acid sequence having at least 85% sequence identity to SEQ ID NO: 26, and comprises HCDR1, HCDR2, and HCDR3 set forth in SEQ ID NOs: 1, 2 and 3; and the amino acid sequence of the light chain variable region is selected from the group consisting of SEQ ID NO: 27, the amino acid sequence derived from SEQ ID NO: 27 by substitution, deletion or addition of one or more amino acids and functionally identical to SEQ ID NO: 27, and the amino acid sequence having at least 85% sequence identity to SEQ ID NO: 27, and comprises LCDR1, LCDR2 and LCDR3 set forth in SEQ ID NOs: 4, 5 and 6.

In some specific embodiments, the present disclosure provides an anti-B7-H3 antibody or antigen-binding fragment thereof, wherein the amino acid sequence of the heavy chain variable region is selected from the group consisting of SEQ ID NO: 28, the amino acid sequence derived from SEQ ID NO: 28 by substitution, deletion or addition of one or more amino acids and functionally identical to SEQ ID NO: 28, and the amino acid sequence having at least 85% sequence identity to SEQ ID NO: 28, and comprises HCDR1, HCDR2, and HCDR3 set forth in SEQ ID NOs: 7, 8 and 9; and the amino acid sequence of the light chain variable region is selected from the group consisting of SEQ ID NO: 29, the amino acid sequence derived from SEQ ID NO: 29 by substitution, deletion or addition of one or more amino acids and functionally identical to SEQ ID NO: 29, and the amino acid sequence having at least 85% sequence identity to SEQ ID NO: 29, and comprises LCDR1, LCDR2 and LCDR3 set forth in SEQ ID NOs: 10, 11 and 12.

In some specific embodiments, the present disclosure provides an anti-B7-H3 antibody or antigen-binding fragment thereof, wherein the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30, and comprises HCDR1, HCDR2, and HCDR3 set forth in SEQ ID NOs: 13, 14 and 15; and the amino acid sequence of the light chain variable region is selected from the group consisting of SEQ ID NO: 31, the amino acid sequence derived from SEQ ID NO: 31 by substitution, deletion or addition of one or more amino acids and functionally identical to SEQ ID NO: 31, and the amino acid sequence having at least 85% sequence identity to SEQ ID NO: 31, and comprises LCDR1, LCDR2 and LCDR3 set forth in SEQ ID NOs: 16, 18 and 19.

In some specific embodiments, the present disclosure provides an anti-B7-H3 antibody or antigen-binding fragment thereof, wherein the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30, and comprises HCDR1, HCDR2, and HCDR3 set forth in SEQ ID NOs: 13, 14 and 15; and the amino acid sequence of the light chain variable region is selected from the group consisting of SEQ ID NO: 32, the amino acid sequence derived from SEQ ID NO: 32 by substitution, deletion or addition of one or more amino acids and functionally identical to SEQ ID NO: 32, and the amino acid sequence having at least 85% sequence identity to SEQ ID NO: 32, and comprises LCDR1, LCDR2 and LCDR3 set forth in SEQ ID NOs: 17, 18 and 19.

In some specific embodiments, the present disclosure provides an anti-B7-H3 antibody or antigen-binding fragment thereof, wherein the amino acid sequence of the heavy chain variable region is selected from the group consisting of SEQ ID NO: 35, the amino acid sequence derived from SEQ ID NO: 35 by substitution, deletion or addition of one or more amino acids and functionally identical to SEQ ID NO: 35, and the amino acid sequence having at least 85% sequence identity to SEQ ID NO: 35, and comprises HCDR1, HCDR2, and HCDR3 set forth in SEQ ID NOs: 20, 21 and 22; and the amino acid sequence of the light chain variable region is selected from the group consisting of SEQ ID NO: 36, the amino acid sequence derived from SEQ ID NO: 36 by substitution, deletion or addition of one or more amino acids and functionally identical to SEQ ID NO: 36, and the amino acid sequence having at least 85% sequence identity to SEQ ID NO: 36, and comprises LCDR1, LCDR2 and LCDR3 set forth in SEQ ID NOs: 23, 24 and 25.

In some specific embodiments, the anti-B7-H3 antibody according to the present disclosure is a murine-derived antibody, which further comprises a heavy chain constant region of murine-derived IgG1, IgG2, IgG3, IgG4, or a variant thereof, and a light chain constant region of murine-derived kappa chain or a variant thereof.

In some preferred embodiments, the anti-B7-H3 murine-derived antibody according to the present disclosure further comprises a heavy chain constant region of murine-derived IgG1, IgG2, or a variant thereof, and a light chain constant region of murine-derived kappa chain or a variant thereof.

In some embodiments, the present disclosure provides an anti-B7-H3 antibody or antigen-binding fragment thereof, wherein the anti-B7-H3 antibody is a humanized antibody, wherein
(1) the amino acid sequence of the heavy chain variable region is selected from the group consisting of:
   (c1) amino acid sequences as set forth in SEQ ID NO: 45, SEQ ID NO: 46, SEQ ID NO: 47, SEQ ID NO: 48, SEQ ID NO: 52, SEQ ID NO: 53, SEQ ID NO: 54, SEQ ID NO: 55, SEQ ID NO: 56, SEQ ID NO: 57, SEQ ID NO: 63, SEQ ID NO: 64, SEQ ID NO: 72 and SEQ ID NO: 73,
   (c2) amino acid sequences derived from the amino acid sequences set forth in (c1) by substitution, deletion or addition of one or more amino acids and functionally identical or similar to the amino acid sequences as set forth in (c1), and
   (c3) amino acid sequences having at least 80% sequence identity to the amino acid sequences set forth in (c1); and
(2) the amino acid sequence of the light chain variable region is selected from the group consisting of:
   (c4) amino acid sequences as set forth in SEQ ID NO: 49, SEQ ID NO: 50, SEQ ID NO: 51, SEQ ID NO: 58, SEQ ID NO: 59, SEQ ID NO: 60, SEQ ID NO: 61, SEQ ID NO: 62, SEQ ID NO: 65, SEQ ID NO: 66, SEQ ID NO: 67, SEQ ID NO: 68, SEQ ID NO: 69, SEQ ID NO: 70, SEQ ID NO: 74, SEQ ID NO: 75, SEQ ID NO: 76, SEQ ID NO: 77, SEQ ID NO: 78 and SEQ ID NO: 79,
   (c5) amino acid sequences derived from the amino acid sequences set forth in (c4) by substitution, deletion or addition of one or more amino acids and functionally identical or similar to the amino acid sequences as set forth in (c4), and
   (c6) amino acid sequences having at least 80% sequence identity to the amino acid sequences set forth in (c4).

In some specific embodiments, the present disclosure provides an anti-B7-H3 antibody or antigen-binding fragment thereof, wherein the anti-B7-H3 antibody is a humanized antibody, wherein
(1) the amino acid sequence of the heavy chain variable region is selected from the group consisting of:
   (c1) amino acid sequences as set forth in SEQ ID NO: 45, SEQ ID NO: 46, SEQ ID NO: 47 and SEQ ID NO: 48,
   (c2) amino acid sequences derived from the amino acid sequences set forth in (c1) by substitution, deletion or addition of one or more amino acids and functionally identical or similar to the amino acid sequences as set forth in (c1), and
   (c3) amino acid sequences having at least 85% sequence identity to the amino acid sequences set forth in (c1) and comprising HCDR1, HCDR2 and HCDR3 set forth in SEQ ID NOs: 1, 2 and 3; and
(2) the amino acid sequence of the light chain variable region is selected from the group consisting of:
   (c4) amino acid sequences as set forth in SEQ ID NO: 49, SEQ ID NO: 50 and SEQ ID NO: 51,
   (c5) amino acid sequences derived from the amino acid sequences set forth in (c4) by substitution, deletion or addition of one or more amino acids and functionally identical or similar to the amino acid sequences as set forth in (c4), and
   (c6) amino acid sequences having at least 85% sequence identity to the amino acid sequences set forth in (c4) and comprising LCDR1, LCDR2 and LCDR3 set forth in SEQ ID NOs: 4, 5 and 6.

In some specific embodiments, the present disclosure provides an anti-B7-H3 antibody or antigen-binding fragment thereof, wherein the anti-B7-H3 antibody is a humanized antibody, wherein
(1) the amino acid sequence of the heavy chain variable region is selected from the group consisting of:
   (c1) amino acid sequences as set forth in SEQ ID NO: 52, SEQ ID NO: 53, SEQ ID NO: 54, SEQ ID NO: 55, SEQ ID NO: 56 and SEQ ID NO: 57,
   (c2) amino acid sequences derived from the amino acid sequences set forth in (c1) by substitution, deletion or addition of one or more amino acids and functionally identical or similar to the amino acid sequences as set forth in (c1), and
   (c3) amino acid sequences having at least 85% sequence identity to the amino acid sequences set forth in (c1) and comprising HCDR1, HCDR2 and HCDR3 set forth in SEQ ID NOs: 7, 8 and 9; and
(2) the amino acid sequence of the light chain variable region is selected from the group consisting of:
   (c4) amino acid sequences as set forth in SEQ ID NO: 58, SEQ ID NO: 59, SEQ ID NO: 60, SEQ ID NO: 61 and SEQ ID NO: 62,
   (c5) amino acid sequences derived from the amino acid sequences set forth in (c4) by substitution, deletion or addition of one or more amino acids and functionally identical or similar to the amino acid sequences as set forth in (c4), and
   (c6) amino acid sequences having at least 85% sequence identity to the amino acid sequences set forth in (c4) and comprising LCDR1, LCDR2 and LCDR3 set forth in SEQ ID NOs: 10, 11 and 12.

In some specific embodiments, the present disclosure provides an anti-B7-H3 antibody or antigen-binding fragment thereof, wherein the anti-B7-H3 antibody is a humanized antibody, wherein
(1) the amino acid sequence of the heavy chain variable region is selected from the group consisting of:
   (c1) amino acid sequences as set forth in SEQ ID NO: 63 and SEQ ID NO: 64,
   (c2) amino acid sequences derived from the amino acid sequences set forth in (c1) by substitution, deletion or addition of one or more amino acids and functionally identical or similar to the amino acid sequences as set forth in (c1), and
   (c3) amino acid sequences having at least 85% sequence identity to the amino acid sequences set forth in (c1) and comprising HCDR1, HCDR2 and HCDR3 set forth in SEQ ID NOs: 13, 14 and 15; and
(2) the amino acid sequence of the light chain variable region is selected from the group consisting of:
   (c4) amino acid sequences as set forth in SEQ ID NO: 65, SEQ ID NO: 66, SEQ ID NO: 67, SEQ ID NO: 68, SEQ ID NO: 69 and SEQ ID NO: 70,
   (c5) amino acid sequences derived from the amino acid sequences set forth in (c4) by substitution, deletion or addition of one or more amino acids and functionally identical or similar to the amino acid sequences as set forth in (c4), and
   (c6) amino acid sequences having at least 85% sequence identity to the amino acid sequences set forth in (c4) and comprising LCDR1, LCDR2 and LCDR3 set forth in SEQ ID NOs: 17, 18 and 19.

In some specific embodiments, the present disclosure provides an anti-B7-H3 antibody or antigen-binding fragment thereof, wherein the anti-B7-H3 antibody is a humanized antibody, wherein
(1) the amino acid sequence of the heavy chain variable region is selected from the group consisting of:
   (c1) amino acid sequences as set forth in SEQ ID NO: 72 and SEQ ID NO: 73,
   (c2) amino acid sequences derived from the amino acid sequences set forth in (c1) by substitution, deletion or addition of one or more amino acids and functionally identical or similar to the amino acid sequences as set forth in (c1), and
   (c3) amino acid sequences having at least 85% sequence identity to the amino acid sequences set forth in (c1) and comprising HCDR1, HCDR2 and HCDR3 set forth in SEQ ID NOs: 20, 21 and 22; and
(2) the amino acid sequence of the light chain variable region is selected from the group consisting of:
   (c4) amino acid sequences as set forth in SEQ ID NO: 74, SEQ ID NO: 75, SEQ ID NO: 76, SEQ ID NO: 77, SEQ ID NO: 78 and SEQ ID NO: 79,
   (c5) amino acid sequences derived from the amino acid sequences set forth in (c4) by substitution, deletion or addition of one or more amino acids and functionally identical or similar to the amino acid sequences as set forth in (c4), and
   (c6) amino acid sequences having at least 85% sequence identity to the amino acid sequences set forth in (c4) and comprising LCDR1, LCDR2 and LCDR3 set forth in SEQ ID NOs: 23, 24 and 25.

In some specific embodiments, the present disclosure provides an anti-B7-H3 antibody or antigen-binding fragment thereof, wherein the amino acid sequence of the heavy chain variable region is selected from the group consisting of SEQ ID NO: 45, the amino acid sequence derived from SEQ ID NO: 45 by substitution, deletion or addition of one or more amino acids and functionally identical to SEQ ID NO: 45, and the amino acid sequence having at least 85% sequence identity to SEQ ID NO: 45 and comprising HCDR1, HCDR2 and HCDR3 set forth in SEQ ID NOs: 1, 2 and 3; and the amino acid sequence of the light chain variable region is selected from the group consisting of SEQ ID NO: 49, the amino acid sequence derived from SEQ ID NO: 49 by substitution, deletion or addition of one or more amino acids and functionally identical to SEQ ID NO: 49, and the amino acid sequence having at least 85% sequence identity to SEQ ID NO: 49 and comprising LCDR1, LCDR2 and LCDR3 set forth in SEQ ID NOs: 4, 5 and 6.

In some specific embodiments, the present disclosure provides an anti-B7-H3 antibody or antigen-binding fragment thereof, wherein the amino acid sequence of the heavy chain variable region is selected from the group consisting of SEQ ID NO: 52, the amino acid sequence derived from SEQ ID NO: 52 by substitution, deletion or addition of one or more amino acids and functionally identical to SEQ ID NO: 52, and the amino acid sequence having at least 85% sequence identity to SEQ ID NO: 52 and comprising HCDR1, HCDR2 and HCDR3 set forth in SEQ ID NOs: 7, 8 and 9; and the amino acid sequence of the light chain variable region is selected from the group consisting of SEQ ID NO: 59, the amino acid sequence derived from SEQ ID NO: 59 by substitution, deletion or addition of one or more amino acids and functionally identical to SEQ ID NO: 59, and the amino acid sequence having at least 85% sequence identity to SEQ ID NO: 59 and comprising LCDR1, LCDR2 and LCDR3 set forth in SEQ ID NOs: 10, 11 and 12.

In some specific embodiments, the present disclosure provides an anti-B7-H3 antibody or antigen-binding fragment thereof, wherein the amino acid sequence of the heavy chain variable region is selected from the group consisting of SEQ ID NO: 63, the amino acid sequence derived from SEQ ID NO: 63 by substitution, deletion or addition of one or more amino acids and functionally identical to SEQ ID NO: 63, and the amino acid sequence having at least 85% sequence identity to SEQ ID NO: 63 and comprising HCDR1, HCDR2 and HCDR3 set forth in SEQ ID NOs: 13, 14 and 15; and the amino acid sequence of the light chain variable region is selected from the group consisting of SEQ ID NO: 65, the amino acid sequence derived from SEQ ID NO: 65 by substitution, deletion or addition of one or more amino acids and functionally identical to SEQ ID NO: 65, and the amino acid sequence having at least 85% sequence identity to SEQ ID NO: 65 and comprising LCDR1, LCDR2 and LCDR3 set forth in SEQ ID NOs: 17, 18 and 19.

In some specific embodiments, the present disclosure provides an anti-B7-H3 antibody or antigen-binding fragment thereof, wherein the amino acid sequence of the heavy chain variable region is selected from the group consisting of SEQ ID NO: 64, the amino acid sequence derived from SEQ ID NO: 64 by substitution, deletion or addition of one or more amino acids and functionally identical to SEQ ID NO: 64, and the amino acid sequence having at least 85% sequence identity to SEQ ID NO: 64 and comprising HCDR1, HCDR2 and HCDR3 set forth in SEQ ID NOs: 13, 14 and 15; and the amino acid sequence of the light chain variable region is selected from the group consisting of SEQ ID NO: 67, the amino acid sequence derived from SEQ ID NO: 67 by substitution, deletion or addition of one or more amino acids and functionally identical to SEQ ID NO: 67, and the amino acid sequence having at least 85% sequence identity to SEQ ID NO: 67 and comprising LCDR1, LCDR2 and LCDR3 set forth in SEQ ID NOs: 17, 18 and 19.

In some specific embodiments, the present disclosure provides an anti-B7-H3 antibody or antigen-binding fragment thereof, wherein the amino acid sequence of the heavy chain variable region is selected from the group consisting of SEQ ID NO: 72, the amino acid sequence derived from SEQ ID NO: 72 by substitution, deletion or addition of one or more amino acids and functionally identical to SEQ ID NO: 72, and the amino acid sequence having at least 85% sequence identity to SEQ ID NO: 72 and comprising HCDR1, HCDR2 and HCDR3 set forth in SEQ ID NOs: 20, 21 and 22; and the amino acid sequence of the light chain variable region is selected from the group consisting of SEQ ID NO: 77, the amino acid sequence derived from SEQ ID NO: 77 by substitution, deletion or addition of one or more amino acids and functionally identical to SEQ ID NO: 77, and the amino acid sequence having at least 85% sequence identity to SEQ ID NO: 77 and comprising LCDR1, LCDR2 and LCDR3 set forth in SEQ ID NOs: 23, 24 and 25.

In some specific embodiments, the present disclosure provides an anti-B7-H3 antibody or antigen-binding fragment thereof, wherein the amino acid sequence of the heavy chain variable region is selected from the group consisting of SEQ ID NO: 22, the amino acid sequence derived from SEQ ID NO: 22 by substitution, deletion or addition of one or more amino acids and functionally identical to SEQ ID NO: 22, and the amino acid sequence having at least 85% sequence identity to SEQ ID NO: 22 and comprising HCDR1, HCDR2 and HCDR3 set forth in SEQ ID NOs: 1, 2 and 3; and the amino acid sequence of the light chain variable region is selected from the group consisting of SEQ ID NO: 25, the amino acid sequence derived from SEQ ID NO: 25 by substitution, deletion or addition of one or more amino acids and functionally identical to SEQ ID NO: 25, and the amino acid sequence having at least 85% sequence identity to SEQ ID NO: 25 and comprising LCDR1, LCDR2 and LCDR3 set forth in SEQ ID NOs: 10, 11 and 12.

In some embodiments, the present disclosure provides an anti-B7-H3 humanized antibody or antigen-binding fragment thereof, wherein the heavy chain comprises a heavy chain constant region of humanized IgG1, IgG2, IgG3, IgG4 or a variant thereof, and the light chain comprises a light chain constant region of humanized kappa, lambda chain, or a variant thereof.

In a preferred embodiment of the present disclosure, the murine-derived anti-B7-H3 antibody or antigen-binding fragment thereof may further comprise a light chain constant region of murine-derived kappa, lambda chain, or a variant thereof, and/or further comprise a heavy chain constant region of murine-derived IgG1, IgG2, IgG3, IgG4, or a variant thereof.

In a preferred embodiment of the present disclosure, the antibody light chain of the anti-B7-H3 chimeric antibody or antigen-binding fragment thereof further comprises a light chain constant region of murine-derived kappa, lambda chain, or mutant sequences thereof. The antibody heavy chain of the anti-B7-H3 chimeric antibody or antigen-binding fragment thereof further comprises a heavy chain constant region of murine-derived IgG1, IgG2, IgG3, IgG4, or mutant sequences thereof, and preferably comprises a heavy chain constant region of humanized IgG1 or IgG2, or IgG4 constant region that significantly reduces ADCC (antibody-dependent cell-mediated cytotoxicity) after amino acid mutation.

In some specific embodiments, the anti-B7-H3 humanized antibody or antigen-binding fragment thereof of the present disclosure further comprises a heavy chain constant region of humanized IgG1, IgG2, IgG3, IgG4, or a variant thereof, and a light chain constant region of humanized kappa chain, lambda chain, or a variant thereof. In some preferred embodiments, the anti-B7-H3 humanized antibody or antigen-binding fragment thereof of the present disclosure further comprises a heavy chain constant region of humanized IgG1, IgG2, or a variant thereof, and a light chain constant region of humanized kappa chain or a variant thereof.

In some embodiments, the present disclosure provides an anti-B7-H3 antibody or antigen-binding fragment thereof, wherein the antigen-binding fragment is Fab, Fv, sFv or F(ab)₂.

Another aspect of the present disclosure provides an isolated nucleic acid encoding the anti-B7-H3 antibody or antigen-binding fragment thereof according to the present disclosure.

In some specific embodiments, the isolated nucleic acid according to the present disclosure, wherein the nucleotide sequence encoding the amino acid sequence of the heavy chain variable region is selected from the group consisting of SEQ ID NO: 80, SEQ ID NO: 82, SEQ ID NO: 84 and SEQ ID NO: 86; and the nucleotide sequence encoding the amino acid sequence of the light chain variable region is selected from the group consisting of SEQ ID NO: 81, SEQ ID NO: 83, SEQ ID NO: 85 and SEQ ID NO: 87.

In a specific embodiment, the isolated nucleic acid according to the present disclosure, wherein the nucleotide sequence encoding the heavy chain variable region SEQ ID NO: 45 is set forth in SEQ ID NO: 80; the nucleotide sequence encoding the light chain variable region SEQ ID NO: 49 is set forth in SEQ ID NO: 81.

In a specific embodiment, the isolated nucleic acid according to the present disclosure, wherein the nucleotide sequence encoding the heavy chain variable region SEQ ID NO: 52 is set forth in SEQ ID NO: 82; the nucleotide sequence encoding the light chain variable region SEQ ID NO: 59 is set forth in SEQ ID NO: 83.

In a specific embodiment, the isolated nucleic acid according to the present disclosure, wherein the nucleotide sequence encoding the heavy chain variable region SEQ ID NO: 64 is set forth in SEQ ID NO: 84; the nucleotide sequence encoding the light chain variable region SEQ ID NO: 67 is set forth in SEQ ID NO: 85.

In a specific embodiment, the isolated nucleic acid according to the present disclosure, wherein the nucleotide sequence encoding the heavy chain variable region SEQ ID NO: 72 is set forth in SEQ ID NO: 86; the nucleotide sequence encoding the light chain variable region SEQ ID NO: 77 is set forth in SEQ ID NO: 87.

Another aspect of the present disclosure provides an expression vector expressing the anti-B7-H3 antibody or antigen-binding fragment thereof of the present disclosure. The expression vector according to the present disclosure comprises the isolated nucleic acid molecule of the present disclosure.

Another aspect of the present disclosure provides a host cell transformed with the expression vector as described above.

In some embodiments, the host cell according to the present disclosure is selected from the group consisting of prokaryotic cells and eukaryotic cells. In some embodiments, the host cell is bacteria, preferably *Escherichia coli.* In another preferred embodiment, the host cell is mammalian cells.

Another aspect of the present disclosure provides a method for producing the anti-B7-H3 antibody or antigen-binding fragment thereof of the present disclosure, comprising expressing the antibody in the host cell and isolating the antibody from the host cell.

Another aspect of the present disclosure provides a pharmaceutical composition comprising the anti-B7-H3 humanized antibody or antigen-binding fragment thereof of the present disclosure and a pharmaceutically acceptable carrier. In some embodiments, the present disclosure provides a pharmaceutical composition comprising the anti-B7-H3 humanized antibody or antigen-binding fragment thereof of the present disclosure, and also other active components, such as other antibodies, targeting drugs, and the like. In some embodiments, the pharmaceutically acceptable carrier is selected from the group consisting of antioxidants, polypeptides, proteins, hydrophilic polymers, amino acids, saccharides, chelating agents, alditols, ions, and surfactants. In a specific embodiment, the pharmaceutically acceptable carrier is a buffered aqueous solution. In another specific embodiment, the pharmaceutically acceptable carrier is in the form of liposomes.

The anti-B7-H3 humanized antibody or antigen-binding fragment thereof of the present disclosure can be combined with a pharmaceutically acceptable carrier, diluent or excipient to prepare a pharmaceutical preparation suitable for oral or parenteral administration. The routes of administration include, but are not limited to oral, intradermal, intramuscular, intraperitoneal, intravenous, intracerebral, intraocular, intratracheal, subcutaneous, and intranasal routes. The preparation can be administered by any means, for example, by infusion or bolus injection, by the means of absorption through epithelium or skin mucosa (for example, oral mucosa or rectum, etc.). Administration can be systemic or local. The preparation can be prepared by methods known in the art, and contains a carrier, diluent or excipient conventionally used in the field of pharmaceutical preparations.

Another aspect of the present disclosure provides a method for inhibiting B7-H3 activity, comprising administering the anti-B7-H3 antibody or antigen-binding fragment thereof of the present disclosure or the pharmaceutical composition of the present disclosure to a subject in need thereof.

Another aspect of the present disclosure provides use of the anti-B7-H3 antibody or antigen-binding fragment thereof of the present disclosure or the pharmaceutical composition of the present disclosure in the manufacture of a medicament for inhibiting B7-H3 activity. In some embodiments, the medicament for inhibiting B7-H3 activity is used to treat leukemia, lymphoma, breast cancer, lung cancer, gastric cancer, intestinal cancer, esophageal cancer, ovarian cancer, cervical cancer, kidney cancer, bladder cancer, pancreatic cancer, glioma and/or melanoma. In some embodiments, the present disclosure provides use of the above-mentioned anti-B7-H3 antibody or antigen-binding fragment thereof or the pharmaceutical composition of the present disclosure in the manufacture of anti-tumor drugs. Preferably, the tumor is selected from the group consisting of leukemia, lymphoma, breast cancer, lung cancer, gastric cancer, intestinal cancer, esophageal cancer, ovarian cancer, kidney cancer, bladder cancer, pancreatic cancer, glioma and melanoma.

The anti-B7-H3 antibody or antigen-binding fragment thereof provided by the present disclosure has a higher affinity and stability and significant anti-tumor effect, and can significantly inhibit tumor growth. The humanized antibody, whose immunogenicity is greatly reduced, effectively eliminates the rejection of exogenous monoclonal antibodies by human immune system and can be used in the manufacture of medicaments for the treatment of various tumor diseases with broad market prospects.

### DEFINITIONS

Unless otherwise defined, the meanings of scientific and technical terms used herein are those commonly understood by those skilled in the art. The nomenclature and techniques used in cell and tissue culture, molecular biology, as well as protein and oligo/polynucleotide chemistry and hybridization described herein are well known and commonly used in the art. Standard techniques are used for recombinant DNA, oligonucleotide synthesis, tissue culture and transformation (e.g. electroporation, lipofection). The enzymatic reaction and purification techniques are carried out according to the manufacturer's instructions or methods commonly used in the art or described herein. The aforementioned techniques and methods are generally used according to what's well known in the art and what's described in the multiple comprehensive and more specific documents cited and discussed in this specification. Reference could be made to, Sambrook et al., Molecular Cloning: A Laboratory Manual (2nd edition, Cold Spring Harbor Laboratory Press, Cold Spring Harbor, New York (1989)). The nomenclature as well as laboratory methods and techniques used in analytical chemistry, synthetic organic chemistry, and medical and pharmaceutical chemistry described herein are well known and commonly used in the art.

In the present disclosure, the term "at least 80% sequence identity" refers to at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity. In the present disclosure, the term "at least 85% sequence identity" refers to at least 85%, 86%, 87%, 88%, 89%, 90%, 91%, 92%, 93%, 94%, 95%, 96%, 97%, 98%, 99%, or 100% sequence identity. In some preferred embodiments, the sequence identity described in the present disclosure may be at least 90%, 91%, 92%, 93%, 94%, 95%, 96%, 97%, 98%, 99%, or 100%. Sequence comparison and determination of percent identity between two sequences can be performed by the BLASTN/BLASTP algorithm on the website of National Center For Biotechnology Institute.

In an antibody molecule, three hypervariable regions of the light chain and three hypervariable regions of the heavy chain are arranged relative to each other in a three-dimensional space to form an antigen-binding surface. The antigen-binding surface is complementary to the three-dimensional surface of the bound antigen, and the three hypervariable regions of each heavy chain and light chain are referred as "complementarity determining region" or "CDR". The assignment of amino acids to each domain is defined by Kabat "Sequences of Proteins of Immunological Interest" (National Institutes of Health, Bethesda, Maryland (1987 and 1991)) or Chothia and Lesk (J. Mol. Biol. 196:901-917 (1987), Chothia et al., Nature 342:878-883 (1989)).

The "antigen-binding fragment" of the present disclosure refers to following fragment with antigen-binding activity: Fab fragment, Fab' fragment, F(ab')₂ fragment, and Fv fragment and scFv fragment that bind to human B7-H3. The Fv fragment comprises a heavy chain variable region and a light chain variable region of the antibody but no constant region, and it is the smallest antibody fragment with all antigen binding sites. Generally, Fv antibody also comprises a polypeptide linker between the VH and VL domains, and is able to form the structure required for antigen binding. Different linkers may also be utilized to link the two variable regions of antibody to form a polypeptide chain, which is called single-chain antibody or single-chain Fv (scFv).

The antibody of the present disclosure refers to an immunoglobulin molecule or an immunologically active part thereof, that is, a molecule that contains antigen-binding sites that specifically bind to the antigen (through immunological reaction). "Specific binding" means that an antibody reacts with one or more antigenic determinants of an antigen but does not react with other polypeptides, or it binds to other polypeptides with very low affinity (Kd >10⁻⁶) . Antibodies include but are not limited to polyclonal, monoclonal, chimeric, dAb (domain antibody), single chain, Fab, Fab' and F(ab')₂ fragment, Fv, scFv and Fab expression library. A monoclonal antibody (mAb) is the antibody obtained from a single cloned cell line, and the said cell line is not limited to eukaryotic, prokaryotic or phage cloned cell lines. A monoclonal antibody or antigen-binding fragment thereof can be obtained by recombination using, for example, hybridoma technology, recombination technology, phage display technology, and synthesis technology such as CDR grafting, or other existing technology.

The "murine-derived antibody" of the present disclosure is a monoclonal antibody against human B7-H3 produced according to the knowledge and skills in the art. During the production, the test subject is injected with the B7-H3 antigen, and then the hybridomas expressing the antibody with the desired sequence or functional property are isolated.

The "chimeric antibody" of the present disclosure is an antibody formed by fusing the variable regions of a murine-derived antibody with the constant regions of a human antibody, which can reduce the immune response induced by the murine-derived antibody. To establish a chimeric antibody, it is necessary to establish a hybridoma secreting murine-derived specific monoclonal antibodies first, clone the variable region genes from the mouse hybridoma cells, and then clone the constant region genes of the human antibody as needed, and chimeric genes formed by linking the mouse variable region with the human constant region genes are inserted into a human vector. Finally, the chimeric antibody is expressed in a eukaryotic system or a prokaryotic system.

The "humanized antibody" of the present disclosure is also called a CDR grafted antibody, which is the antibody produced by grafting mouse CDR sequences into the variable region framework (FR) of a human antibody. Such variable region framework sequences can be obtained from public DNA databases or public references, for example, from the ImMunoGeneTics (IMGT) website http://imgt.cines.fr or from the Journal of Immunoglobulin, 2001ISBN012441351.

### BRIEF DESCRIPTION OF DRAWINGS

FIG. 1 shows the results of the binding activity assay (ELISA) of the anti-B7-H3 humanized antibody to human 4Ig-B7-H3, wherein the abscissa represents the antibody concentration ng/ml and the ordinate represents the OD450 value.
FIG. 2 shows the results of the binding activity assay (ELISA) of the anti-B7-H3 humanized antibody to human 2Ig-B7-H3, wherein the abscissa represents the antibody concentration ng/ml and the ordinate represents the OD450 value.
FIG. 3 shows the results of the binding activity assay (ELISA) of the anti-B7-H3 humanized antibody to monkey B7-H3, wherein the abscissa represents the antibody concentration ng/ml and the ordinate represents the OD450 value.
FIG. 4 shows the detection results of IFNγ release level after anti-human B7-H3 murine monoclonal antibody treatment in SEB-stimulated PBMC system, wherein the PBMC is from donor B. The abscissa represents the different antibodies, and the ordinate represents the amount of IFNγ released (pg/ml).
FIG. 5 shows the detection results of IFNγ release level after anti-human B7-H3 murine monoclonal antibody treatment in SEB-stimulated PBMC system, wherein the PBMC is from donor C. The abscissa represents the different antibodies, and the ordinate represents the amount of IFNγ released (pg/ml).
FIG. 6 shows the detection results of IFNγ release level after anti-B7-H3 humanized antibody hu65 treatment in SEB-stimulated PBMC system. The abscissa represents the different antibodies, and the ordinate represents the amount of IFNγ released (pg/ml).
FIG. 7 shows the detection results of IFNγ release level after anti-B7-H3 humanized antibody hu51-G34K treatment in SEB-stimulated PBMC system. The abscissa represents the different antibodies, and the ordinate represents the amount of IFNγ released (pg/ml).

### DETAILED DESCRIPTION

The following representative examples are used to illustrate the present disclosure better. The experimental methods without conditions indicated in the following examples are usually carried out according to conventional conditions, such as the antibody technology experiment manual and molecular cloning manual of Cold Spring Harbor, or according to the conditions recommended by the raw material or commodity manufacturers. The materials and reagents used in the examples are all commercially available unless otherwise specified.

### Example 1 Preparation of antigen protein and positive control antibody

### 1. Construction of expression vector of antigen protein and positive control antibody (1) Construction of expression vector of antigen protein

A gene fragment encoding the full-length extracellular region of the 4Ig-B7-H3 protein was synthesized, and the amino acid sequence is shown in SEQ ID NO: 88. A gene fragment encoding the 2Ig-B7-H3 protein was synthesized, and the amino acid sequence is shown in SEQ ID NO: 89. The full-length DNA sequence of 4Ig-B7-H3 (SEQ ID NO: 90) was cloned into the eukaryotic expression plasmid pTargeT to generate the expression plasmid pTargeT-4Ig-B7-H3.

The amino acid sequence of the extracellular region of human 4Ig-B7-H3 protein was fused with the amino acid sequence of hIgG1-Fc or his-tag, and the amino acid sequences are shown in SEQ ID NO: 91 and SEQ ID NO: 92. The amino acid sequence of the extracellular region of human 2Ig-B7-H3 protein was fused with the amino acid sequence of hIgG1-Fc or his-tag, and the amino acid sequences are shown in SEQ ID NO: 93 and SEQ ID NO: 94. After codon optimization of the above amino acid sequences, the tagged B7-H3 protein extracellular region coding fragments, 4Ig-B7-H3-hFc, 4Ig-B7-H3-his, 2Ig-B7-H3-hFc, 2Ig-B7-H3-his, were synthesized and cloned into the eukaryotic expression plasmid pHR respectively to generate the respective expression plasmids of the above-mentioned tagged proteins, pHR-4Ig-B7-H3-hFc, pHR-4Ig-B7-H3-his, pHR-2Ig-B7-H3-hFc and pHR-2Ig-B7-H3-his.

### (2) Construction of expression vector of positive control antibody hBRCA84D

The humanized antibody hBRCA84D disclosed in the patent application WO2011/109400 was used as a positive control antibody, and the amino acid sequences of hBRCA84D are as follows:
heavy chain amino acid sequence of hBRCA84D: SEQ ID NO: 95; and
light chain amino acid sequence of hBRCA84D: SEQ ID NO:96.

hBRCA84D was prepared according to the method disclosed in WO2011/109400. hBRCA84D is a human IgG1 monoclonal antibody. Compared with natural IgG1, its Fc fragment contains specific mutation sites (K214R, L235V, F243L, R292P, Y300L, D356E, L358M and P396L). The amino acid sequences of hBRCA84D humanized antibody were artificially codon-optimized to obtain the full-length DNA sequences of the heavy chain and light chain of the positive control antibody hBRCA84D. The full-length DNA fragment of the hBRCA84D heavy chain was cloned into pHR vector to obtain eukaryotic expression plasmid pHR-hBRCA84D-8mIgG1. The full-length DNA fragment of the hBRCA84D light chain was cloned into pHR vector to obtain eukaryotic expression plasmid pHR-hBRCA84D-hK.

### (3) Construction of expression vector for murine-derived positive control antibody BRCA84D

The heavy chain variable region of the positive control antibody hBRCA84D was cloned into the eukaryotic expression plasmid pHR-mG2a containing murine mG2a heavy chain constant region to obtain the eukaryotic expression plasmid of pHR-hBRCA84D-mG2a. The light chain variable region of the positive control antibody hBRCA84D was cloned into the eukaryotic expression plasmid pHR-mK containing murine K light chain constant region to obtain the eukaryotic expression plasmid of pHR-hBRCA84D-mK.

### 2. Expression and purification of antigen protein and positive control antibody (1) Construction of stable transgenic cell line expressing antigen protein

CHO-K1 cells (Shanghai Institute of Biochemistry and Cell Biology, Chinese Academy of Sciences) were electrotransfected with the eukaryotic expression plasmid pTargeT-B7-H3 under a square pulse of 15msec at a voltage of 160V and then cultured in an incubator at 37°C and 5% CO₂. After 24h, the cells were subjected to selection with culture medium containing 1000 µg/ml G418 (Gibco, #10131-027). After 16 days of transfection, the positive rate of the transfection pool was detected by flow cytometry. The cells in the pool with a higher positive rate were plated (according to a cell density of 1× 10⁶ cells/ml, 100 µl/well, plated in a 96-well plate), and incubated with hBRCA84D antibody and Goat pAb to Hu IgG (PE) (Abcam, ab98596) antibody. Flow cytometer (ACEABIO, Novocyte 2060R) was used to read the mean value at a wavelength of 585 nm, and data analysis was performed using GraphPad. The positive cell lines were subcloned, and a CHO-K1 cell line was selected, which expressed B7-H3 at a high level and was named CHO-K1-B7-H3.

### (2) Expression of tagged antigen protein and positive control antibody

293E cells were inoculated into a 1L cell culture flask with a density of 0.5 × 10⁶ cells/ml. Fresh and pre-warmed FreeStyle 293 expression medium was added to make the total volume of 250mL. The cells were cultured in a humidified CO₂ incubator at 37°C and 8% CO₂ overnight. 500µl of 1 mg/ml PEI solution was added to 8.5 mL FreeStyle 293 expression medium and mixed well. 250µg of plasmid to be transfected was added to 8.5 ml FreeStyle 293 expression medium and mixed well, wherein, the tagged antigen protein plasmids pHR-4Ig-B7-H3-hFc, pHR-4Ig-B7-H3-his, pHR-2Ig-B7-H3-hFc and pHR-2Ig-B7-H3-his were transfected respectively; the heavy chain plasmid pHR-hBRCA84D-8mIgG1 and the light chain plasmid pHR-hBRCA84D-hK of positive control antibody hBRCA84D were co-transfected; and the heavy chain plasmid pHR-hBRCA84D-mG2a and the light chain plasmid pHR-hBRCA84D-mK of murine-derived positive control antibody BRCA84D were co-transfected. The FreeStyle 293 expression medium containing PEI was added to the expression medium containing plasmid and mixed well, and then the mixture was added to the cells, and cultured in a humidified CO₂ incubator at 37°C and 8% CO₂. The cells were fed on the 1st and 3rd day after cell transfection with 2.5 ml of 200 mM glutamine and 5 ml of 180 g/L glucose per flask. The cell culture supernatant was collected when the cell viability dropped to 65%~75%. The cell culture was centrifuged at 1,500 rpm for 5 min to collect the supernatant, and then centrifuged at 8,000 rpm for 20 min to collect the supernatant.

### (3) Affinity chromatography purification

Different affinity chromatography columns were used to perform purification by using AKTA machine (GE, AKTA pure-150) according to the properties of the proteins (see Table 1 for affinity chromatography columns suitable for different proteins). The specific purification steps are as follows.

**Table 1 Affinity chromatography columns suitable for different proteins**

| Protein | Column | Brand | Model |
|---|---|---|---|
| Murine monoclonal antibody (hybridoma), murine-derived positive control antibody | Protein G prepacked column | Bestchrom | Ezfast Protein G 4FF |
| 4Ig-B7-H3-his/ 2Ig-B7-H3-his | NI prepacked column | GE | His Trap, HP 5ml |
| 4Ig-B7-H3-hFc/ 2Ig-B7-H3-hFc | Protein A prepacked column | GE | Hi Trap, MabselectSuRe 5ml |
| Chimeric antibody, humanized antibody | Protein A self-packed column | GE | Mabselect LX 18ml |

### Cleaning

The equipment and pipelines were cleaned with ultrapure water for 2min with a flow rate of 10mL/min, and then the chromatography system was cleaned with 0.1M NaOH.

### Column connection

The chromatography column was connected to the chromatography equipment and rinsed with ultrapure water for 5min, and then the chromatography system was rinsed with 0.1M NaOH for 30min with a retention time of 5min.

### Equilibration

Five CVs (column volume) of 20mM PB + 0.15M NaCl, pH 7.2 was used to equilibrate the column.

### Sample loading

The supernatant from cell expression was loaded to the column with a retention time of 5 min.

### Post-equilibration

Five CVs 20mM PB+ 0.15M NaCl, pH 7.2 was used to equilibrate.

### Elution

Elution was performed with 50 mM acetic acid (pH=3.4), for a retention time of 5 min. Collection started when UV280 reached about 50 mAu, and stopped when UV280 dropped to about 50 mAu. The sample was adjusted to the pH of 7.0 with 1M Tris-HCl (pH 9.0).

### Re-equilibration

Three CVs of 20mM PB+ 0.15M NaCl, pH 7.2, was used to equilibrate with a retention time of 5min.

### On-column Cleaning

Cleaning was performed with 0.1M NaOH for 30 min with a retention time of 5 min.

### Cleaning and preservation

Cleaning was performed with purified water for 10 minutes, and then 2 CVs of 20% ethanol.

### Example 2 Preparation of monoclonal antibodies

### 1. Preparation of hybridomas

### (1) Animal immunization

The experimental animals were immunized with different tagged 4Ig-B7-H3 antigen and 2Ig-B7-H3 antigen proteins with an adjuvant, and the experimental animals included SJL mice, Balb/c mice, and SD rats. The animals were immunized with 50 µg of antigen per animal for the first shoot, and subsequently 25 µg of antigen per animal was used for immunization. The immune adjuvant used in the experiments may be Freund's adjuvant (Sigma) or Quick Antibody-Mouse5W (Beijing Biodragon immunotech. Co., Ltd.). Freund's adjuvant was used to emulsify antigen and different tagged 4Ig-B7-H3 or 2Ig-B7-H3 antigen protein samples were added dropwise to the adjuvant solution with vortex to mix thoroughly. The dosages of the adjuvant were referred to the instructions. After the mixture was mixed well to form a water-in-oil emulsion, mice were immunized. Quick Antibody-Mouse5W was used as an adjuvant and different tagged 4Ig-B7-H3 or 2Ig-B7-H3 antigen protein samples were mixed with Quick Antibody-Mouse5W at a volume ratio of 1:1. After the mixture was mixed well, SD rats were immunized by intramuscular injection.

The stable transgenic cell line CHO-K1-B7-H3 expressing B7-H3 was used to immunize SD rats and Balb/c mice to produce anti-B7-H3 antibodies. The CHO-K1-B7-H3 cells obtained in Example 1 were treated with trypsin and then centrifuged at 1,000 rpm for 5 min. The cell supernatant was discarded and the cell pellet was resuspended in PBS. Part of the cells was taken out and counted with a cell counter, and the remaining cells were centrifuged at 1,000 rpm for 5 min. The supernatant was discarded and the cell pellet was resuspended in PBS. An appropriate amount of PBS was added to obtain a cell suspension of 1 × 10⁸ cells/ml. Each mouse in the experimental group was immunized with 1×10⁷ cells, and each SD rat in the experimental group was immunized with 2×10⁷ cells.

The immunization protocol is shown in Table 2.

**Table 2 Animal immunization protocol**

| **Grou p** | **Antigen** | **Animal** | **Adjuvant** | **Immunizati on Route** |
|---|---|---|---|---|
| 1 | PBS | SJL/Balb/c/SD rat | None | s.c./i.m. |
| 2 | 4Ig-B7-H3-his/4Ig-B7-H3-hFc | SJL/Balb/c | Freund's adjuvant | s.c./i.m. |
| 3 | 2Ig-B7-H3-his/2Ig-B7-H3-hFc | Balb/c | Freund's adjuvant | s.c./i.m. |
| 4 | 4Ig-B7-H3-his/4Ig-B7-H3-hFc | SD rat | Quick Antibody-Mouse5W | i.m. |
| 5 | CHO-K1-B7-H3 | Balb/c/SD rat | None | i.p. |

| | | | | |
|---|---|---|---|---|
| ^{∗} i.m.: intramuscular injection; s.c.: subcutaneous injection; i.p.: intraperitoneal injection. | | | | |

### (2) Hybridoma fusion

Acquisition and preparation of spleen cells. The mice/rats after booster immunization were sacrificed and soaked in 75% alcohol. The spleen was dissected out, ground with a grinding rod, and filtered through a cell strainer to prepare a single cell suspension. The spleen cell suspension was centrifuged at 2,000 rpm for 5 min, and the supernatant was discarded. 2mL red blood cell lysate was added to lyse red blood cells at room temperature for 2 min and PBS was added to reach 20 mL. After centrifugation at 1,500 rpm for 7 min, the supernatant was discarded. Viable cells were counted after resuspension. The Sp2/0 cells in the culture flask were collected and after centrifuged at 1,000 rpm for 5 min, the supernatant was discarded. Viable cells were counted after resuspension. The spleen cells were mixed with Sp2/0 cells at a ratio of 1:1 and subjected to centrifugation at 1,500 rpm for 7 min, the supernatant was discarded. The cells were resuspended in 20 mL electroporation buffer. After centrifugation at 1,500 rpm for 7 min, the supernatant was discarded and the step was repeated once. The cells were resuspended with an appropriate amount of electroporation buffer to ensure the cell concentration of about 2×10⁷ cells/mL. The cell suspension was added to a 9mL electroporation tank for fusion. After fusion, the cell suspension was transferred to 15mL RPMI 1640 complete medium containing 20% FBS and then left at room temperature for 20 min. The fused cells were resuspended with RPMI 1640 medium containing 1×HAT, 1×BIOMYC3, and 20% FBS. The cell suspension was added to several 96-well cell culture plates at 100 µl/well to ensure that the cell volume per well was about 4×10⁴ cells/well, and the plates was placed in a 37°C cell incubator. After 5 days, additional 100 µL of RPMI 1640 complete medium containing 20% FBS, 1×HAT, and 1×BIOMYC-3 was added to each well.

### (3) Screening of hybridoma and subclones

After one week of fusion, the cell culture supernatants from hybridoma parent clones culture were collected and used for screening hybridoma parent clones binding to 4Ig-B7-H3-his protein and cynomolgus monkey B7-H3 protein by ELISA. The parent clones that can bind to CHO-K1-B7-H3 stable transgenic cell line were further screened by flow cytometry. The positive parent clones were expanded in 24-well plates, and the culture supernatants of the parent clones that had been cultured for 2 days were selected and co-cultured with T cells to detect the release of IFN-γ in the culture supernatant. The specific experimental operations are as follows:

1. On Day 1, T25 cell culture flasks were coated with 1 µg/ml anti-CD3e (R&D) and incubated overnight at 4°C. Liquid was discarded before the experiment, and the flasks were washed three times with PBS beforelater use.

2. On Day 2, CD8+ T cells (obtained by inducive expansion with CD3 and CD28, cryopreserved in 90% FBS + 10% DMSO) were thawed, resuspended in culture medium with IMDM + 10% FBS + free 100 ng/ml SEB + 4 ng/ml IL-2 + 100 × double antibiotics, and cultured in coated T25 in step 1 at a culture density of 2-3×10⁶ cells/ml for 48h.

3. On Day 3, 96-well plates were coated with 1 µg/ml anti-CD3e and 10 µg/ml 4Ig-B7-H3-his, and divided into the coating blank group, anti-CD3e group and anti-CD3e+B7-H3-4Ig-His group. Plates were incubated overnight at 4°C.

4. On Day 4, the 96-well plates in the previous step was added with culture supernatant from hybridoma at 100 µl/well, with 100 µl of hybridoma medium added to control well, and incubate at 37°C for 30 min.

5. CD8+T cells that had been stimulated for 2 days were collected and adjusted to a cell density of 1 × 10⁶ cells/ml, added to the 96-well plates at 100 µl/well, and cultured for 48h.

6. On Day 6, IFN-γ release in the supernatant was tested according to R&D's duset (DY285B) detection kit to detect IFN-γ in the supernatant.

The parent clones with binding ability were screened by ELISA and FACS, and the release level of cytokine IFN-γ induced by the supernatant from the parent clones was further evaluated to screened out positive parent clones that could promote the release of IFN-γ. The positive parent clones were subcloned by the limiting dilution method. After one week of culture, the binding activity of the supernatant from the subclone to 4Ig-B7-H3 was detected by ELISA, and the monoclonal cell lines secreting anti-B7-H3 antibodies were then obtained.

### 2. Identification of antibody subtypes

The antibody subtypes were identified according to the instructions of the mouse antibody subtype identification kit "SBA Clonotyping Systerm-C57BL/6-HRP" (SouthernBiotech, Cat. No. 5300-05B). The results are shown in Table 3.

**Table 3 Identification results of antibody subtypes**

| **Antibody** | **Antibody subtype** |
|---|---|
| SHS007-17 | IgG/k |
| SHS007-39 | IgG/k |
| SHS007-51 | IgG/k |
| SHS007-65 | IgG/k |

### 3. Preparation of monoclonal antibodies

According to the activity analysis results of the supernatants from cell culture, the parent clones of monoclonal antibodies 17, 39, 51, and 65 were identified and expanded. The culture medium was 1640 medium containing 10% fetal bovine serum, 1x NAEE, 1x sodium pyruvate, and 1% penicillin-streptomycin double antibiotics. When the cell confluence was >80%, the cells were subcultured and expanded. 50 ml of the supernatant was collected and the antibody was purified. The obtained antibody was subjected to SDS-PAGE gel electrophoresis and showed a good purity.

### 4. Sequencing of monoclonal antibodies

The subcloned positive hybridomas were expanded, and an appropriate amount of cells was used for total RNA extraction according to the instructions of RNeasy Plus Mini Kit (Qiagen, 74134). The first strand of cDNA was synthesized using Prime Script 1st strand cDNA Synthesis Kit (Takara, 6110A).

Specific primers were designed according to the variable region of the mouse antibody subtype, and 5' end of the primers contained the homologous arm sequence for homologous recombination with the eukaryotic expression vector. PCR amplification for the variable region of antibodies was performed using cDNA as a template to obtain the gene fragments of the light chain variable region and heavy chain variable region of the mouse antibody respectively. The design of primers refers to references: Anke Krebber, Susanne Bornhauser, Jorg Burmester et al. Reliable cloning of functional antibody variable domains from hybridomas and spleen cell repertoires employing a reengineered phage display system. Journal of Immunological Methods, 1997, 201: 35-55; 2. Simon KorenMihaKosmačAnjaColjaVenturinietal. Antibody variable-region sequencing as a method for hybridoma cell-line authentication, 2008, 78: 1071-1078. DNA sequencing was performed and the results are shown in Table 4.

**Table 4 Sequence table of anti-B7-H3 murine-derived monoclonal antibody**

| Antibody | Amino acid sequence of the heavy chain variable region | Amino acid sequence of the light chain variable region |
|---|---|---|
| SHS007-17 | SEQ ID NO: 26 | SEQ ID NO: 27 |
| SHS007-39 | SEQ ID NO: 28 | SEQ ID NO: 29 |
| SHS007-51 | SEQ ID NO: 30 | SEQ ID NO: 31 |
| SHS007-59 | SEQ ID NO: 33 | SEQ ID NO:34 |
| SHS007-65 | SEQ ID NO:35 | SEQ ID NO:36 |
| SHS007-72 | SEQ ID NO:37 | SEQ ID NO:38 |
| SHS007-90 | SEQ ID NO:39 | SEQ ID NO:40 |
| SHS007-95 | SEQ ID NO:41 | SEQ ID NO:42 |
| SHS007-108 | SEQ ID NO:43 | SEQ ID NO:44 |

### Example 3 Construction of chimeric antibodies

The gene fragments of heavy chain variable region of mouse antibodies obtained by sequencing as shown in Table 4 were cloned into an eukaryotic expression plasmid (pHR-8mIgG1) containing the heavy chain constant region with specific mutation sites (K214R, L235V, F243L, R292P, Y300L, D356E, L358M and P396L). The gene fragments of light chain variable region of mouse antibodies obtained by sequencing as shown in Table 4 were cloned into an eukaryotic expression plasmid (pHR-hK) containing human light chain constant region. The constructed plasmids expressing the heavy chain or light chain were transformed into *Escherichia coli* DH5α competent cells with the linearized eukaryotic expression plasmid containing the light chain constant region or the heavy chain constant region of the human antibody. The mixture was spread evenly on the surface of the agar plates containing the corresponding antibiotics. The agar plates were incubated in a 37°C constant temperature incubator overnight, and then several single colonies were picked out for DNA sequencing. The positive clones with correct sequences were inoculated in 2×YT liquid medium containing the corresponding antibiotics and cultured at 37°C for more than 12 hours with shaking. The bacterial cells were collected for plasmid extraction to obtain the plasmids expressing the light chain and the heavy chain of the chimeric antibody. The concentration and purity of the plasmids were detected by a nucleic acid quantitative analyzer.

The plasmids encoding chimeric antibody's heavy and light chains were co-transfected into HEK293E cells. The chimeric antibodies were expressed, purified and named SHS007-17-CHI, SHS007-39-CHI, SHS007-51-CHI and SHS007-65-CHI. The purity, activity, and affinity were then tested and analyzed.

The sequences of the chimeric antibodies SHS007-17-CHI, SHS007-39-CHI, SHS007-51-CHI and SHS007-65-CHI were subjected to gene mutation by site-directed mutagenesis to screen for better antibodies. G at position 34 of SHS007-51-CHI light chain CDR was mutated to K, and the obtained antibody had improved stability, and was marked as SHS007-51-G34K-CHI. The sequencing results of the chimeric antibodies are shown in Table 5.

**Table 5 Sequences of anti-B7-H3 chimeric antibodies**

| Chimeric antibody | Amino acid sequence of the heavy chain variable region | Amino acid sequence of the light chain variable region |
|---|---|---|
| SHS007-17-CHI | SEQ ID NO: 26 | SEQ ID NO: 27 |
| SHS007-39-CHI | SEQ ID NO: 28 | SEQ ID NO: 29 |
| SHS007-51-CHI | SEQ ID NO: 30 | SEQ ID NO: 31 |
| SHS007-51-G34K-CHI | SEQ ID NO: 30 | SEQ ID NO: 32 |
| SHS007-65-CHI | SEQ ID NO: 35 | SEQ ID NO: 36 |

### Example 4 Construction and production of humanized antibodies

According to the analysis of immune activity and killing activity of chimeric antibodies, SHS007-17-CHI, SHS007-39-CHI, SHS007-51-G34K-CHI and SHS007-65-CHI were modified to humanized antibodies.

To construct the humanized antibodies, the variable regions of SHS007-17-CHI, SHS007-39-CHI, SHS007-51-G34K-CHI and SHS007-65-CHI antibodies were compared with the mouse antibody sequences in the ImMunoGeneTics (IMGT) to determine their murine-derived germlines. After homology comparison, it was found that the FR region sequences of the heavy chain variable region of SHS007-17-CHI, SHS007-39-CHI, SHS007-51-G34K-CHI and SHS007-65-CHI antibodies were the most similar to the germline gene of the mouse antibody IGHV1-69^{∗}02, IGHV1-2^{∗}02, IGHV3-21^{∗}01 and IGHV3-48^{∗}01 respectively; the FR sequences of the light chain variable region of the antibodies were the most similar to the mouse antibody IGKV2-28^{∗}01, IGKV3-11^{∗}01, IGKV2-30^{∗}02 and IGKV3-11^{∗}01 respectively. With the framework region sequence FR1-FR3 of SHS007-17-CHI, SHS007-39-CHI, SHS007-51-G34K-CHI and SHS007-65-CHI antibodies as templates, full human framework regions with similar 3D structure but low immunogenicity were screened in the human framework region library to replace FR1-FR3 sequence of SHS007-17-CHI, SHS007-39-CHI, SHS007-51-G34K-CHI and SHS007-65-CHI. The full-length sequences of the heavy/light chain were 3D modeled and compared structurally with the heavy/light chain sequences of the original antibodies. Considering the antigenicity and 3D structural similarity, 4 humanized heavy chain variable regions (see SEQ ID NOs: 45, 46, 47 and 48) and 3 humanized light chain variable regions (see SEQ ID NOs: 49, 50 and 51) of SHS007-17-CHI, 6 humanized heavy chain variable regions (see SEQ ID NOs: 52, 53, 54, 55, 56 and 57) and 5 humanized light chain variable regions (see SEQ ID NOs: 58, 59, 60, 61 and 62) of SHS007-39-CHI, 2 humanized heavy chain variable regions (see SEQ ID NOs: 63 and 64) and 6 humanized light chain variable regions (see SEQ ID NOs: 65, 66, 67, 68, 69 and 70) of SHS007-51-G34K-2, and 3 humanized heavy chain variable regions (see SEQ ID NOs: 71, 72 and 73) and 6 humanized light chain variable regions (see SEQ ID NOs: 74, 75, 76, 77, 78 and 79) of SHS007-65-CHI were ultimately selected for further optimization. The sequences of non-CDR regions of the humanized antibodies of SHS007-17-CHI, SHS007-39-CHI, SHS007-51-G34K-CHI and SHS007-65-CHI all realized more than 95% humanization.

The amino acid sequences of the light chain and heavy chain variable region of humanized antibody obtained above were reversely transcribed into their corresponding nucleotide sequences, and oligonucleotide fragments containing complementary sequences between adjacent fragments were generated. The oligonucleotide fragments were annealed and assembled by Overlap PCR. Then nucleotide fragments of the entire light chain and heavy chain variable regions were amplified using specific primers (5' end contained the homologous arm sequence for homologous recombination with the eukaryotic expression vector). The purified nucleotide fragments of the light chain variable region were co-transformed into *Escherichia coli* DH5α competent cells with the linearized eukaryotic expression plasmid (pHR-hK) containing light chain constant region of human K. The purified nucleotide fragments of the heavy chain variable region were co-transformed into *Escherichia coli* DH5α competent cells with the eukaryotic expression plasmid (pHR-IgG1) containing heavy chain constant region of human IgG1. The competent cells transformed with the plasmids were spread evenly on the surface of the agar plates containing the corresponding antibiotics. The agar plates were incubated in a 37°C constant temperature incubator overnight, and then several single colonies were picked out for DNA sequencing.

The positive clones with correct sequences were inoculated in 2×YT liquid medium containing the corresponding antibiotics and cultured at 37°C with shaking for more than 12 hours. The bacterial cells were collected for plasmid extraction to obtain the expression plasmids for the light chain and the heavy chain of humanized antibodies. The concentration and purity of the plasmids were detected by a nucleic acid quantitative analyzer.

The plasmids were transfected into HEK293E cells, and a large number of antibodies were expressed and purified. The purity, activity and affinity were tested and analyzed.

The humanized antibodies with good purity, activity and affinity were selected, and named as hu17-00, hu39-01, hu65-13 and hu51-G34K-12. The sequences are shown in Table 6.

**Table 6 Sequence table of anti-B7-H3 humanized antibodies**

| Humanized antibody | Amino acid sequence | | Nucleotide sequence | |
|---|---|---|---|---|
| | Heavy chain variable region | Light chain variable region | Heavy chain variable region | Light chain variable region |
| hu17-00 | SEQ ID NO: 45 | SEQ ID NO: 49 | SEQ ID NO: 80 | SEQ ID NO: 81 |
| hu39-01 | SEQ ID NO: 52 | SEQ ID NO: 59 | SEQ ID NO: 82 | SEQ ID NO: 83 |
| hu51-G34K-12 | SEQ ID NO: 64 | SEQ ID NO: 67 | SEQ ID NO: 84 | SEQ ID NO: 85 |
| hu65-13 | SEQ ID NO: 72 | SEQ ID NO: 77 | SEQ ID NO: 86 | SEQ ID NO: 87 |

### Example 5 Using ELISA to measure binding activity of the antibodies to human B7-H3

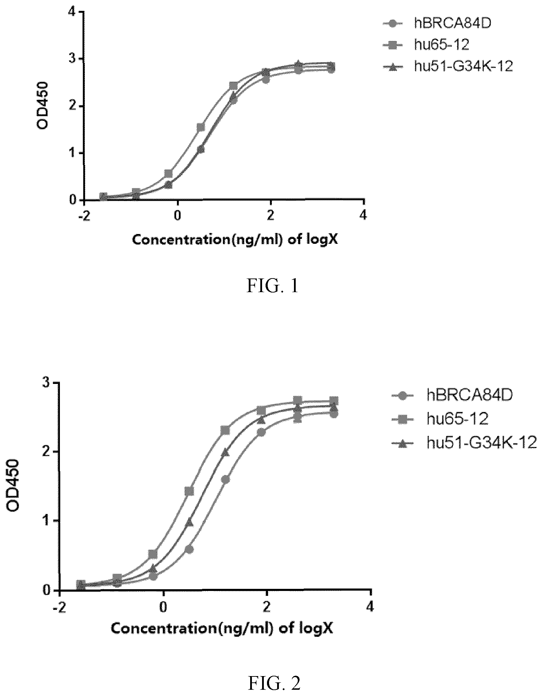

The binding activity of antibodies was analyzed by ELISA. 96-well ELISA plates were coated with human 4Ig-B7-H3-His protein or 2Ig-B7-H3-His protein (1 µg/well, prepared in Examples 1 and 2) and incubated overnight at 4°C. The plates were washed 3 times with 1xPBST and then blocked with 5% skim milk at 37°C for 2h. After washing 3 times with 1xPBST, the anti-B7-H3 antibody of the present disclosure was used as the primary antibody and added to the ELISA plates in 5-fold gradient dilution starting from 2 µg/mL with a total of 8 concentrations: 2,000 ng/mL, 400 ng/mL, 80 ng/mL, 16 ng/mL, 3.2 ng/mL, 0.64 ng/mL, 0.128 ng/mL and 0.0256 ng/mL respectively. The plates were incubated at 37°C for 2h with hBRCA84D as the positive control antibody. After washing 5 times with 1xPBST, Anti-Human IgG HRP (Jackson, 109-035-003, 1:5000) was used as the secondary antibody and incubated at 37°C for 1 h. After washing 5 times with 1xPBST, color developing solution TMB was added to the plate, and microplate reader (Thermo, Multiskan FC) was used to read OD450 value after termination of the reaction. EC₅₀ was generated by GraphPad. The result is shown in FIG. 1 and FIG. 2.

The experimental results show that the humanized anti-B7-H3 antibodies hu51-G34K-12 and hu65-13 of the present disclosure had the ability to bind to human 4Ig-B7-H3 and 2Ig-B7-H3, and the binding ability was comparable to that of the positive control antibody hBRCA84D.

### Example 6 Using ELISA to measure binding activity of the antibodies to cynomolgus monkey B7-H3

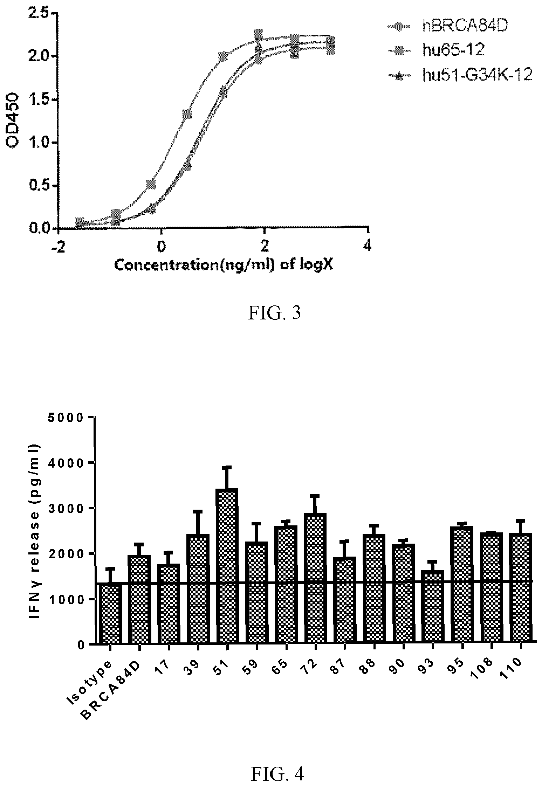

The binding activity of antibodies was analyzed by protein based ELISA. Cynomolgus B7-H3-His (0.5 µg/well, Sino Biological, Cat. No. 90806-C08H) was coated on 96-well ELISA plates. The anti-B7-H3 antibodies of the present disclosure was used as the primary antibody and added to the ELISA plates in 5-fold gradient dilution starting from 2 µg/mL with a total of 8 concentrations: 2,000 ng/mL, 400 ng/mL, 80 ng/mL, 16 ng/mL, 3.2 ng/mL, 0.64 ng/mL, 0.128 ng/mL, and 0.0256 ng/mL respectively. The plates were incubated at 37°C for 2h with AB06.12-4P as the positive control antibody. Anti-Human IgG HRP (Jackson, 109-035-003, 1:10,000) was used as the secondary antibody. Color developing solution TMB (3,3',5,5'-tetramethylbenzidine) was added to the plate, and microplate reader (Thermo, Multiskan FC) was used to read OD450 value after termination of the reaction. EC₅₀ was generated by GraphPad. The result is shown in FIG. 3.

The experimental results show that the humanized anti-B7-H3 antibodies hu51-G34K-12 and hu65-13 of the present disclosure had the ability to bind to cynomolgus B7-H3, and the binding ability was comparable to that of the positive control antibody hBRCA84D.

### Example 7 Using FACS to measure binding activity of the antibodies to B7-H3 on surface of tumor cell

The binding activity of antibodies to B7-H3 on the surface of tumor cells was analyzed by FACS. Mean fluorescence intensity (MFI) and EC₅₀ detected by FACS indicate the binding activity of the antibody to cells. After 786-0 and MDA-MB-231 cells were digested, they were resuspended in 2% FBS-PBS solution and counted. After raji-B7-H3 cells were collected, they were resuspended in 2% FBS-PBS and counted. The above cells were plated in a cell plate with 1×10⁵ cells per well. The anti-B7-H3 antibody of the present disclosure was used as the primary antibody and added to cell plates in gradient dilution starting from 20 µg/mL with a total of 8 concentrations: 20,000 ng/mL, 10,000 ng/mL, 2,000 ng/mL, 400 ng/mL, 80 ng/mL, 16 ng/mL, 3.2 ng/mL, and 0.64 ng/mL respectively. The plates were incubated at 4°C for 1h with hBRCA84D as the positive control antibody. PE-Anti-Human IgG (Biolegend, Cat. No. 409303, 1.25 µl/well) was used as the secondary antibody, washed, and detected for the fluorescence intensity generated by the binding of the antibody to the cell surface by a flow cytometer. EC₅₀ was calculated using GraphPad and the results are shown in Tables 7a and 7b.

**Table 7a Assay for binding to B7-H3 on surface of tumor cell**

| Tumor cell type | Mean fluorescence intensity (MFI) | | |
|---|---|---|---|
| | hBRCA84D | hu65-13 | hu51-G34K-12 |
| 786-0 | 2.33E+05 | 5.66E+05 | 5.84E+05 |
| MDA-MB-231 | 3.58E+04 | 1.51E+05 | 1.56E+05 |
| Raji-B7-H3 | 2.33E+05 | 5.66E+05 | 5.84E+05 |

**Table 7b Assay for binding to B7-H3 on surface of tumor cell**

| Tumor cell type | EC₅₀ (µg/ml) | | |
|---|---|---|---|
| | hBRCA84D | hu65-13 | hu51-G34K-12 |
| 786-0 | 0.2256 | 0.2658 | 0.1199 |
| MDA-MB-231 | 0.3844 | 0.1403 | 0.0501 |
| Raji-B7-H3 | 0.2256 | 0.2658 | 0.1199 |

The experimental results show that the humanized anti-B7-H3 antibodies, hu51-G34K-12 and hu65-13, can bind to B7-H3 on the surface of tumor cells, and the binding density to tumor cells was significantly higher than that of the control antibody hBRCA84D. Both EC₅₀ and Emax of the binding were significantly better than that of the control antibody hBRCA84D.

### Example 8 Using Biacore to measure binding activity of the antibodies

The humanized anti-B7-H3 antibodies prepared in Example 4 were subjected to affinity assay by Biacore. The antigen 4Ig-B7-H3-His was first coupled to the surface of a CM5 chip (GE, Cat. No. BR100012), and the active groups of the uncoupled protein were blocked with ethanolamine. After the baseline was stable, the hu39-01, hu65-13, and hBRCA84D antibodies were respectively diluted in equal proportions. The diluted antibodies of each concentration were placed on the sample injection tray of the instrument in sequence, and the program was set to inject samples respectively. The affinities between hu51-G34K-12, hu65-13, hBRCA84D antibodies and 4Ig-B7-H3-His antigen were determined according to the operating procedures of Biacore XI00. The specific parameters and experimental results are shown in Table 8.

**Table 8 Affinity of antibody to human B7-H3 protein**

| Antibody | KD (M) | kon (1/Ms) | kdis(1/s) |
|---|---|---|---|
| hu51-G34K-12 | 490E-10 | 7.85E+05 | 3.85E-04 |
| hu65-13 | 1.98E-10 | 9.54E+04 | 1.89E-05 |
| hBRCA84D | 4.55E-10 | 2.43E+05 | 1.11E-04 |

The experimental results show that hu51-G34K-12 and hu65-13 bound to human 4Ig-B7-H3 protein with good affinity, and the affinity value was comparable to that of the positive control antibody hBRCA84D.

### Example 9 Detection of blocking activity of B7-H3 murine monoclonal antibody and humanized antibody by IFN-γ release

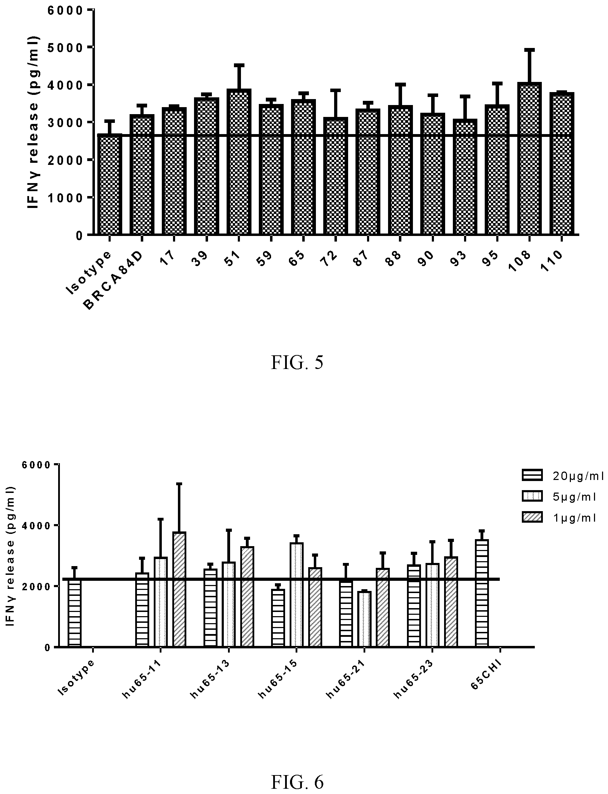

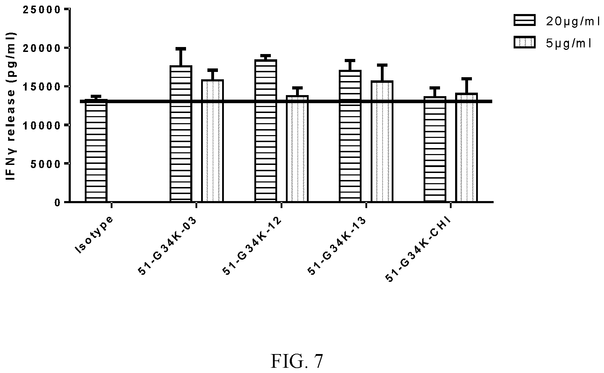

Human peripheral blood mononuclear cells (PBMCs) were isolated by Ficoll (GE Healthcare, Cat. No. 10268731). The isolated PBMCs were resuspended in IMDM (Life technologies, 12440053) complete medium containing 10% FBS and adjusted to a density of 1^{∗}10⁶ cells/mL. PBMC cells were added to a 96-well plate at 100 µL/well and allowed to stand for 30 min. The antibody to be detected and the isotype antibody were prepared in the same medium to a final concentration of 20 µg/ml, added to the 96-well plate, and incubated in a constant temperature incubator at 37°C for 1 h. SEB (Toxin techonology, BT202) solution with a final concentration of 100 ng/ml was prepared with the culture medium and added to the corresponding wells of the 96-well plate. After co-culture in the 96-well plate in the constant temperature incubator for 72 h, the concentration of IFN-γ in the culture supernatant was detected according to the instructions of DuoSet detection kit (R&D, DY285B). FIG. 4 and FIG. 5 show that anti-human B7-H3 murine monoclonal antibodies SHS007-39, 51, 59, 65, 72, etc., in the PBMC-SEB system derived from donor B and donor C, increased the release of cytokine IFN-γ in the system. FIG. 6 and FIG. 7 show that anti-human B7-H3 humanized antibodies hu51 and hu65 increased the release of IFN-γ in the PBMC-SEB experimental system.

### Example 10 Antibody-dependent cell-mediated cytotoxicity (ADCC) assay (LDH method)

Human renal carcinoma cells 786-0 have a high expression level of human B7-H3. 786-0 cells were plated in 96-well plates at 20,000 cells per well. RPMI-1640 medium containing 2% FBS was prepared with heat-inactivated fetal bovine serum (FBS, Gibco, Cat. No. 10091-148). SHS007-17CHI, SHS007-39CHI, SHS007-51CHI, SHS007-65CHI, hBRCA84D antibodies were diluted in 10-fold gradient dilution in the above medium, and incubated in a constant temperature incubator at 37°C for 1 h. According to the instructions of Ficoll (GE Healthcare, Cat. No. 10268731) kit, human peripheral blood PBMCs were isolated as effector cells, and the PBMC cells and the above antibody solution were added to the 96-well cell plates plated with 786-0 cells. The cell plates were incubated in a 37°C incubator for 4 h, and detected for cytotoxicity according to the instructions of Cytotoxicity LDH Assay Kit-WST^{®} (Dojindo, CK12). The fold reduction of the killing EC₅₀ of antibodies of the present disclosure (i.e., the ratio of the EC₅₀ value of the positive control hBRCA84D to the EC₅₀ value of the antibody of the present disclosure) was calculated. The experimental results are shown in Table 9.

**Table 9 ADCC activity of chimeric antibodies**

| **Antibody** | **EC₅₀ Ratio** |
|---|---|
| **hBRCA84D** | 1 |
| **SHS007-17CHI** | 20.1 |
| **SHS007-39-CHI** | 211.5 |
| **SHS007-51-CHI** | 6.6 |
| **SHS007-59-CHI** | 4.4 |
| **SHS007-65-CHI** | 18.6 |
| **SHS007-72-CHI** | 7.1 |
| **SHS007-95-CHI** | 11.4 |

The experimental results show that, compared with the positive control hBRCA84D antibody, the antibodies of the present disclosure had a better EC₅₀ value and greatly improved the killing activity of ADCC.

### Example 11 Anti-tumor test in mouse xenograft model from human tumor cells

### 1. Experimental materials

### (1) Experimental cells and animals

Human malignant glioma U87 cells were purchased from the American Type Culture Collection (ATCC).

Human renal carcinoma cells 786-0 cells were purchased from the American Type Culture Collection (ATCC).

Human triple-negative breast cancer MDA-MB-231 cells were purchased from the American Type Culture Collection (ATCC).

SCID-Beige mice, female, 6-8 weeks old, weighing 18-20 grams, were purchased from Shanghai Lingchang Laboratory Animal Co., Ltd..

### (2) Test samples and controls

Negative control isotype IgG1 (Cat. No. C0001-4) was purchased from Crown Bioscience Co., Ltd. In the mouse xenograft model, the effect of hBRCA84D-IgG1 was much better than that of hBRCA84D, so hBRCA84D-IgG1 was used as a positive control. hBRCA84D-IgG1 was a human IgG1 monoclonal antibody constructed using the variable region sequence of the reference antibody hBRCA84D, of which the constant region was the same as the human IgG1 chimeric antibody of the present disclosure.

Before the test, the anti-B7-H3 chimeric antibodies of the present disclosure (SHS007-39-IgG1 and SHS007-65-IgG1) were prepared in PBS at 1 mg/mL, and isotype IgG1 and hBRCA84D-IgG1 were prepared at 1 mg/mL.

### 2. Experimental method

### (1) U87 xenograft model

U87 cells were inoculated on the right rear of the mice at 5×10⁶ cells /mouse. Grouping was performed when the tumors were at an average volume of 140 mm³. Mice were administered twice a week through tail vein, and the diameter of the tumor was measured with a vernier caliper twice a week to calculate the tumor volume according to V= 0.5a×b², where a and b represented the long and short diameters of the tumor, respectively. The tumor volume was measured and recorded for 24 days of continuous administration, and the tumor growth curve was plotted with GraphPad Prism. The results are shown in Table 10.

**Table 10 Results of anti-tumor test in human malignant glioma U87 xenograft model**

| **Antibody** | **Dose (mg/kg)** | **T/C (%)** |
|---|---|---|
| **Isotype IgG1** | 10 | 100 |
| **hBRCA84D-IgG1** | 10 | 56.9 |
| **SHS007-39-CHI** | 10 | 41.3 |

### (2) 786-0 xenograft model

786-0 cells were inoculated on the right rear of the mice at 1×10⁷ cells/mice. After 2 weeks of inoculation, the tumor mass formed by 786-0 cell inoculation was removed with sterile surgical instruments, and the tumor tissue in the vigorous growth stage was cut into about 30 mm³ in normal saline, and inoculated into the right back of mice under sterile conditions. Grouping was performed when the tumor grew to an average volume of 160 mm³. Mice were administered twice a week through tail vein, and the diameter of the tumor was measured with a vernier caliper twice a week to calculate the tumor volume according to V= 0.5a×b², where a and b represented the long and short diameters of the tumor, respectively. The tumor volume was measured and recorded for 19 days of continuous administration, and the tumor growth curve was plotted with GraphPad Prism. The results are shown in Table 11.

**Table 11 Results of anti-tumor test in human renal carcinoma cell 786-0 xenograft model**

| **Antibody** | **Dose (mg/kg)** | **T/C (%)** |
|---|---|---|
| **Isotype IgG1** | 10 | 100 |
| **hBRCA84D-IgG1** | 10 | 69.78 |
| **SHS007-39-CHI** | 10 | 63.93 |
| **SHS007-65-CHI** | 10 | 52.94 |

### (3) MDA-MB-231 xenograft model

MDA-MB-231 cells were inoculated on the right rear of the mice at 8×10⁶ cells/mice. After 2 weeks of inoculation, the tumor mass formed by MDA-MB-231 cell inoculation was removed with sterile surgical instruments, and the tumor tissue in the vigorous growth stage was cut into about 30 mm³ in normal saline, and inoculated into the right back of mice under sterile conditions. Grouping was performed when the tumor grew to an average volume of 150 mm³. Mice were administered twice a week through tail vein, and the diameter of the tumor was measured with a vernier caliper twice a week to calculate the tumor volume according to V= 0.5a×b², where a and b represented the long and short diameters of the tumor, respectively. The tumor volume was measured and recorded for 21 days of continuous administration, and the tumor growth curve was plotted with GraphPad Prism. The results are shown in Table 12.

**Table 12 Results of anti-tumor test in human triple-negative breast cancer cell MDA-MB-231 xenograft model**

| **Antibody** | **Dose (mg/kg)** | **T/C (%)** |
|---|---|---|
| **Isotype IgG1** | 10 | 100 |
| **hBRCA84D-IgG1** | 10 | 86.33 |
| **SHS007-39-CHI** | 10 | 62.84 |
| **SHS007-65-CHI** | 10 | 54.26 |

The experimental results show that the antibodies of the present disclosure had the effect of inhibiting tumor growth in the SCID-Beige mouse xenograft model inoculated with human malignant glioma U87 cells, human renal carcinoma cells 786-0 cells, and human triple-negative breast cancer cells MDA-MB-231. SHS007-39-CHI and SHS007-65-IgG1 had better effect on inhibiting tumor than the control antibody hBRCA84D-IgG1. The anti-B7-H3 antibodies of the present disclosure had a more significant effect of inhibiting tumor growth.

### Example 12 Anti-tumor test in mouse xenograft model from human tumor cells

### 1. Experimental materials

### (1) Experimental cells and animals

Human renal carcinoma cells 786-0 cells were purchased from the American Type Culture Collection (ATCC).

Human triple-negative breast cancer MDA-MB-231 cells were purchased from the American Type Culture Collection (ATCC).

SCID-Beige mice, female, 6-8 weeks old, weighing 18-20 grams, were purchased from Shanghai Lingchang Laboratory Animal Co., Ltd..

### (2) Test samples and controls

The control isotype IgG1 (Cat. No. C0001-4) was purchased from Crown Bioscience Co., Ltd. and used as a negative control. The control hBRCA84D-IgG1 was a human IgG1 monoclonal antibody constructed using the variable region sequence of the reference antibody hBRCA84D, of which the constant region was the same as the humanized chimeric antibody of the present disclosure, and used as a positive control.

Before the test, the humanized anti-B7-H3 antibodies of the present disclosure hu65-13 and hu51-G34K-12 were prepared in PBS at 1 mg/mL or 2 mg/mL, and isotype IgG1 and hBRCA84D-IgG1 were prepared at 1 mg/mL or 2 mg/mL.

### 2. Experimental method

### (1) 786-0 xenograft model

786-0 cells were inoculated on the right rear of the mice at 1×10⁷ cells/mice. After 2 weeks of inoculation, the tumor mass formed by 786-0 cell inoculation was removed with sterile surgical instruments, and the tumor tissue in the vigorous growth stage was cut into about 15 mm³ in normal saline, and inoculated into the right back of mice under sterile conditions. Grouping was performed when the tumor grew to an average volume of 100 mm³. Mice were administered twice a week through tail vein until day 21, and then the frequency of administration was reduced to once a week. The diameter of the tumor was measured with a vernier caliper twice a week to calculate the tumor volume according to V= 0.5a×b², where a and b represented the long and short diameters of the tumor, respectively. The tumor volume was measured and recorded for 36 days of continuous administration, and the tumor growth curve was plotted with GraphPad Prism. The results are shown in Table 13.

**Table 13 Results of anti-tumor test in human renal carcinoma cell 786-0 xenograft model**

| **Antibody** | **Dose (mg/kg)** | **T/C (%)** |
|---|---|---|
| Isotype | 10 | 100 |
| hBRCA84D-IgG1 | 10 | 85.4 |
| hu51-G34K-12 | 10 | 52.0 |
| hu65-13 | 10 | 60.8 |

### (2) MDA-MB-231 xenograft model

Human tumor cell MDA-MB-231 cells were inoculated on the right rear of the mice at 8×10⁶ cells/mice. After 2 weeks of inoculation, the tumor mass formed by MDA-MB-231 cell inoculation was removed with sterile surgical instruments, and the tumor tissue in the vigorous growth stage was cut into about 30 mm³ in normal saline, and inoculated into the right back of mice under sterile conditions. Grouping was performed when the tumor grew to an average volume of 100 mm³. Mice were administered twice a week through tail vein, and the diameter of the tumor was measured with a vernier caliper twice a week to calculate the tumor volume according to V= 0.5a×b², where a and b represented the long and short diameters of the tumor, respectively. The tumor volume was measured and recorded for 25 days of continuous administration, and the tumor growth curve was plotted with GraphPad Prism. The results are shown in Table 14.

**Table 14 Results of anti-tumor test in human triple-negative breast cancer cell MDA-MB-231 xenograft model**

| **Antibody** | **Dose (mg/kg)** | **T/C (%)** |
|---|---|---|
| Isotype IgG1 | 10 | 100 |
| hBRCA84D-IgG1 | 20 | 59.38 |
| hu65-13 | 10 | 52.55 |

The experimental results show that the humanized antibodies of the present disclosure hu51-G34K-12 and hu65-13 had the effect of inhibiting tumor growth in the SCID-Beige mouse xenograft model inoculated with human renal carcinoma cells 786-0 cells and human triple-negative breast cancer cells MDA-MB-231. hu51-G34K-12 and hu65-13 had better effect on inhibiting tumor than the control antibody hBRCA84D-IgG1. The anti-B7-H3 antibodies of the present disclosure had a more significant effect of inhibiting tumor growth.

The above examples demonstrate that the anti-B7-H3 antibodies of the present disclosure have a significant anti-tumor effect and can significantly inhibit tumor growth, suggesting that the antibody can be used in the manufacture of anti-tumor drugs and has a good market prospect.
week through tail vein, and the diameter of the tumor was measured with a vernier caliper twice a week to calculate the tumor volume according to V= 0.5a×b², where a and b represented the long and short diameters of the tumor, respectively. The tumor volume was measured and recorded for 25 days of continuous administration, and the tumor growth curve was plotted with GraphPad Prism. The results are shown in Table 14.

**Table 14 Results of anti-tumor test in human triple-negative breast cancer cell MDA-MB-231 xenograft model**

| **Antibody** | **Dose (mg/kg)** | **T/C (%)** |
|---|---|---|
| Isotype IgG1 | 10 | 100 |
| hBRCA84D-IgG1 | 20 | 59.38 |
| hu65-13 | 10 | 52.55 |

The experimental results show that the humanized antibodies of the present disclosure hu51-G34K-12 and hu65-13 had the effect of inhibiting tumor growth in the SCID-Beige mouse xenograft model inoculated with human renal carcinoma cells 786-0 cells and human triple-negative breast cancer cells MDA-MB-231. hu51-G34K-12 and hu65-13 had better effect on inhibiting tumor than the control antibody hBRCA84D-IgG1. The anti-B7-H3 antibodies of the present disclosure had a more significant effect of inhibiting tumor growth.

The above examples demonstrate that the anti-B7-H3 antibodies of the present disclosure have a significant anti-tumor effect and can significantly inhibit tumor growth, suggesting that the antibody can be used in the manufacture of anti-tumor drugs and has a good market prospect.

### Claims

1. An anti-B7-H3 antibody or antigen-binding fragment thereof, comprising a heavy chain variable region and a light chain variable region, wherein
   (1) the heavy chain variable region comprises HCDR1, HCDR2 and HCDR3, selected from the group consisting of:
      (a1) amino acid sequences as set forth in SEQ ID NOs: 1, 2 and 3,
      (a2) amino acid sequences as set forth in SEQ ID NOs: 7, 8 and 9,
      (a3) amino acid sequences as set forth in SEQ ID NOs: 13, 14 and 15,
      (a4) amino acid sequences as set forth in SEQ ID NOs: 20, 21 and 22, and
      (a5) CDRs having at least 85% sequence identity to the amino acid sequences set forth in (a1), (a2), (a3), or (a4); and
   (2) the light chain variable region comprises LCDR1, LCDR2 and LCDR3, selected from the group consisting of:
      (a6) amino acid sequences as set forth in SEQ ID NOs: 4, 5 and 6,
      (a7) amino acid sequences as set forth in SEQ ID NOs: 10, 11 and 12,
      (a8) amino acid sequences as set forth in SEQ ID NOs: 16, 18 and 19,
      (a9) amino acid sequences as set forth in SEQ ID NOs: 17, 18 and 19,
      (a10) amino acid sequences as set forth in SEQ ID NOs: 23, 24 and 25, and
      (a11) CDRs having at least 85% sequence identity to the amino acid sequences set forth in (a6), (a7), (a8), (a9), or (a10).
2. The anti-B7-H3 antibody or antigen-binding fragment thereof according to claim 1, wherein
   the heavy chain variable region comprises HCDR1, HCDR2, and HCDR3 set forth in SEQ ID NOs: 1, 2 and 3 respectively, or the CDRs having at least 85% sequence identity to the amino acid sequences set forth in SEQ ID NOs: 1, 2 and 3; and the light chain variable region comprises LCDR1, LCDR2 and LCDR3 set forth in SEQ ID NOs: 4, 5 and 6 respectively, or the CDRs having at least 85% sequence identity to the amino acid sequences set forth in SEQ ID NOs: 4, 5 and 6;
   the heavy chain variable region comprises HCDR1, HCDR2, and HCDR3 set forth in SEQ ID NOs: 7, 8 and 9 respectively, or the CDRs having at least 85% sequence identity to the amino acid sequences set forth in SEQ ID NOs: 7, 8 and 9; and the light chain variable region comprises LCDR1, LCDR2 and LCDR3 set forth in SEQ ID NOs: 10, 11 and 12 respectively, or the CDRs having at least 85% sequence identity to the amino acid sequences set forth in SEQ ID NOs: 10, 11 and 12;
   the heavy chain variable region comprises HCDR1, HCDR2, and HCDR3 set forth in SEQ ID NOs: 13, 14 and 15 respectively, or the CDRs having at least 85% sequence identity to the amino acid sequences set forth in SEQ ID NOs: 13, 14 and 15; and the light chain variable region comprises LCDR1, LCDR2 and LCDR3 set forth in SEQ ID NOs: 16, 18 and 19 respectively, or the CDRs having at least 85% sequence identity to the amino acid sequences set forth in SEQ ID NOs: 16, 18 and 19;
   the heavy chain variable region comprises HCDR1, HCDR2, and HCDR3 set forth in SEQ ID NOs: 13, 14 and 15 respectively, or the CDRs having at least 85% sequence identity to the amino acid sequences set forth in SEQ ID NOs: 13, 14 and 15; and the light chain variable region comprises LCDR1, LCDR2 and LCDR3 set forth in SEQ ID NOs: 17, 18 and 19 respectively, or the CDRs having at least 85% sequence identity to the amino acid sequences set forth in SEQ ID NOs: 17, 18 and 19; or
   the heavy chain variable region comprises HCDR1, HCDR2, and HCDR3 set forth in SEQ ID NOs: 20, 21 and 22 respectively, or the CDRs having at least 85% sequence identity to the amino acid sequences set forth in SEQ ID NOs: 20, 21 and 22; and the light chain variable region comprises LCDR1, LCDR2 and LCDR3 set forth in SEQ ID NOs: 23, 24 and 25 respectively, or the CDRs having at least 85% sequence identity to the amino acid sequences set forth in SEQ ID NOs: 23, 24 and 25.
3. The anti-B7-H3 antibody or antigen-binding fragment thereof according to claim 1 or 2, wherein the antibody is a murine-derived monoclonal antibody, a human-murine chimeric antibody, or a humanized antibody.
4. The anti-B7-H3 antibody or antigen-binding fragment thereof according to any one of claims 1-3, wherein
   (1) the amino acid sequence of the heavy chain variable region is selected from the group consisting of:
      (b1) amino acid sequences as set forth in SEQ ID NO: 26, SEQ ID NO: 28, SEQ ID NO: 30 and SEQ ID NO: 35,
      (b2) amino acid sequences derived from the amino acid sequences set forth in (b1) by substitution, deletion or addition of one or more amino acids and functionally identical or similar to the amino acid sequences set forth in (b1), and
      (b3) amino acid sequences having at least 80% sequence identity to the amino acid sequences set forth in (b1); and
   (2) the amino acid sequence of the light chain variable region is selected from the group consisting of:
      (b4) amino acid sequences as set forth in SEQ ID NO: 27, SEQ ID NO: 29, SEQ ID NO: 31, SEQ ID NO: 32 and SEQ ID NO: 36,
      (b5) amino acid sequences derived from the amino acid sequences set forth in (b4) by substitution, deletion or addition of one or more amino acids and functionally identical or similar to the amino acid sequences set forth in (b4), and
      (b6) amino acid sequences having at least 80% sequence identity to the amino acid sequences set forth in (b4).
5. The anti-B7-H3 antibody or antigen-binding fragment thereof according to claim 4, wherein the antibody further comprises a heavy chain constant region of murine-derived IgG1, IgG2, or a variant thereof, and a light chain constant region of murine-derived kappa chain or a variant thereof.
6. The anti-B7-H3 antibody or antigen-binding fragment thereof according to any one of claims 1-3, wherein
   the amino acid sequence of the heavy chain variable region is selected from the group consisting of SEQ ID NO: 26, the amino acid sequence derived from SEQ ID NO: 26 by substitution, deletion or addition of one or more amino acids and functionally identical to SEQ ID NO: 26, and the amino acid sequence having at least 85% sequence identity to SEQ ID NO: 26; and the amino acid sequence of the light chain variable region is selected from the group consisting of SEQ ID NO: 27, the amino acid sequence derived from SEQ ID NO: 27 by substitution, deletion or addition of one or more amino acids and functionally identical to SEQ ID NO: 27, and the amino acid sequence having at least 85% sequence identity to SEQ ID NO: 27;
   the amino acid sequence of the heavy chain variable region is selected from the group consisting of SEQ ID NO: 28, the amino acid sequence derived from SEQ ID NO: 28 by substitution, deletion or addition of one or more amino acids and functionally identical to SEQ ID NO: 28, and the amino acid sequence having at least 85% sequence identity to SEQ ID NO: 28; and the amino acid sequence of the light chain variable region is selected from the group consisting of SEQ ID NO: 29, the amino acid sequence derived from SEQ ID NO: 29 by substitution, deletion or addition of one or more amino acids and functionally identical to SEQ ID NO: 29, and the amino acid sequence having at least 85% sequence identity to SEQ ID NO: 29;
   the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30; and the amino acid sequence of the light chain variable region is selected from the group consisting of SEQ ID NO: 31, the amino acid sequence derived from SEQ ID NO: 31 by substitution, deletion or addition of one or more amino acids and functionally identical to SEQ ID NO: 31, and the amino acid sequence having at least 85% sequence identity to SEQ ID NO: 31;
   the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30; and the amino acid sequence of the light chain variable region is selected from the group consisting of SEQ ID NO: 32, the amino acid sequence derived from SEQ ID NO: 32 by substitution, deletion or addition of one or more amino acids and functionally identical to SEQ ID NO: 32, and the amino acid sequence having at least 85% sequence identity to SEQ ID NO: 32; or
   the amino acid sequence of the heavy chain variable region is selected from the group consisting of SEQ ID NO: 35, the amino acid sequence derived from SEQ ID NO: 35 by substitution, deletion or addition of one or more amino acids and functionally identical to SEQ ID NO: 35, and the amino acid sequence having at least 85% sequence identity to SEQ ID NO: 35; and the amino acid sequence of the light chain variable region is selected from the group consisting of SEQ ID NO: 36, the amino acid sequence derived from SEQ ID NO: 36 by substitution, deletion or addition of one or more amino acids and functionally identical to SEQ ID NO: 36, and the amino acid sequence having at least 85% sequence identity to SEQ ID NO: 36.
7. The anti-B7-H3 antibody or antigen-binding fragment thereof according to any one of claims 1-3, wherein the anti-B7-H3 antibody is a humanized antibody, wherein
   (1) the amino acid sequence of the heavy chain variable region is selected from the group consisting of:
      (c1) amino acid sequences as set forth in SEQ ID NO: 45, SEQ ID NO: 46, SEQ ID NO: 47, SEQ ID NO: 48, SEQ ID NO: 52, SEQ ID NO: 53, SEQ ID NO: 54, SEQ ID NO: 55, SEQ ID NO: 56, SEQ ID NO: 57, SEQ ID NO: 63, SEQ ID NO: 64, SEQ ID NO: 72 and SEQ ID NO: 73,
      (c2) amino acid sequences derived from the amino acid sequences set forth in (c1) by substitution, deletion or addition of one or more amino acids and functionally identical or similar to the amino acid sequences as set forth in (c1), and
      (c3) amino acid sequences having at least 80% sequence identity to the amino acid sequences set forth in (c1); and
   (2) the amino acid sequence of the light chain variable region is selected from the group consisting of:
      (c4) amino acid sequences as set forth in SEQ ID NO: 49, SEQ ID NO: 50, SEQ ID NO: 51, SEQ ID NO: 58, SEQ ID NO: 59, SEQ ID NO: 60, SEQ ID NO: 61, SEQ ID NO: 62, SEQ ID NO: 65, SEQ ID NO: 66, SEQ ID NO: 67, SEQ ID NO: 68, SEQ ID NO: 69, SEQ ID NO: 70, SEQ ID NO: 74, SEQ ID NO: 75, SEQ ID NO: 76, SEQ ID NO: 77, SEQ ID NO: 78 and SEQ ID NO: 79,
      (c5) amino acid sequences derived from the amino acid sequences set forth in (c4) by substitution, deletion or addition of one or more amino acids and functionally identical or similar to the amino acid sequences as set forth in (c4), and
      (c6) amino acid sequences having at least 80% sequence identity to the amino acid sequences set forth in (c4).
8. The anti-B7-H3 antibody or antigen-binding fragment thereof according to claim 7, wherein
   the amino acid sequence of the heavy chain variable region is selected from the group consisting of SEQ ID NO: 45, the amino acid sequence derived from SEQ ID NO: 45 by substitution, deletion or addition of one or more amino acids and functionally identical to SEQ ID NO: 45, and the amino acid sequence having at least 85% sequence identity to SEQ ID NO: 45; and the amino acid sequence of the light chain variable region is selected from the group consisting of SEQ ID NO: 49, the amino acid sequence derived from SEQ ID NO: 49 by substitution, deletion or addition of one or more amino acids and functionally identical to SEQ ID NO: 49, and the amino acid sequence having at least 85% sequence identity to SEQ ID NO: 49;
   the amino acid sequence of the heavy chain variable region is selected from the group consisting of SEQ ID NO: 52, the amino acid sequence derived from SEQ ID NO: 52 by substitution, deletion or addition of one or more amino acids and functionally identical to SEQ ID NO: 52, and the amino acid sequence having at least 85% sequence identity to SEQ ID NO: 52; and the amino acid sequence of the light chain variable region is selected from the group consisting of SEQ ID NO: 59, the amino acid sequence derived from SEQ ID NO: 59 by substitution, deletion or addition of one or more amino acids and functionally identical to SEQ ID NO: 59, and the amino acid sequence having at least 85% sequence identity to SEQ ID NO: 59;
   the amino acid sequence of the heavy chain variable region is selected from the group consisting of SEQ ID NO: 63, the amino acid sequence derived from SEQ ID NO: 63 by substitution, deletion or addition of one or more amino acids and functionally identical to SEQ ID NO: 63, and the amino acid sequence having at least 85% sequence identity to SEQ ID NO: 63; and the amino acid sequence of the light chain variable region is selected from the group consisting of SEQ ID NO: 65, the amino acid sequence derived from SEQ ID NO: 65 by substitution, deletion or addition of one or more amino acids and functionally identical to SEQ ID NO: 65, and the amino acid sequence having at least 85% sequence identity to SEQ ID NO: 65;
   the amino acid sequence of the heavy chain variable region is selected from the group consisting of SEQ ID NO: 64, the amino acid sequence derived from SEQ ID NO: 64 by substitution, deletion or addition of one or more amino acids and functionally identical to SEQ ID NO: 64, or the amino acid sequence having at least 85% sequence identity to SEQ ID NO: 64; and the amino acid sequence of the light chain variable region is selected from the group consisting of SEQ ID NO: 67, the amino acid sequence derived from SEQ ID NO: 67 by substitution, deletion or addition of one or more amino acids and functionally identical to SEQ ID NO: 67, and the amino acid sequence having at least 85% sequence identity to SEQ ID NO: 67; or
   the amino acid sequence of the heavy chain variable region is selected from the group consisting of SEQ ID NO: 72, the amino acid sequence derived from SEQ ID NO: 72 by substitution, deletion or addition of one or more amino acids and functionally identical to SEQ ID NO: 72, or the amino acid sequence having at least 85% sequence identity to SEQ ID NO: 72; and the amino acid sequence of the light chain variable region is selected from the group consisting of SEQ ID NO: 77, the amino acid sequence derived from SEQ ID NO: 77 by substitution, deletion or addition of one or more amino acids and functionally identical to SEQ ID NO: 77, and the amino acid sequence having at least 85% sequence identity to SEQ ID NO: 77.
9. An isolated nucleic acid encoding the anti-B7-H3 antibody or antigen-binding fragment thereof according to any one of claims 1-8.
10. The nucleic acid according to claim 9, wherein
   (1) the nucleotide sequence encoding the amino acid sequence of the heavy chain variable region is selected from the group consisting of SEQ ID NO: 80, SEQ ID NO: 82, SEQ ID NO: 84 and SEQ ID NO: 86; and
   (2) the nucleotide sequence encoding the amino acid sequence of the light chain variable region is selected from the group consisting of SEQ ID NO: 81, SEQ ID NO: 83, SEQ ID NO: 85 and SEQ ID NO: 87.
11. An expression vector comprising the nucleic acid according to claim 9 or 10.
12. A host cell, wherein the host cell is transformed with the expression vector according to claim 11, and wherein the host cell is selected from the group consisting of prokaryotic cells and eukaryotic cells, preferably mammalian cells.
13. A method for producing the anti-B7-H3 antibody or antigen-binding fragment thereof according to any one of claims 1-8, comprising
   expressing the antibody in the host cell according to claim 12, and
   isolating the antibody from the host cell.
14. A pharmaceutical composition comprising the anti-B7-H3 antibody or antigen-binding fragment thereof according to any one of claims 1-8 and a pharmaceutically acceptable carrier.
15. Use of the anti-B7-H3 antibody or antigen-binding fragment thereof according to any one of claims 1-8 or the pharmaceutical composition according to claim 14 in the manufacture of a medicament for inhibiting B7-H3 activity.
16. The use according to claim 15, wherein the medicament for inhibiting B7-H3 activity is used to treat breast cancer, kidney cancer, lung cancer, gastric cancer, intestinal cancer, esophageal cancer, ovarian cancer, cervical cancer, bladder cancer, pancreatic cancer, glioma, leukemia, lymphoma, and/or melanoma.

## Claims

1. An anti-B7-H3 antibody or antigen-binding fragment thereof, comprising a heavy chain variable region and a light chain variable region, wherein
(1) the heavy chain variable region comprises HCDR1, HCDR2 and HCDR3, selected from the group consisting of:
(a1) amino acid sequences as set forth in SEQ ID NOs: 1, 2 and 3,
(a2) amino acid sequences as set forth in SEQ ID NOs: 7, 8 and 9,
(a3) amino acid sequences as set forth in SEQ ID NOs: 13, 14 and 15,
(a4) amino acid sequences as set forth in SEQ ID NOs: 20, 21 and 22, and
(a5) CDRs having at least 85% sequence identity to the amino acid sequences set forth in (a1), (a2), (a3), or (a4); and
(2) the light chain variable region comprises LCDR1, LCDR2 and LCDR3, selected from the group consisting of:
(a6) amino acid sequences as set forth in SEQ ID NOs: 4, 5 and 6,
(a7) amino acid sequences as set forth in SEQ ID NOs: 10, 11 and 12,
(a8) amino acid sequences as set forth in SEQ ID NOs: 16, 18 and 19,
(a9) amino acid sequences as set forth in SEQ ID NOs: 17, 18 and 19,
(a10) amino acid sequences as set forth in SEQ ID NOs: 23, 24 and 25, and
(a11) CDRs having at least 85% sequence identity to the amino acid sequences set forth in (a6), (a7), (a8), (a9), or (a10).

2. The anti-B7-H3 antibody or antigen-binding fragment thereof according to claim 1, wherein
the heavy chain variable region comprises HCDR1, HCDR2, and HCDR3 set forth in SEQ ID NOs: 1, 2 and 3 respectively, or the CDRs having at least 85% sequence identity to the amino acid sequences set forth in SEQ ID NOs: 1, 2 and 3; and the light chain variable region comprises LCDR1, LCDR2 and LCDR3 set forth in SEQ ID NOs: 4, 5 and 6 respectively, or the CDRs having at least 85% sequence identity to the amino acid sequences set forth in SEQ ID NOs: 4, 5 and 6;
the heavy chain variable region comprises HCDR1, HCDR2, and HCDR3 set forth in SEQ ID NOs: 7, 8 and 9 respectively, or the CDRs having at least 85% sequence identity to the amino acid sequences set forth in SEQ ID NOs: 7, 8 and 9; and the light chain variable region comprises LCDR1, LCDR2 and LCDR3 set forth in SEQ ID NOs: 10, 11 and 12 respectively, or the CDRs having at least 85% sequence identity to the amino acid sequences set forth in SEQ ID NOs: 10, 11 and 12;
the heavy chain variable region comprises HCDR1, HCDR2, and HCDR3 set forth in SEQ ID NOs: 13, 14 and 15 respectively, or the CDRs having at least 85% sequence identity to the amino acid sequences set forth in SEQ ID NOs: 13, 14 and 15; and the light chain variable region comprises LCDR1, LCDR2 and LCDR3 set forth in SEQ ID NOs: 16, 18 and 19 respectively, or the CDRs having at least 85% sequence identity to the amino acid sequences set forth in SEQ ID NOs: 16, 18 and 19;
the heavy chain variable region comprises HCDR1, HCDR2, and HCDR3 set forth in SEQ ID NOs: 13, 14 and 15 respectively, or the CDRs having at least 85% sequence identity to the amino acid sequences set forth in SEQ ID NOs: 13, 14 and 15; and the light chain variable region comprises LCDR1, LCDR2 and LCDR3 set forth in SEQ ID NOs: 17, 18 and 19 respectively, or the CDRs having at least 85% sequence identity to the amino acid sequences set forth in SEQ ID NOs: 17, 18 and 19; or
the heavy chain variable region comprises HCDR1, HCDR2, and HCDR3 set forth in SEQ ID NOs: 20, 21 and 22 respectively, or the CDRs having at least 85% sequence identity to the amino acid sequences set forth in SEQ ID NOs: 20, 21 and 22; and the light chain variable region comprises LCDR1, LCDR2 and LCDR3 set forth in SEQ ID NOs: 23, 24 and 25 respectively, or the CDRs having at least 85% sequence identity to the amino acid sequences set forth in SEQ ID NOs: 23, 24 and 25.

3. The anti-B7-H3 antibody or antigen-binding fragment thereof according to claim 1 or 2, wherein the antibody is a murine-derived monoclonal antibody, a human-murine chimeric antibody, or a humanized antibody.

4. The anti-B7-H3 antibody or antigen-binding fragment thereof according to any one of claims 1-3, wherein
(1) the amino acid sequence of the heavy chain variable region is selected from the group consisting of:
(b1) amino acid sequences as set forth in SEQ ID NO: 26, SEQ ID NO: 28, SEQ ID NO: 30 and SEQ ID NO: 35,
(b2) amino acid sequences derived from the amino acid sequences set forth in (b1) by substitution, deletion or addition of one or more amino acids and functionally identical or similar to the amino acid sequences set forth in (b1), and
(b3) amino acid sequences having at least 80% sequence identity to the amino acid sequences set forth in (b1); and
(2) the amino acid sequence of the light chain variable region is selected from the group consisting of:
(b4) amino acid sequences as set forth in SEQ ID NO: 27, SEQ ID NO: 29, SEQ ID NO: 31, SEQ ID NO: 32 and SEQ ID NO: 36,
(b5) amino acid sequences derived from the amino acid sequences set forth in (b4) by substitution, deletion or addition of one or more amino acids and functionally identical or similar to the amino acid sequences set forth in (b4), and
(b6) amino acid sequences having at least 80% sequence identity to the amino acid sequences set forth in (b4).

5. The anti-B7-H3 antibody or antigen-binding fragment thereof according to claim 4, wherein the antibody further comprises a heavy chain constant region of murine-derived IgG1, IgG2, or a variant thereof, and a light chain constant region of murine-derived kappa chain or a variant thereof.

6. The anti-B7-H3 antibody or antigen-binding fragment thereof according to any one of claims 1-3, wherein
the amino acid sequence of the heavy chain variable region is selected from the group consisting of SEQ ID NO: 26, the amino acid sequence derived from SEQ ID NO: 26 by substitution, deletion or addition of one or more amino acids and functionally identical to SEQ ID NO: 26, and the amino acid sequence having at least 85% sequence identity to SEQ ID NO: 26; and the amino acid sequence of the light chain variable region is selected from the group consisting of SEQ ID NO: 27, the amino acid sequence derived from SEQ ID NO: 27 by substitution, deletion or addition of one or more amino acids and functionally identical to SEQ ID NO: 27, and the amino acid sequence having at least 85% sequence identity to SEQ ID NO: 27;
the amino acid sequence of the heavy chain variable region is selected from the group consisting of SEQ ID NO: 28, the amino acid sequence derived from SEQ ID NO: 28 by substitution, deletion or addition of one or more amino acids and functionally identical to SEQ ID NO: 28, and the amino acid sequence having at least 85% sequence identity to SEQ ID NO: 28; and the amino acid sequence of the light chain variable region is selected from the group consisting of SEQ ID NO: 29, the amino acid sequence derived from SEQ ID NO: 29 by substitution, deletion or addition of one or more amino acids and functionally identical to SEQ ID NO: 29, and the amino acid sequence having at least 85% sequence identity to SEQ ID NO: 29;
the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30; and the amino acid sequence of the light chain variable region is selected from the group consisting of SEQ ID NO: 31, the amino acid sequence derived from SEQ ID NO: 31 by substitution, deletion or addition of one or more amino acids and functionally identical to SEQ ID NO: 31, and the amino acid sequence having at least 85% sequence identity to SEQ ID NO: 31;
the amino acid sequence of the heavy chain variable region is selected from the group consisting of SEQ ID NO: 30, the amino acid sequence derived from SEQ ID NO: 30 by substitution, deletion or addition of one or more amino acids and functionally identical to SEQ ID NO: 30, and the amino acid sequence having at least 85% sequence identity to SEQ ID NO: 30; and the amino acid sequence of the light chain variable region is selected from the group consisting of SEQ ID NO: 32, the amino acid sequence derived from SEQ ID NO: 32 by substitution, deletion or addition of one or more amino acids and functionally identical to SEQ ID NO: 32, and the amino acid sequence having at least 85% sequence identity to SEQ ID NO: 32; or
the amino acid sequence of the heavy chain variable region is selected from the group consisting of SEQ ID NO: 35, the amino acid sequence derived from SEQ ID NO: 35 by substitution, deletion or addition of one or more amino acids and functionally identical to SEQ ID NO: 35, and the amino acid sequence having at least 85% sequence identity to SEQ ID NO: 35; and the amino acid sequence of the light chain variable region is selected from the group consisting of SEQ ID NO: 36, the amino acid sequence derived from SEQ ID NO: 36 by substitution, deletion or addition of one or more amino acids and functionally identical to SEQ ID NO: 36, and the amino acid sequence having at least 85% sequence identity to SEQ ID NO: 36.

7. The anti-B7-H3 antibody or antigen-binding fragment thereof according to any one of claims 1-3, wherein the anti-B7-H3 antibody is a humanized antibody, wherein
(1) the amino acid sequence of the heavy chain variable region is selected from the group consisting of:
(c1) amino acid sequences as set forth in SEQ ID NO: 45, SEQ ID NO: 46, SEQ ID NO: 47, SEQ ID NO: 48, SEQ ID NO: 52, SEQ ID NO: 53, SEQ ID NO: 54, SEQ ID NO: 55, SEQ ID NO: 56, SEQ ID NO: 57, SEQ ID NO: 63, SEQ ID NO: 64, SEQ ID NO: 72 and SEQ ID NO: 73,
(c2) amino acid sequences derived from the amino acid sequences set forth in (c1) by substitution, deletion or addition of one or more amino acids and functionally identical or similar to the amino acid sequences as set forth in (c1), and
(c3) amino acid sequences having at least 80% sequence identity to the amino acid sequences set forth in (c1); and
(2) the amino acid sequence of the light chain variable region is selected from the group consisting of:
(c4) amino acid sequences as set forth in SEQ ID NO: 49, SEQ ID NO: 50, SEQ ID NO: 51, SEQ ID NO: 58, SEQ ID NO: 59, SEQ ID NO: 60, SEQ ID NO: 61, SEQ ID NO: 62, SEQ ID NO: 65, SEQ ID NO: 66, SEQ ID NO: 67, SEQ ID NO: 68, SEQ ID NO: 69, SEQ ID NO: 70, SEQ ID NO: 74, SEQ ID NO: 75, SEQ ID NO: 76, SEQ ID NO: 77, SEQ ID NO: 78 and SEQ ID NO: 79,
(c5) amino acid sequences derived from the amino acid sequences set forth in (c4) by substitution, deletion or addition of one or more amino acids and functionally identical or similar to the amino acid sequences as set forth in (c4), and
(c6) amino acid sequences having at least 80% sequence identity to the amino acid sequences set forth in (c4).

8. The anti-B7-H3 antibody or antigen-binding fragment thereof according to claim 7, wherein
the amino acid sequence of the heavy chain variable region is selected from the group consisting of SEQ ID NO: 45, the amino acid sequence derived from SEQ ID NO: 45 by substitution, deletion or addition of one or more amino acids and functionally identical to SEQ ID NO: 45, and the amino acid sequence having at least 85% sequence identity to SEQ ID NO: 45; and the amino acid sequence of the light chain variable region is selected from the group consisting of SEQ ID NO: 49, the amino acid sequence derived from SEQ ID NO: 49 by substitution, deletion or addition of one or more amino acids and functionally identical to SEQ ID NO: 49, and the amino acid sequence having at least 85% sequence identity to SEQ ID NO: 49;
the amino acid sequence of the heavy chain variable region is selected from the group consisting of SEQ ID NO: 52, the amino acid sequence derived from SEQ ID NO: 52 by substitution, deletion or addition of one or more amino acids and functionally identical to SEQ ID NO: 52, and the amino acid sequence having at least 85% sequence identity to SEQ ID NO: 52; and the amino acid sequence of the light chain variable region is selected from the group consisting of SEQ ID NO: 59, the amino acid sequence derived from SEQ ID NO: 59 by substitution, deletion or addition of one or more amino acids and functionally identical to SEQ ID NO: 59, and the amino acid sequence having at least 85% sequence identity to SEQ ID NO: 59;
the amino acid sequence of the heavy chain variable region is selected from the group consisting of SEQ ID NO: 63, the amino acid sequence derived from SEQ ID NO: 63 by substitution, deletion or addition of one or more amino acids and functionally identical to SEQ ID NO: 63, and the amino acid sequence having at least 85% sequence identity to SEQ ID NO: 63; and the amino acid sequence of the light chain variable region is selected from the group consisting of SEQ ID NO: 65, the amino acid sequence derived from SEQ ID NO: 65 by substitution, deletion or addition of one or more amino acids and functionally identical to SEQ ID NO: 65, and the amino acid sequence having at least 85% sequence identity to SEQ ID NO: 65;
the amino acid sequence of the heavy chain variable region is selected from the group consisting of SEQ ID NO: 64, the amino acid sequence derived from SEQ ID NO: 64 by substitution, deletion or addition of one or more amino acids and functionally identical to SEQ ID NO: 64, or the amino acid sequence having at least 85% sequence identity to SEQ ID NO: 64; and the amino acid sequence of the light chain variable region is selected from the group consisting of SEQ ID NO: 67, the amino acid sequence derived from SEQ ID NO: 67 by substitution, deletion or addition of one or more amino acids and functionally identical to SEQ ID NO: 67, and the amino acid sequence having at least 85% sequence identity to SEQ ID NO: 67; or
the amino acid sequence of the heavy chain variable region is selected from the group consisting of SEQ ID NO: 72, the amino acid sequence derived from SEQ ID NO: 72 by substitution, deletion or addition of one or more amino acids and functionally identical to SEQ ID NO: 72, or the amino acid sequence having at least 85% sequence identity to SEQ ID NO: 72; and the amino acid sequence of the light chain variable region is selected from the group consisting of SEQ ID NO: 77, the amino acid sequence derived from SEQ ID NO: 77 by substitution, deletion or addition of one or more amino acids and functionally identical to SEQ ID NO: 77, and the amino acid sequence having at least 85% sequence identity to SEQ ID NO: 77.

9. An isolated nucleic acid encoding the anti-B7-H3 antibody or antigen-binding fragment thereof according to any one of claims 1-8.

10. The nucleic acid according to claim 9, wherein
(1) the nucleotide sequence encoding the amino acid sequence of the heavy chain variable region is selected from the group consisting of SEQ ID NO: 80, SEQ ID NO: 82, SEQ ID NO: 84 and SEQ ID NO: 86; and
(2) the nucleotide sequence encoding the amino acid sequence of the light chain variable region is selected from the group consisting of SEQ ID NO: 81, SEQ ID NO: 83, SEQ ID NO: 85 and SEQ ID NO: 87.

11. An expression vector comprising the nucleic acid according to claim 9 or 10.

12. A host cell, wherein the host cell is transformed with the expression vector according to claim 11, and wherein the host cell is selected from the group consisting of prokaryotic cells and eukaryotic cells, preferably mammalian cells.

13. A method for producing the anti-B7-H3 antibody or antigen-binding fragment thereof according to any one of claims 1-8, comprising
expressing the antibody in the host cell according to claim 12, and
isolating the antibody from the host cell.

14. A pharmaceutical composition comprising the anti-B7-H3 antibody or antigen-binding fragment thereof according to any one of claims 1-8 and a pharmaceutically acceptable carrier.

15. Use of the anti-B7-H3 antibody or antigen-binding fragment thereof according to any one of claims 1-8 or the pharmaceutical composition according to claim 14 in the manufacture of a medicament for inhibiting B7-H3 activity.

16. The use according to claim 15, wherein the medicament for inhibiting B7-H3 activity is used to treat breast cancer, kidney cancer, lung cancer, gastric cancer, intestinal cancer, esophageal cancer, ovarian cancer, cervical cancer, bladder cancer, pancreatic cancer, glioma, leukemia, lymphoma, and/or melanoma.


SEQUENCE LISTING

<110>  NANJING SANHOME PHARMACEUTICAL CO., LTD.

<120>  ANTI-B7-H3 ANTIBODY AND APPLICATION THEREOF

<130>  P22525EPPC

<150>  CN 201910872843.3
<151>  2019-09-16

<160>  96

<170>  PatentIn version 3.3

<210>  1
<211>  8
<212>  PRT
<213>  Artificial sequence

<220>
<223>  HCDR1

<400>  1

Gly Phe Thr Phe Thr Asp Tyr Val
1               5


<210>  2
<211>  8
<212>  PRT
<213>  Artificial sequence

<220>
<223>  HCDR2

<400>  2

Ile Tyr Pro Gly Gly Gly Phe Ile
1               5


<210>  3
<211>  8
<212>  PRT
<213>  Artificial sequence

<220>
<223>  HCDR3

<400>  3

Phe Pro Lys Tyr Tyr Phe Asp Tyr
1               5


<210>  4
<211>  11
<212>  PRT
<213>  Artificial sequence

<220>

<223>  LCDR1

<400>  4

Gln Ser Ile Val His Ser Asn Arg Tyr Thr Tyr
1               5                   10


<210>  5
<211>  3
<212>  PRT
<213>  Artificial sequence

<220>
<223>  LCDR2

<400>  5

Gly Val Ser
1


<210>  6
<211>  9
<212>  PRT
<213>  Artificial sequence

<220>
<223>  LCDR3

<400>  6

Phe Gln Ser Thr Leu Phe Pro Trp Thr
1               5


<210>  7
<211>  8
<212>  PRT
<213>  Artificial sequence

<220>
<223>  HCDR1

<400>  7

Gly Tyr Thr Phe Thr Glu Tyr Thr
1               5


<210>  8
<211>  8
<212>  PRT
<213>  Artificial sequence

<220>
<223>  HCDR2

<400>  8

Leu Asn Pro Asn Thr Gly Gly Thr
1               5

<210>  9
<211>  11
<212>  PRT
<213>  Artificial sequence

<220>
<223>  HCDR3

<400>  9

Ala Arg Lys Gly Gly Trp Phe Phe Leu Asp Tyr
1               5                   10


<210>  10
<211>  5
<212>  PRT
<213>  Artificial sequence

<220>
<223>  LCDR1

<400>  10

Ser Ser Val Ser Tyr
1               5


<210>  11
<211>  3
<212>  PRT
<213>  Artificial sequence

<220>
<223>  LCDR2

<400>  11

Asp Thr Ser
1


<210>  12
<211>  9
<212>  PRT
<213>  Artificial sequence

<220>
<223>  LCDR3

<400>  12

Gln Gln Trp Ser Ser Asn Pro Pro Thr
1               5


<210>  13
<211>  8
<212>  PRT
<213>  Artificial sequence

<220>

<223>  HCDR1

<400>  13

Gly Phe Thr Phe Ser Ser Phe Gly
1               5


<210>  14
<211>  8
<212>  PRT
<213>  Artificial sequence

<220>
<223>  HCDR2

<400>  14

Ile Asn Ser Gly Asp Asn Tyr Ser
1               5


<210>  15
<211>  16
<212>  PRT
<213>  Artificial sequence

<220>
<223>  HCDR3

<400>  15

Ala Arg Pro His Ile Phe Tyr Tyr Gly Ser Pro Ala Trp Phe Ala Tyr
1               5                   10                  15


<210>  16
<211>  11
<212>  PRT
<213>  Artificial sequence

<220>
<223>  LCDR1

<400>  16

Gln Ser Leu Val His Ser Asn Gly Asn Thr Tyr
1               5                   10


<210>  17
<211>  11
<212>  PRT
<213>  Artificial sequence

<220>
<223>  LCDR1

<400>  17

Gln Ser Leu Val His Ser Asn Lys Asn Thr Tyr
1               5                   10

<210>  18
<211>  3
<212>  PRT
<213>  Artificial sequence

<220>
<223>  LCDR2

<400>  18

Lys Val Ser
1


<210>  19
<211>  9
<212>  PRT
<213>  Artificial sequence

<220>
<223>  LCDR3

<400>  19

Ser Gln Ser Thr His Val Pro Leu Thr
1               5


<210>  20
<211>  8
<212>  PRT
<213>  Artificial sequence

<220>
<223>  HCDR1

<400>  20

Gly Phe Thr Phe Ser Asp Tyr Gly
1               5


<210>  21
<211>  8
<212>  PRT
<213>  Artificial sequence

<220>
<223>  HCDR2

<400>  21

Ile Ser Ser Gly Ser Asn Thr Ile
1               5


<210>  22
<211>  14
<212>  PRT
<213>  Artificial sequence

<220>

<223>  HCDR3

<400>  22

Ala Thr Met Val Thr Arg Trp Phe Tyr Tyr Ala Met Asp Tyr
1               5                   10


<210>  23
<211>  5
<212>  PRT
<213>  Artificial sequence

<220>
<223>  LCDR1

<400>  23

Ser Ser Val Ser Tyr
1               5


<210>  24
<211>  3
<212>  PRT
<213>  Artificial sequence

<220>
<223>  LCDR2

<400>  24

Asp Thr Ser
1


<210>  25
<211>  9
<212>  PRT
<213>  Artificial sequence

<220>
<223>  LCDR3

<400>  25

Gln Gln Trp Ser Ser Asn Pro Pro Thr
1               5


<210>  26
<211>  117
<212>  PRT
<213>  Artificial sequence

<220>
<223>  SHS007-17 VH

<400>  26

Gln Val His Leu Gln Gln Ser Gly Pro Glu Leu Val Lys Pro Gly Ala
1               5                   10                  15

Ser Val Lys Met Ser Cys Lys Ala Ser Gly Phe Thr Phe Thr Asp Tyr
            20                  25                  30

Val Val Ser Trp Val Lys Gln Arg Thr Gly Gln Gly Leu Glu Trp Ile
        35                  40                  45

Gly Glu Ile Tyr Pro Gly Gly Gly Phe Ile His Tyr Asn Glu Lys Phe
    50                  55                  60

Lys Gly Lys Ala Thr Leu Thr Ala Asp Lys Ser Ser Asn Thr Ala Tyr
65                  70                  75                  80

Met Gln Leu Thr Ser Leu Thr Ser Glu Asp Ser Ala Val Tyr Phe Cys
                85                  90                  95

Ala Arg Phe Pro Lys Tyr Tyr Phe Asp Tyr Trp Gly Gln Gly Thr Thr
            100                 105                 110

Leu Thr Val Ser Ser
        115

<210> 27
<211> 112
<212> PRT
<213> Artificial sequence

<220>
<223> SHS007-17 VL

<400> 27

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Ile Val His Ser
            20                  25                  30

Asn Arg Tyr Thr Tyr Leu Glu Trp Tyr Leu Gln Lys Pro Gly Gln Ser
        35                  40                  45

Leu Lys Leu Leu Ile Tyr Gly Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Met Gly Val Tyr Tyr Cys Phe Gln Ser
                85                  90                  95

Thr Leu Phe Pro Trp Thr Phe Gly Gly Gly Thr Lys Leu Glu Ile Lys
            100                 105                 110

<210>  28
<211>  118
<212>  PRT
<213>  Artificial sequence

<220>
<223>  SHS007-39 VH

<400>  28

Glu Val Gln Leu Gln Gln Ser Gly Pro Glu Leu Val Lys Pro Gly Ala
1               5                   10                  15

Ser Val Lys Ile Ser Cys Lys Thr Ser Gly Tyr Thr Phe Thr Glu Tyr
            20                  25                  30

Thr Ile His Trp Met Lys Gln Ser His Gly Lys Ser Leu Glu Trp Ile
        35                  40                  45

Gly Gly Leu Asn Pro Asn Thr Gly Gly Thr Thr Tyr Asn Gln Lys Phe
    50                  55                  60

Lys Asp Lys Ala Thr Leu Thr Val Asp Lys Ser Ser Ser Thr Ala Tyr
65                  70                  75                  80

Met Ala Leu Arg Ser Leu Thr Ser Glu Asp Ser Ala Val Tyr Tyr Cys
                85                  90                  95

Ala Arg Lys Gly Gly Trp Phe Phe Leu Asp Tyr Trp Gly Gln Gly Thr
            100                 105                 110

Thr Leu Ile Val Ser Ser
        115

<210>  29
<211>  106
<212>  PRT
<213>  Artificial sequence

<220>
<223>  SHS007-39 VL

<400>  29

Gln Ile Val Leu Thr Gln Ser Pro Ala Ile Met Ser Ala Ser Pro Arg
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Arg Gly Ser
    50                  55                  60

Gly Ser Gly Thr Ser Tyr Ser Leu Thr Ile Ser Asn Met Glu Ala Glu
65                  70                  75                  80

Asp Ala Ala Thr Tyr Tyr Cys Gln Gln Trp Ser Ser Asn Pro Pro Thr
                85                  90                  95

Phe Gly Gly Gly Thr Lys Val Glu Ile Lys
            100                 105

<210>  30
<211>  123
<212>  PRT
<213>  Artificial sequence

<220>
<223>  SHS007-51 VH

<400>  30

Glu Val Gln Leu Val Glu Ser Gly Gly Asp Ser Val Lys Pro Gly Gly
1               5                   10                  15

Ser Leu Lys Leu Ser Cys Ala Ala Ser Gly Phe Thr Phe Ser Ser Phe
            20                  25                  30

Gly Met Ser Trp Val Arg Gln Thr Pro Asp Lys Arg Leu Glu Trp Val
        35                  40                  45

Ala Thr Ile Asn Ser Gly Asp Asn Tyr Ser Tyr Ser Pro Asp Ser Val
    50                  55                  60

Lys Gly Arg Phe Thr Ile Ser Arg Asp Asn Ala Lys Asn Ile Leu Tyr
65                  70                  75                  80

Leu Gln Met Ser Ser Leu Lys Ser Glu Asp Thr Ala Met Tyr Tyr Cys
                85                  90                  95

Ala Arg Pro His Ile Phe Tyr Tyr Gly Ser Pro Ala Trp Phe Ala Tyr
            100                 105                 110

Trp Gly Gln Gly Thr Leu Val Thr Val Ser Ala
        115                 120

<210>  31
<211>  112
<212>  PRT
<213>  Artificial sequence

<220>
<223>  SHS007-51 VL

<400>  31

Asp Val Val Met Thr Gln Thr Pro Leu Ser Leu Pro Val Ser Leu Gly
1               5                   10                  15

Asp Gln Ala Ser Val Ser Cys Arg Ser Arg Gln Ser Leu Val His Ser
            20                  25                  30

Asn Gly Asn Thr Tyr Leu His Trp Tyr Leu Gln Arg Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Leu Lys
            100                 105                 110


<210>  32
<211>  112
<212>  PRT
<213>  Artificial sequence

<220>
<223>  SHS007-51-G34K-CHI VL

<400>  32

Asp Val Val Met Thr Gln Thr Pro Leu Ser Leu Pro Val Ser Leu Gly
1               5                   10                  15

Asp Gln Ala Ser Val Ser Cys Arg Ser Arg Gln Ser Leu Val His Ser
            20                  25                  30

Asn Lys Asn Thr Tyr Leu His Trp Tyr Leu Gln Arg Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Leu Lys
            100                 105                 110

<210>  33
<211>  115
<212>  PRT
<213>  Artificial sequence

<220>
<223>  SHS007-59 VH

<400>  33

Glu Val Gln Leu Gln Gln Ser Gly Pro Glu Leu Val Lys Pro Gly Thr
1               5                   10                  15

Ser Val Lys Ile Pro Cys Lys Ala Ser Gly Tyr Thr Phe Thr Asp Tyr
            20                  25                  30

His Met Asp Trp Val Lys Gln Ser His Gly Lys Ser Leu Glu Trp Ile
        35                  40                  45

Gly Asp Ile Asn Pro Asn Asn Gly Gly Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Gly Leu Thr Ser Glu Asp Thr Ala Val Tyr Tyr Cys
                85                  90                  95

Ala Arg Gly Gly Leu His Asp Tyr Trp Gly Pro Gly Thr Thr Leu Thr
            100                 105                 110

Val Ser Ser
        115

<210>  34
<211>  107
<212>  PRT
<213>  Artificial sequence

<220>
<223>  SHS007-59 VL

<400>  34

Asp Ile Val Met Thr Gln Ser Gln Lys Phe Met Ser Thr Lys Val Gly
1               5                   10                  15

Asp Arg Val Ser Ile Thr Cys Lys Ala Ser Gln Ser Val Gly Thr Ala
            20                  25                  30

Val Ala Trp Tyr Gln Gln Lys Pro Gly Gln Ser Pro Lys Leu Leu Ile
        35                  40                  45

Tyr Ser Thr Ser Asn Arg Tyr Thr Gly Val Pro Asp Arg Phe Thr Asn
    50                  55                  60

Asn Ala Ser Gly Thr Asp Phe Thr Leu Thr Ile Ser Asn Val Gln Ser
65                  70                  75                  80

Ala Asp Leu Ala Asp Tyr Phe Cys Gln Gln Tyr Gly Asn Tyr Pro Leu
                85                  90                  95

Thr Phe Gly Gly Gly Thr Lys Leu Glu Ile Lys
            100                 105


<210>  35
<211>  121
<212>  PRT
<213>  Artificial sequence

<220>
<223>  SHS007-65 VH

<400>  35

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Asp Tyr
            20                  25                  30

Gly Ile His Trp Val Arg Gln Ala Pro Glu Lys Gly Leu Glu Trp Val
        35                  40                  45

Ala Tyr Ile Ser Ser Gly Ser Asn Thr Ile Tyr Tyr Ala Asp Thr Val
    50                  55                  60

Lys Gly Arg Phe Thr Ile Ser Arg Asp Asn Ala Lys Asn Thr Leu Phe
65                  70                  75                  80

Leu Gln Met Thr Ser Leu Arg Ser Glu Asp Thr Ala Ile Tyr Tyr Cys
                85                  90                  95

Ala Thr Met Val Thr Arg Trp Phe Tyr Tyr Ala Met Asp Tyr Trp Gly
            100                 105                 110

Gln Gly Thr Ser Val Thr Val Ser Ser
        115                 120

<210>  36
<211>  106
<212>  PRT
<213>  Artificial sequence

<220>
<223>  SHS007-65 VL

<400>  36

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Tyr Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Asn Asn Met Glu Ala Glu
65                  70                  75                  80

Asp Ala Ala Thr Tyr Tyr Cys Gln Gln Trp Ser Ser Asn Pro Pro Thr
                85                  90                  95

Phe Gly Ala Gly Thr Lys Leu Glu Leu Lys
            100                 105

<210>  37
<211>  121
<212>  PRT
<213>  Artificial sequence

<220>
<223>  SHS007-72 VH

<400>  37

Gln Val Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Tyr Trp Val Lys Gln Ala Pro Gly Glu Gly Leu Lys Trp Met
        35                  40                  45

Gly Trp Ile Asn Thr His Thr Gly Glu Pro Thr Tyr Gly Asp Asp Phe
    50                  55                  60

Lys Gly Arg Phe Ala Phe Ser Leu Glu Thr Pro Ala Thr Ile Ala Phe
65                  70                  75                  80

Leu Glu Ile Ser Ile Leu Lys Asn Glu Asp Thr Ala Thr Tyr Phe Cys
                85                  90                  95

Ala Arg Lys Ser Phe Gly Asp Phe Ile Tyr Ala Met Asp Phe Trp Gly
            100                 105                 110

Gln Gly Thr Ser Val Thr Val Ser Ser
        115                 120

<210>  38
<211>  107
<212>  PRT
<213>  Artificial sequence

<220>
<223>  SHS007-72 VL

<400>  38

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Gly Lys Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Leu Gly Val Pro Ser Arg Phe Ser Gly
    50                  55                  60

Ser Gly Ser Gly Thr Asp Phe Ser Leu Thr Ile Ser Asn Leu Glu Gln
65                  70                  75                  80

Glu Asp Thr Ala Thr Tyr Phe Cys Gln Gln Gly Thr Thr Leu Pro Pro
                85                  90                  95

Thr Phe Gly Gly Gly Thr Lys Val Glu Ile Lys
            100                 105

<210>  39
<211>  123
<212>  PRT
<213>  Artificial sequence

<220>
<223>  SHS007-90 VH

<400>  39

Glu Val Gln Leu Gln Gln Ser Gly Pro Glu Leu Val Lys Pro Gly Ala
1               5                   10                  15

Ala Val Gln Met Ser Cys Gln Ala Ser Gly Tyr Thr Phe Thr Asn Tyr
            20                  25                  30

Val Ile His Trp Ile Gln Gln Lys Pro Gly Gln Gly Leu Glu Trp Val
        35                  40                  45

Gly Tyr Ile Asn Pro Tyr Asn Asp Gly Val Asn Tyr Asn Glu Lys Phe
    50                  55                  60

Lys Gly Lys Ala Thr Leu Thr Ser Asp Lys Ser Ser Asn Thr Thr Tyr
65                  70                  75                  80

Met Glu Phe Arg Ser Leu Thr Ser Glu Asp Ser Ala Val Tyr Phe Cys
                85                  90                  95

Ala Arg Trp Gly Asn Ser Tyr Gly His Tyr Gly Phe Ala Leu Asp Tyr
            100                 105                 110

Trp Gly Gln Gly Thr Ser Val Thr Val Ser Ser
        115                 120


<210>  40
<211>  106
<212>  PRT
<213>  Artificial sequence

<220>
<223>  SHS007-90 VL

<400>  40

Gln Ile Val Leu Ser Gln Ser Pro Thr Ile Leu Ser Ala Ser Pro Gly
1               5                   10                  15

Glu Lys Val Thr Met Thr Cys Arg Pro Thr Ser Ser Val Asn Tyr Ile
            20                  25                  30

His Trp Phe Gln Gln Lys Pro Gly Ser Ser Pro Lys Pro Trp Ile Tyr
        35                  40                  45

Ala Ser Ser Ser Leu Val Ser Gly Val Pro Thr Arg Phe Ser Gly Arg
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Ser Gly Asn Pro Leu Thr
                85                  90                  95

Phe Gly Ala Gly Thr Lys Leu Glu Val Arg
            100                 105

<210>  41
<211>  121
<212>  PRT
<213>  Artificial sequence

<220>
<223>  SHS007-95 VH

<400>  41

Glu Val Lys Leu Val Glu Ser Gly Gly Glu Leu Val Lys Pro Gly Gly
1               5                   10                  15

Ser Leu Lys Leu Ser Cys Ala Ala Ser Gly Phe Thr Leu Ser Ser Tyr
            20                  25                  30

Val Met Ser Trp Val Arg Gln Thr Pro Glu Lys Arg Leu Glu Trp Val
        35                  40                  45

Ala Ala Ile Ser Arg Tyr Gly Asp Pro Thr Asn Phe Pro Asp Asn Met
    50                  55                  60

Arg Asp Arg Phe Thr Ile Ser Arg Asp Asn Ala Lys Asn Thr Leu Ser
65                  70                  75                  80

Leu Gln Met Ser Ser Leu Arg Ser Glu Asp Thr Ala Leu Tyr Phe Cys
                85                  90                  95

Ala Arg His His Phe Asp Gly Thr Ser Trp Tyr Phe Asp Val Trp Gly
            100                 105                 110

Pro Gly Thr Thr Val Thr Val Ser Ser
        115                 120

<210>  42
<211>  108
<212>  PRT

<213>  Artificial sequence

<220>
<223>  SHS007-95 VL

<400>  42

Glu Ile Val Leu Thr Gln Ser Pro Ala Leu Met Ala Ala Ser Pro Gly
1               5                   10                  15

Glu Arg Val Thr Ile Thr Cys Ser Val Ser Ser Ser Ile Ser Ser Ile
            20                  25                  30

Asp Leu His Trp Tyr Gln Gln Lys Ser Gly Thr Phe Pro Lys Leu Trp
        35                  40                  45

Ile Tyr Gly Thr Ser Asn Leu Ala Ser Gly Val Pro Val Arg Phe Ser
    50                  55                  60

Gly Ser Gly Ser Gly Thr Ser Tyr Ser Leu Thr Ile Ser Thr Met Glu
65                  70                  75                  80

Ala Glu Asp Ala Ala Thr Tyr Tyr Cys Gln Gln Trp Ser Ser Phe Pro
                85                  90                  95

Leu Thr Phe Gly Ser Gly Thr Lys Leu Glu Ile Lys
            100                 105

<210>  43
<211>  119
<212>  PRT
<213>  Artificial sequence

<220>
<223>  SHS007-108 VH

<400>  43

Glu Val Gln Leu Val Glu Ser Gly Gly Gly Leu Val Gln Pro Gly Arg
1               5                   10                  15

Ser Leu Lys Leu Ser Cys Leu Ala Ser Gly Phe Thr Phe Asn Asn Tyr
            20                  25                  30

Gly Met Asn Trp Ile Arg Gln Ala Pro Gly Lys Gly Leu Glu Trp Val
        35                  40                  45

Ala Ser Ile Ser Ser Ser Ser Ser Tyr Ile His Tyr Gly Asp Thr Val
    50                  55                  60

Lys Gly Arg Phe Thr Ile Ser Arg Asp Asn Ala Lys Asn Thr Leu Tyr
65                  70                  75                  80

Leu Gln Val Thr Ser Leu Arg Ser Glu Asp Thr Ala Leu Tyr Tyr Cys
                85                  90                  95

Thr Arg Gly Leu Ser Tyr Val Gly Trp Phe Ala His Trp Gly Arg Gly
            100                 105                 110

Thr Leu Val Thr Val Ser Ser
        115

<210>  44
<211>  107
<212>  PRT
<213>  Artificial sequence

<220>
<223>  SHS007-108 VL

<400>  44

Asp Ile Gln Met Thr Gln Ser Pro Ala Ser Leu Ser Ala Ser Leu Glu
1               5                   10                  15

Glu Ile Val Thr Ile Thr Cys Gln Ser Ser Gln Asn Ile Gly Asn Trp
            20                  25                  30

Leu Ala Trp Tyr Gln Gln Lys Pro Gly Lys Ser Pro Gln Leu Leu Ile
        35                  40                  45

Tyr Gly Ala Thr Thr Leu Ala Asp Gly Val Pro Ser Arg Phe Ser Gly
    50                  55                  60

Ser Arg Ser Gly Thr Gln Tyr Ser Leu Lys Ile Ser Arg Leu Gln Val
65                  70                  75                  80

Glu Asp Ile Gly Ile Tyr Tyr Cys Gln Gln Ala Ser Ser Thr Ser Trp
                85                  90                  95

Thr Phe Gly Gly Gly Thr Lys Leu Glu Leu Lys
            100                 105

<210>  45
<211>  117
<212>  PRT
<213>  Artificial sequence

<220>
<223>  Humanized SHS007-17-CHI VH

<400>  45

Gln Val Gln Leu Val Gln Ser Gly Ala Glu Val Lys Lys Pro Gly Ser

1               5                   10                  15

Ser Val Lys Val Ser Cys Lys Ala Ser Gly Phe Thr Phe Thr Asp Tyr
            20                  25                  30

Val Val Ser Trp Val Arg Gln Ala Pro Gly Gln Gly Leu Glu Trp Met
        35                  40                  45

Gly Glu Ile Tyr Pro Gly Gly Gly Phe Ile His Tyr Asn Glu Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Pro Lys Tyr Tyr Phe Asp Tyr Trp Gly Gln Gly Thr Thr
            100                 105                 110

Val Thr Val Ser Ser
        115

<210>  46
<211>  117
<212>  PRT
<213>  Artificial sequence

<220>
<223>  Humanized SHS007-17-CHI VH

<400>  46

Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Tyr
            20                  25                  30

Val Val Ser Trp Val Arg Gln Ala Pro Gly Gln Gly Leu Glu Trp Met
        35                  40                  45

Gly Glu Ile Tyr Pro Gly Gly Gly Phe Ile His Tyr Asn Glu Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Phe Pro Lys Tyr Tyr Phe Asp Tyr Trp Gly Gln Gly Thr Thr
            100                 105                 110

Val Thr Val Ser Ser
        115

<210>  47
<211>  117
<212>  PRT
<213>  Artificial sequence

<220>
<223>  Humanized SHS007-17-CHI VH

<400>  47

Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Tyr
            20                  25                  30

Val Val Ser Trp Val Arg Gln Ala Pro Gly Gln Gly Leu Glu Trp Ile
        35                  40                  45

Gly Glu Ile Tyr Pro Gly Gly Gly Phe Ile His Tyr Asn Glu Lys Phe
    50                  55                  60

Lys Gly Arg Ala Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Phe Pro Lys Tyr Tyr Phe Asp Tyr Trp Gly Gln Gly Thr Thr
            100                 105                 110

Val Thr Val Ser Ser
        115

<210>  48
<211>  117
<212>  PRT
<213>  Artificial sequence

<220>
<223>  Humanized SHS007-17-CHI VH

<400>  48

Gln Val Gln Leu Val Gln Ser Gly Ala Glu Val Lys Lys Pro Gly Ser

1               5                   10                  15

Ser Val Lys Met Ser Cys Lys Ala Ser Gly Phe Thr Phe Thr Asp Tyr
            20                  25                  30

Val Val Ser Trp Val Lys Gln Ala Pro Gly Gln Gly Leu Glu Trp Ile
        35                  40                  45

Gly Glu Ile Tyr Pro Gly Gly Gly Phe Ile His Tyr Asn Glu Lys Phe
    50                  55                  60

Lys Gly Arg Ala Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Phe Pro Lys Tyr Tyr Phe Asp Tyr Trp Gly Gln Gly Thr Thr
            100                 105                 110

Leu Thr Val Ser Ser
        115

<210>  49
<211>  112
<212>  PRT
<213>  Artificial sequence

<220>
<223>  Humanized SHS007-17-CHI VL

<400>  49

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Ile Val His Ser
            20                  25                  30

Asn Arg Tyr Thr Tyr Leu Glu Trp Tyr Leu Gln Lys Pro Gly Gln Ser
        35                  40                  45

Pro Gln Leu Leu Ile Tyr Gly Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ser
                85                  90                  95

Thr Leu Phe Pro Trp Thr Phe Gly Gln Gly Thr Lys Val Glu Ile Lys
            100                 105                 110

<210> 50
<211> 112
<212> PRT
<213> Artificial sequence

<220>
<223> Humanized SHS007-17-CHI VL

<400> 50

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Ile Val His Ser
            20                  25                  30

Asn Arg Tyr Thr Tyr Leu Glu Trp Tyr Leu Gln Lys Pro Gly Gln Ser
        35                  40                  45

Leu Gln Leu Leu Ile Tyr Gly Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ser
                85                  90                  95

Thr Leu Phe Pro Trp Thr Phe Gly Gln Gly Thr Lys Val Glu Ile Lys
            100                 105                 110

<210> 51
<211> 112
<212> PRT
<213> Artificial sequence

<220>
<223> Humanized SHS007-17-CHI VL

<400> 51

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Ile Val His Ser
            20                  25                  30

Asn Arg Tyr Thr Tyr Leu Glu Trp Tyr Leu Gln Lys Pro Gly Gln Ser

35                  40                  45

Leu Gln Leu Leu Ile Tyr Gly Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ser
                85                  90                  95

Thr Leu Phe Pro Trp Thr Phe Gly Gln Gly Thr Lys Leu Glu Ile Lys
            100                 105                 110

<210>  52
<211>  118
<212>  PRT
<213>  Artificial sequence

<220>
<223>  Humanized SHS007-39-CHI VH

<400>  52

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Tyr
            20                  25                  30

Thr Ile His Trp Val Arg Gln Ala Pro Gly Gln Gly Leu Glu Trp Met
        35                  40                  45

Gly Gly Leu Asn Pro Asn Thr Gly Gly Thr Thr Tyr Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Lys Gly Gly Trp Phe Phe Leu Asp Tyr Trp Gly Gln Gly Thr
            100                 105                 110

Leu Val Thr Val Ser Ser
        115

<210>  53
<211>  118

<212>  PRT
<213>  Artificial sequence

<220>
<223>  Humanized SHS007-39-CHI VH

<400>  53

Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Val Arg Gln Ala Pro Gly Gln Gly Leu Glu Trp Met
        35                  40                  45

Gly Gly Leu Asn Pro Asn Thr Gly Gly Thr Thr Tyr Asn Gln Lys Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Lys Gly Gly Trp Phe Phe Leu Asp Tyr Trp Gly Gln Gly Thr
            100                 105                 110

Leu Val Thr Val Ser Ser
        115


<210>  54
<211>  118
<212>  PRT
<213>  Artificial sequence

<220>
<223>  Humanized SHS007-39-CHI VH

<400>  54

Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Met Arg Gln Ala Pro Gly Gln Gly Leu Glu Trp Met
        35                  40                  45

Gly Gly Leu Asn Pro Asn Thr Gly Gly Thr Thr Tyr Asn Gln Lys Phe

50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Lys Gly Gly Trp Phe Phe Leu Asp Tyr Trp Gly Gln Gly Thr
            100                 105                 110

Leu Val Thr Val Ser Ser
        115

<210>  55
<211>  118
<212>  PRT
<213>  Artificial sequence

<220>
<223>  Humanized SHS007-39-CHI VH

<400>  55

Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Met Arg Gln Ala Pro Gly Gln Gly Leu Glu Trp Ile
        35                  40                  45

Gly Gly Leu Asn Pro Asn Thr Gly Gly Thr Thr Tyr Asn Gln Lys Phe
    50                  55                  60

Lys Asp Arg Ala Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Lys Gly Gly Trp Phe Phe Leu Asp Tyr Trp Gly Gln Gly Thr
            100                 105                 110

Leu Val Thr Val Ser Ser
        115

<210>  56
<211>  118

<212>  PRT
<213>  Artificial sequence

<220>
<223>  Humanized SHS007-39-CHI VH

<400>  56

Gln Val Gln Leu Val Gln Ser Gly Ala Glu Val Val Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Met Arg Gln Ala Pro Gly Gln Gly Leu Glu Trp Ile
        35                  40                  45

Gly Gly Leu Asn Pro Asn Thr Gly Gly Thr Thr Tyr Asn Gln Lys Phe
    50                  55                  60

Lys Asp Lys Ala Thr Met Thr Val Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Lys Gly Gly Trp Phe Phe Leu Asp Tyr Trp Gly Gln Gly Thr
            100                 105                 110

Leu Val Thr Val Ser Ser
        115


<210>  57
<211>  118
<212>  PRT
<213>  Artificial sequence

<220>
<223>  Humanized SHS007-39-CHI VH

<400>  57

Gln Val Gln Leu Val Gln Ser Gly Ala Glu Val Val Lys Pro Gly Ala
1               5                   10                  15

Ser Val Lys Ile Ser Cys Lys Thr Ser Gly Tyr Thr Phe Thr Glu Tyr
            20                  25                  30

Thr Ile His Trp Met Lys Gln Ala Pro Gly Gln Gly Leu Glu Trp Ile
        35                  40                  45

Gly Gly Leu Asn Pro Asn Thr Gly Gly Thr Thr Tyr Asn Gln Lys Phe

50                  55                  60

Lys Asp Lys Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Lys Gly Gly Trp Phe Phe Leu Asp Tyr Trp Gly Gln Gly Thr
            100                 105                 110

Leu Leu Thr Val Ser Ser
        115

<210>  58
<211>  106
<212>  PRT
<213>  Artificial sequence

<220>
<223>  Humanized SHS007-39-CHI VL

<400>  58

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gly Gly Thr Lys Val Glu Ile Lys
            100                 105

<210>  59
<211>  106
<212>  PRT
<213>  Artificial sequence

<220>

<223>  Humanized SHS007-39-CHI VL

<400>  59

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Arg Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gly Gly Thr Lys Val Glu Ile Lys
            100                 105

<210>  60
<211>  106
<212>  PRT
<213>  Artificial sequence

<220>
<223>  Humanized SHS007-39-CHI VL

<400>  60

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Arg Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr

85                  90                  95

Phe Gly Gly Gly Thr Lys Val Glu Ile Lys
            100                 105

<210>  61
<211>  106
<212>  PRT
<213>  Artificial sequence

<220>
<223>  Humanized SHS007-39-CHI VL

<400>  61

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Arg Leu Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Ala Ala Thr Tyr Tyr Cys Gln Gln Trp Ser Ser Asn Pro Pro Thr
                85                  90                  95

Phe Gly Gly Gly Thr Lys Val Glu Ile Lys
            100                 105

<210>  62
<211>  106
<212>  PRT
<213>  Artificial sequence

<220>
<223>  Humanized SHS007-39-CHI VL

<400>  62

Glu Ile Val Leu Thr Gln Ser Pro Ala Thr Leu Ser Ala Ser Pro Gly
1               5                   10                  15

Glu Arg Val Thr Met Ser Cys Ser Ala Ser Ser Ser Val Ser Tyr Met
            20                  25                  30

His Trp Tyr Gln Gln Lys Pro Gly Gln Ala Pro Arg Arg Leu Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Thr Leu Thr Ile Ser Ser Met Glu Pro Glu
65                  70                  75                  80

Asp Ala Ala Thr Tyr Tyr Cys Gln Gln Trp Ser Ser Asn Pro Pro Thr
                85                  90                  95

Phe Gly Gly Gly Thr Lys Val Glu Ile Lys
            100                 105

<210>  63
<211>  123
<212>  PRT
<213>  Artificial sequence

<220>
<223>  Humanized SHS007-51-G34K-2 VH

<400>  63

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Phe
            20                  25                  30

Gly Met Ser Trp Val Arg Gln Ala Pro Gly Lys Gly Leu Glu Trp Val
        35                  40                  45

Ser Thr Ile Asn Ser Gly Asp Asn Tyr Ser Tyr Ser Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Pro His Ile Phe Tyr Tyr Gly Ser Pro Ala Trp Phe Ala Tyr
            100                 105                 110

Trp Gly Gln Gly Thr Leu Val Thr Val Ser Ser
        115                 120

<210>  64

<211>  123
<212>  PRT
<213>  Artificial sequence

<220>
<223>  Humanized SHS007-51-G34K-2 VH

<400>  64

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Phe
            20                  25                  30

Gly Met Ser Trp Val Arg Gln Ala Pro Gly Lys Gly Leu Glu Trp Val
        35                  40                  45

Ala Thr Ile Asn Ser Gly Asp Asn Tyr Ser Tyr Ser Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Pro His Ile Phe Tyr Tyr Gly Ser Pro Ala Trp Phe Ala Tyr
            100                 105                 110

Trp Gly Gln Gly Thr Leu Val Thr Val Ser Ser
        115                 120


<210>  65
<211>  112
<212>  PRT
<213>  Artificial sequence

<220>
<223>  Humanized SHS007-51-G34K-2 VL

<400>  65

Asp Val Val Met Thr Gln Ser Pro Leu Ser Leu Pro Val Thr Leu Gly
1               5                   10                  15

Gln Pro Ala Ser Ile Ser Cys Arg Ser Arg Gln Ser Leu Val His Ser
            20                  25                  30

Asn Lys Asn Thr Tyr Leu His Trp Phe Gln Gln Arg Pro Gly Gln Ser
        35                  40                  45

Pro Arg Arg Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Leu Thr Phe Gly Gly Gly Thr Lys Val Glu Ile Lys
            100                 105                 110

<210>  66
<211>  112
<212>  PRT
<213>  Artificial sequence

<220>
<223>  Humanized SHS007-51-G34K-2 VL

<400>  66

Asp Val Val Met Thr Gln Ser Pro Leu Ser Leu Pro Val Thr Leu Gly
1               5                   10                  15

Gln Pro Ala Ser Ile Ser Cys Arg Ser Arg Gln Ser Leu Val His Ser
            20                  25                  30

Asn Lys Asn Thr Tyr Leu His Trp Tyr Gln Gln Arg Pro Gly Gln Ser
        35                  40                  45

Pro Arg Arg Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Leu Thr Phe Gly Gly Gly Thr Lys Val Glu Ile Lys
            100                 105                 110

<210>  67
<211>  112
<212>  PRT
<213>  Artificial sequence

<220>
<223>  Humanized SHS007-51-G34K-2 VL

<400>  67

Asp Val Val Met Thr Gln Ser Pro Leu Ser Leu Pro Val Thr Leu Gly
1               5                   10                  15

Gln Pro Ala Ser Ile Ser Cys Arg Ser Arg Gln Ser Leu Val His Ser
            20                  25                  30

Asn Lys Asn Thr Tyr Leu His Trp Phe Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Leu Thr Phe Gly Gly Gly Thr Lys Val Glu Ile Lys
            100                 105                 110

<210>  68
<211>  112
<212>  PRT
<213>  Artificial sequence

<220>
<223>  Humanized SHS007-51-G34K-2 VL

<400>  68

Asp Val Val Met Thr Gln Ser Pro Leu Ser Leu Pro Val Thr Leu Gly
1               5                   10                  15

Gln Pro Ala Ser Ile Ser Cys Arg Ser Arg Gln Ser Leu Val His Ser
            20                  25                  30

Asn Lys Asn Thr Tyr Leu His Trp Tyr Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Leu Thr Phe Gly Gly Gly Thr Lys Val Glu Ile Lys
            100                 105                 110

<210>  69
<211>  112
<212>  PRT
<213>  Artificial sequence

<220>
<223>  Humanized SHS007-51-G34K-2 VL

<400>  69

Asp Val Val Met Thr Gln Ser Pro Leu Ser Leu Pro Val Thr Leu Gly
1               5                   10                  15

Gln Pro Ala Ser Ile Ser Cys Arg Ser Arg Gln Ser Leu Val His Ser
            20                  25                  30

Asn Lys Asn Thr Tyr Leu His Trp Tyr Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Phe Cys Ser Gln Ser
                85                  90                  95

Thr His Val Pro Leu Thr Phe Gly Gly Gly Thr Lys Val Glu Ile Lys
            100                 105                 110

<210>  70
<211>  112
<212>  PRT
<213>  Artificial sequence

<220>
<223>  Humanized SHS007-51-G34K-2 VL

<400>  70

Asp Val Val Met Thr Gln Ser Pro Leu Ser Leu Pro Val Thr Leu Gly
1               5                   10                  15

Gln Pro Ala Ser Val Ser Cys Arg Ser Arg Gln Ser Leu Val His Ser
            20                  25                  30

Asn Lys Asn Thr Tyr Leu His Trp Tyr Gln Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Phe Cys Ser Gln Ser
                85                  90                  95

Thr His Val Pro Leu Thr Phe Gly Gly Gly Thr Lys Leu Glu Ile Lys
            100                 105                 110

<210>  71
<211>  121
<212>  PRT
<213>  Artificial sequence

<220>
<223>  Humanized SHS007-65-CHI VH

<400>  71

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Ile His Trp Val Arg Gln Ala Pro Gly Lys Gly Leu Glu Trp Val
        35                  40                  45

Ser Tyr Ile Ser Ser Gly Ser Asn Thr Ile Tyr Tyr Ala Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Met Val Thr Arg Trp Phe Tyr Tyr Ala Met Asp Tyr Trp Gly
            100                 105                 110

Gln Gly Thr Thr Val Thr Val Ser Ser
        115                 120

<210>  72
<211>  121
<212>  PRT
<213>  Artificial sequence

<220>
<223>  Humanized SHS007-65-CHI VH

<400>  72

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Ile His Trp Val Arg Gln Ala Pro Gly Lys Gly Leu Glu Trp Val
        35                  40                  45

Ser Tyr Ile Ser Ser Gly Ser Asn Thr Ile Tyr Tyr Ala Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Thr Met Val Thr Arg Trp Phe Tyr Tyr Ala Met Asp Tyr Trp Gly
            100                 105                 110

Gln Gly Thr Thr Val Thr Val Ser Ser
        115                 120


<210>  73
<211>  121
<212>  PRT
<213>  Artificial sequence

<220>
<223>  Humanized SHS007-65-CHI VH

<400>  73

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Ile His Trp Val Arg Gln Ala Pro Gly Lys Gly Leu Glu Trp Val
        35                  40                  45

Ala Tyr Ile Ser Ser Gly Ser Asn Thr Ile Tyr Tyr Ala Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Thr Met Val Thr Arg Trp Phe Tyr Tyr Ala Met Asp Tyr Trp Gly
            100                 105                 110

Gln Gly Thr Thr Val Thr Val Ser Ser
        115                 120

<210>  74
<211>  106
<212>  PRT
<213>  Artificial sequence

<220>
<223>  Humanized SHS007-65-CHI VL

<400>  74

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

Tyr Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gln Gly Thr Lys Leu Glu Ile Lys
            100                 105

<210>  75
<211>  106
<212>  PRT
<213>  Artificial sequence

<220>
<223>  Humanized SHS007-65-CHI VL

<400>  75

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

Tyr Trp Tyr Gln Gln Lys Pro Gly Gln Ala Pro Arg Arg Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gln Gly Thr Lys Leu Glu Ile Lys
            100                 105


<210>  76
<211>  106
<212>  PRT
<213>  Artificial sequence

<220>
<223>  Humanized SHS007-65-CHI VL

<400>  76

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

Tyr Trp Tyr Gln Gln Lys Pro Gly Gln Ala Pro Arg Arg Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gln Gly Thr Lys Leu Glu Ile Lys
            100                 105

<210>  77
<211>  106
<212>  PRT
<213>  Artificial sequence

<220>
<223>  Humanized SHS007-65-CHI VL

<400>  77

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

Tyr Trp Tyr Gln Gln Lys Pro Gly Gln Ala Pro Arg Arg Leu Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gln Gly Thr Lys Leu Glu Ile Lys
            100                 105

<210>  78
<211>  106
<212>  PRT
<213>  Artificial sequence

<220>
<223>  Humanized SHS007-65-CHI VL

<400>  78

Glu Ile Val Leu Thr Gln Ser Pro Ala Thr Leu Ser Ala Ser Pro Gly
1               5                   10                  15

Glu Arg Val Thr Leu Ser Cys Ser Ala Ser Ser Ser Val Ser Tyr Met
            20                  25                  30

Tyr Trp Tyr Gln Gln Lys Pro Gly Gln Ala Pro Arg Arg Leu Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys Gln Gln Trp Ser Ser Asn Pro Pro Thr
                85                  90                  95

Phe Gly Gln Gly Thr Lys Leu Glu Ile Lys
            100                 105

<210>  79
<211>  106
<212>  PRT
<213>  Artificial sequence

<220>
<223>  Humanized SHS007-65-CHI VL

<400>  79

Glu Ile Val Leu Thr Gln Ser Pro Ala Thr Leu Ser Ala Ser Pro Gly
1               5                   10                  15

Glu Arg Val Thr Met Ser Cys Ser Ala Ser Ser Ser Val Ser Tyr Met
            20                  25                  30

Tyr Trp Tyr Gln Gln Lys Pro Gly Gln Ala Pro Arg Arg Leu Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Thr Leu Thr Ile Ser Ser Met Glu Pro Glu
65                  70                  75                  80

Asp Phe Ala Val Tyr Tyr Cys Gln Gln Trp Ser Ser Asn Pro Pro Thr
                85                  90                  95

Phe Gly Gln Gly Thr Lys Leu Glu Ile Lys
            100                 105

<210>  80
<211>  351
<212>  DNA
<213>  Artificial sequence

<220>
<223>  hu17-00 VH nucleotide sequence

<400>  80
caggtgcagc tggttcagag cggcgctgag gtgaagaagc ccggcagcag cgtgaaggtg      60

agctgcaagg ccagcggctt tacctttacc gactacgtgg taagctgggt gaggcaggca     120

ccgggccagg gcctggagtg gatgggcgag atctaccctg gcggaggatt cattcactac     180

aacgagaagt tcaagggcag ggtgaccatc accgctgaca agagcacaag caccgcctac     240

atggagctgt caagcctgag gagcgaggac accgccgtgt attactgcgc caggttcccc     300

aaatactatt tcgactactg gggccaaggc accaccgtga ccgtgagcag c              351


<210>  81
<211>  336
<212>  DNA
<213>  Artificial sequence

<220>
<223>  hu17-00 VL nucleotide sequence

<400>  81
gacatcgtga tgacccagag ccccctgagc ctgcccgtga ccccaggcga gcccgccagc      60

atcagctgca ggagcagcca gagcatcgtg cacagcaaca ggtacaccta cctggagtgg     120

tacctgcaga agcccggtca gtcaccacag ctgctgatct acggcgtgag caaccgcttc     180

agcggcgtgc ccgacaggtt tagcggaagc ggcagcggga ccgacttcac cctgaagatc     240

tctagggtgg aggccgagga cgtgggcgtg tactactgct tccagtctac cttgttcccc     300

tggaccttcg gccagggcac caaggtggag atcaag                               336


<210>  82
<211>  354
<212>  DNA
<213>  Artificial sequence

<220>
<223>  hu39-01 VH nucleotide sequence

<400>  82
caggtgcagc tggtgcagag tggggccgag gtgaaaaagc ccggtgccag cgtgaaagtg      60

agctgcaagg ccagcggcta taccttcacc gagtacacca tccattgggt gaggcaggca     120

cccggccagg gcctggagtg gatgggcggt ctgaacccca acaccggcgg aaccacctac     180

aaccaaaagt tcaaggaccg cgtaactatg accagggaca ccagcatcag caccgcctac      240

atggagctga gcaggctgag gagcgacgac accgccgtgt actactgcgc caggaagggc     300

ggctggttct tcttggacta ctggggccaa ggcaccttgg tgactgttag ctct           354


<210>  83
<211>  318
<212>  DNA
<213>  Artificial sequence

<220>
<223>  hu39-01 VL nucleotide sequence

<400>  83
gagatcgtgc tgacccagag ccccgctact ctgagcctga gccctggcga gagggccacg      60

ctgagctgct ctgcctcaag cagcgtgagc tacatgcatt ggtatcagca gaagcccgga     120

caggccccca ggcgcttgat ctacgacacc agcaagctgg cctctggcat ccccgccagg     180

ttcagcggca gcggctccgg caccgacttc accctgacca taagcagcct ggagcccgag     240

gacttcgccg tgtactactg ccagcagtgg agcagcaacc cccccacctt cggcggaggc     300

accaaggtgg agatcaag                                                   318


<210>  84
<211>  369
<212>  DNA
<213>  Artificial sequence

<220>
<223>  hu51-G34K-12 VH nucleotide sequence

<400>  84
gaggtgcagc tggtggagag cggtggcggt ctggtgaaac ccggtggcag cctgaggctg      60

agttgtgcgg cgagcggctt caccttcagc agcttcggca tgagctgggt gaggcaagcc     120

cctggcaagg gcttggagtg ggtggctacc atcaacagcg gcgacaacta cagctacagc     180

cccgacagcg tgaagggcag gttcaccatc agcagggaca acgcgaagaa ctccctgtac     240

ctgcagatga acagccttag ggccgaagac accgccgtgt actactgcgc caggccccac     300

atcttctact acggctcccc cgcatggttc gcctactggg gccagggcac cctggtgact     360

gtgagcagc                                                             369


<210>  85
<211>  336
<212>  DNA
<213>  Artificial sequence

<220>
<223>  hu51-G34K-12 VL nucleotide sequence

<400>  85
gacgtggtga tgacccagag ccccctcagc ctgcccgtga ccctcggaca gccggcaagc      60

atcagctgca ggagcaggca gagcctggtg cacagcaaca agaacaccta cctgcactgg     120

ttccagcaaa ggcctggcca atcacccagg ctgctgatct acaaggtgag caacaggttc     180

agcggcgtgc ccgacaggtt tagtggcagt ggctcaggca ccgattttac cctgaagatt     240

agcagggtgg aggccgagga cgtgggcgtg tactactgca gccagagcac ccatgtgcct     300

ctgaccttcg gtggcggaac taaggtggag atcaag                               336

<210>  86
<211>  363
<212>  DNA
<213>  Artificial sequence

<220>
<223>  hu65-13 VH nucleotide sequence

<400>  86
gaggtgcagc tggtggagag cggaggcggc ctggtacagc ccggtggctc actgaggctg      60

agctgtgccg ccagcggctt caccttcagc gactacggca tccactgggt gcgacaagcc     120

cctggcaagg gcctggagtg ggtgagctac atcagcagcg gcagcaacac catctactac     180

gccgacacag tgaagggcag gttcaccatt tctagggaca acgccaagaa ctctctttac     240

ctgcagatga acagcctgag ggccgaagac accgccgtgt actactgcgc caccatggtg     300

accaggtggt tctattacgc tatggactac tggggccagg gcaccaccgt gactgtgagc     360

agc                                                                   363


<210>  87
<211>  318
<212>  DNA
<213>  Artificial sequence

<220>
<223>  hu65-13 VL nucleotide sequence

<400>  87
gagatcgtgc tgacccagag ccccgcaaca ctgagcctga gtcccggcga gagggccact      60

ctgagctgct cagccagcag cagcgtgagc tatatgtatt ggtatcagca gaagcctggc     120

caagccccca ggcggttgat ctacgacacc agcaagctgg caagcggcgt ccccgccagg     180

ttcagtggta gcggcagcgg gacagactac accctgacca tctccagcct tgagcccgag     240

gacttcgccg tgtactactg ccagcaatgg agcagcaacc cccccacctt cggacaggga     300

accaaactgg aaatcaag                                                   318


<210>  88
<211>  506
<212>  PRT
<213>  Artificial sequence

<220>
<223>  4Ig-B7-H3 extracellular domain

<400>  88

Leu Glu Val Gln Val Pro Glu Asp Pro Val Val Ala Leu Val Gly Thr
1               5                   10                  15


Asp Ala Thr Leu Cys Cys Ser Phe Ser Pro Glu Pro Gly Phe Ser Leu
            20                  25                  30

Ala Gln Leu Asn Leu Ile Trp Gln Leu Thr Asp Thr Lys Gln Leu Val
        35                  40                  45

His Ser Phe Ala Glu Gly Gln Asp Gln Gly Ser Ala Tyr Ala Asn Arg
    50                  55                  60

Thr Ala Leu Phe Pro Asp Leu Leu Ala Gln Gly Asn Ala Ser Leu Arg
65                  70                  75                  80

Leu Gln Arg Val Arg Val Ala Asp Glu Gly Ser Phe Thr Cys Phe Val
                85                  90                  95

Ser Ile Arg Asp Phe Gly Ser Ala Ala Val Ser Leu Gln Val Ala Ala
            100                 105                 110

Pro Tyr Ser Lys Pro Ser Met Thr Leu Glu Pro Asn Lys Asp Leu Arg
        115                 120                 125

Pro Gly Asp Thr Val Thr Ile Thr Cys Ser Ser Tyr Gln Gly Tyr Pro
    130                 135                 140

Glu Ala Glu Val Phe Trp Gln Asp Gly Gln Gly Val Pro Leu Thr Gly
145                 150                 155                 160

Asn Val Thr Thr Ser Gln Met Ala Asn Glu Gln Gly Leu Phe Asp Val
                165                 170                 175

His Ser Ile Leu Arg Val Val Leu Gly Ala Asn Gly Thr Tyr Ser Cys
            180                 185                 190

Leu Val Arg Asn Pro Val Leu Gln Gln Asp Ala His Ser Ser Val Thr
        195                 200                 205

Ile Thr Pro Gln Arg Ser Pro Thr Gly Ala Val Glu Val Gln Val Pro
    210                 215                 220

Glu Asp Pro Val Val Ala Leu Val Gly Thr Asp Ala Thr Leu Arg Cys
225                 230                 235                 240

Ser Phe Ser Pro Glu Pro Gly Phe Ser Leu Ala Gln Leu Asn Leu Ile
                245                 250                 255

Trp Gln Leu Thr Asp Thr Lys Gln Leu Val His Ser Phe Thr Glu Gly
            260                 265                 270

Arg Asp Gln Gly Ser Ala Tyr Ala Asn Arg Thr Ala Leu Phe Pro Asp

275                 280                 285

Leu Leu Ala Gln Gly Asn Ala Ser Leu Arg Leu Gln Arg Val Arg Val
    290                 295                 300

Ala Asp Glu Gly Ser Phe Thr Cys Phe Val Ser Ile Arg Asp Phe Gly
305                 310                 315                 320

Ser Ala Ala Val Ser Leu Gln Val Ala Ala Pro Tyr Ser Lys Pro Ser
                325                 330                 335

Met Thr Leu Glu Pro Asn Lys Asp Leu Arg Pro Gly Asp Thr Val Thr
            340                 345                 350

Ile Thr Cys Ser Ser Tyr Arg Gly Tyr Pro Glu Ala Glu Val Phe Trp
        355                 360                 365

Gln Asp Gly Gln Gly Val Pro Leu Thr Gly Asn Val Thr Thr Ser Gln
    370                 375                 380

Met Ala Asn Glu Gln Gly Leu Phe Asp Val His Ser Val Leu Arg Val
385                 390                 395                 400

Val Leu Gly Ala Asn Gly Thr Tyr Ser Cys Leu Val Arg Asn Pro Val
                405                 410                 415

Leu Gln Gln Asp Ala His Gly Ser Val Thr Ile Thr Gly Gln Pro Met
            420                 425                 430

Thr Phe Pro Pro Glu Ala Leu Trp Val Thr Val Gly Leu Ser Val Cys
        435                 440                 445

Leu Ile Ala Leu Leu Val Ala Leu Ala Phe Val Cys Trp Arg Lys Ile
    450                 455                 460

Lys Gln Ser Cys Glu Glu Glu Asn Ala Gly Ala Glu Asp Gln Asp Gly
465                 470                 475                 480

Glu Gly Glu Gly Ser Lys Thr Ala Leu Gln Pro Leu Lys His Ser Asp
                485                 490                 495

Ser Lys Glu Asp Asp Gly Gln Glu Ile Ala
            500                 505

<210>  89
<211>  217
<212>  PRT
<213>  Artificial sequence

<220>
<223>  2Ig-B7-H3 extracellular domain

<400>  89

Leu Glu Val Gln Val Pro Glu Asp Pro Val Val Ala Leu Val Gly Thr
1               5                   10                  15

Asp Ala Thr Leu Cys Cys Ser Phe Ser Pro Glu Pro Gly Phe Ser Leu
            20                  25                  30

Ala Gln Leu Asn Leu Ile Trp Gln Leu Thr Asp Thr Lys Gln Leu Val
        35                  40                  45

His Ser Phe Ala Glu Gly Gln Asp Gln Gly Ser Ala Tyr Ala Asn Arg
    50                  55                  60

Thr Ala Leu Phe Pro Asp Leu Leu Ala Gln Gly Asn Ala Ser Leu Arg
65                  70                  75                  80

Leu Gln Arg Val Arg Val Ala Asp Glu Gly Ser Phe Thr Cys Phe Val
                85                  90                  95

Ser Ile Arg Asp Phe Gly Ser Ala Ala Val Ser Leu Gln Val Ala Ala
            100                 105                 110

Pro Tyr Ser Lys Pro Ser Met Thr Leu Glu Pro Asn Lys Asp Leu Arg
        115                 120                 125

Pro Gly Asp Thr Val Thr Ile Thr Cys Ser Ser Tyr Arg Gly Tyr Pro
    130                 135                 140

Glu Ala Glu Val Phe Trp Gln Asp Gly Gln Gly Val Pro Leu Thr Gly
145                 150                 155                 160

Asn Val Thr Thr Ser Gln Met Ala Asn Glu Gln Gly Leu Phe Asp Val
                165                 170                 175

His Ser Val Leu Arg Val Val Leu Gly Ala Asn Gly Thr Tyr Ser Cys
            180                 185                 190

Leu Val Arg Asn Pro Val Leu Gln Gln Asp Ala His Gly Ser Val Thr
        195                 200                 205

Ile Thr Gly Gln Pro Met Thr Phe Pro
    210                 215

<210>  90

<211>  1602
<212>  DNA
<213>  Artificial sequence

<220>
<223>  4Ig-B7-H3 full-length cDNA

<400>  90
atgctgcgga ggagaggaag ccctggcatg ggtgtccatg tgggggcagc acttggagcc      60

ctgtggtttt gcctcaccgg ggcactcgag gtccaggtgc ctgaggatcc ggttgtggcc     120

ctcgtaggga cagacgcgac tctttgctgc tccttctctc ccgagcctgg gtttagcctt     180

gcccagctca acctgatttg gcagctgacg gacaccaagc agttggtcca ctctttcgcc     240

gagggacaag accagggcag cgcgtatgcc aacagaaccg ccctttttcc tgatctcttg     300

gctcaaggca acgctagtct gcggctgcaa agggtccggg tggctgacga agggagtttc     360

acctgctttg tgagcatccg agactttggt agcgcagccg ttagtttgca ggtggcagcc     420

ccctatagca agccatccat gaccctggaa cccaataagg atctgaggcc gggtgacacg     480

gtgaccatca cgtgtagcag ctatcagggc tatccagagg ctgaggtttt ctggcaggac     540

ggccaaggcg tgccgcttac cggaaacgtg accacgagcc aaatggcgaa tgaacaaggg     600

ctgtttgacg tacactctat cctgagagtg gtgttgggcg ctaacggacc ctatagttgt     660

ttggtcagaa atcccgtcct tcaacaagac gcgcatagca gcgtcacgat cacgccccaa     720

aggagcccga caggggccgt agaggttcaa gtacccgaag acccagtcgt ggcgttggtt     780

ggaacagatg ccaccctgag gtgtagcttt agcccggaac ccggcttctc cctggcccaa     840

cttaacttga tctggcaatt gaccgacact aagcagctgg tgcacagctt cacagagggc     900

agggatcaag ggagcgccta cgcaaatagg accgcactgt ttcccgatct gctcgcccaa     960

ggtaacgcgt ccctgagact gcagcgggtg agggtcgccg acgagggatc ttttacatgt    1020

tttgttagta ttcgcgactt cggttcagcc gccgtgagcc tgcaagttgc agcgccatat    1080

tctaaacctt ctatgacatt ggaaccaaat aaagaccttc gcccaggcga cacagtaaca    1140

ataacgtgtt ctagttacag gggatacccg gaggcggagg tattttggca agacggtcaa    1200

ggtgtgcccc tgaccggcaa tgtaaccacc agccagatgg ctaatgagca aggcctgttc    1260

gacgtgcact cagtattgcg ggtcgtgctt ggcgcgaatg gcacgtattc ttgtctggtg    1320

aggaatccag ttctgcaaca ggacgcccac ggaagtgtta ccataacagg gcaaccgatg    1380

acttttcccc ccgaagcact ctgggtaacc gtaggcctga gtgtgtgcct catcgcgctt    1440

ttggtggcac tggcgttcgt gtgctggagg aagatcaaac aaagctgcga agaggagaac    1500

gccggtgccg aggaccaaga tggagaaggc gagggtagca aaacggcact gcaacccctg    1560

aaacacagcg acagcaagga agacgatgga caggagatag cc                        1602

<210>  91
<211>  836
<212>  PRT
<213>  Artificial sequence

<220>
<223>  4Ig-B7-H3 Fc fusion protein

<400>  91

Leu Glu Val Gln Val Pro Glu Asp Pro Val Val Ala Leu Val Gly Thr
1               5                   10                  15

Asp Ala Thr Leu Cys Cys Ser Phe Ser Pro Glu Pro Gly Phe Ser Leu
            20                  25                  30

Ala Gln Leu Asn Leu Ile Trp Gln Leu Thr Asp Thr Lys Gln Leu Val
        35                  40                  45

His Ser Phe Ala Glu Gly Gln Asp Gln Gly Ser Ala Tyr Ala Asn Arg
    50                  55                  60

Thr Ala Leu Phe Pro Asp Leu Leu Ala Gln Gly Asn Ala Ser Leu Arg
65                  70                  75                  80

Leu Gln Arg Val Arg Val Ala Asp Glu Gly Ser Phe Thr Cys Phe Val
                85                  90                  95

Ser Ile Arg Asp Phe Gly Ser Ala Ala Val Ser Leu Gln Val Ala Ala
            100                 105                 110

Pro Tyr Ser Lys Pro Ser Met Thr Leu Glu Pro Asn Lys Asp Leu Arg
        115                 120                 125

Pro Gly Asp Thr Val Thr Ile Thr Cys Ser Ser Tyr Gln Gly Tyr Pro
    130                 135                 140

Glu Ala Glu Val Phe Trp Gln Asp Gly Gln Gly Val Pro Leu Thr Gly
145                 150                 155                 160

Asn Val Thr Thr Ser Gln Met Ala Asn Glu Gln Gly Leu Phe Asp Val
                165                 170                 175

His Ser Ile Leu Arg Val Val Leu Gly Ala Asn Gly Thr Tyr Ser Cys
            180                 185                 190

Leu Val Arg Asn Pro Val Leu Gln Gln Asp Ala His Ser Ser Val Thr
        195                 200                 205

Ile Thr Pro Gln Arg Ser Pro Thr Gly Ala Val Glu Val Gln Val Pro

210                 215                 220

Glu Asp Pro Val Val Ala Leu Val Gly Thr Asp Ala Thr Leu Arg Cys
225                 230                 235                 240

Ser Phe Ser Pro Glu Pro Gly Phe Ser Leu Ala Gln Leu Asn Leu Ile
                245                 250                 255

Trp Gln Leu Thr Asp Thr Lys Gln Leu Val His Ser Phe Thr Glu Gly
            260                 265                 270

Arg Asp Gln Gly Ser Ala Tyr Ala Asn Arg Thr Ala Leu Phe Pro Asp
        275                 280                 285

Leu Leu Ala Gln Gly Asn Ala Ser Leu Arg Leu Gln Arg Val Arg Val
    290                 295                 300

Ala Asp Glu Gly Ser Phe Thr Cys Phe Val Ser Ile Arg Asp Phe Gly
305                 310                 315                 320

Ser Ala Ala Val Ser Leu Gln Val Ala Ala Pro Tyr Ser Lys Pro Ser
                325                 330                 335

Met Thr Leu Glu Pro Asn Lys Asp Leu Arg Pro Gly Asp Thr Val Thr
            340                 345                 350

Ile Thr Cys Ser Ser Tyr Arg Gly Tyr Pro Glu Ala Glu Val Phe Trp
        355                 360                 365

Gln Asp Gly Gln Gly Val Pro Leu Thr Gly Asn Val Thr Thr Ser Gln
    370                 375                 380

Met Ala Asn Glu Gln Gly Leu Phe Asp Val His Ser Val Leu Arg Val
385                 390                 395                 400

Val Leu Gly Ala Asn Gly Thr Tyr Ser Cys Leu Val Arg Asn Pro Val
                405                 410                 415

Leu Gln Gln Asp Ala His Gly Ser Val Thr Ile Thr Gly Gln Pro Met
            420                 425                 430

Thr Phe Pro Pro Glu Ala Leu Trp Val Thr Val Gly Leu Ser Val Cys
        435                 440                 445

Leu Ile Ala Leu Leu Val Ala Leu Ala Phe Val Cys Trp Arg Lys Ile
    450                 455                 460

Lys Gln Ser Cys Glu Glu Glu Asn Ala Gly Ala Glu Asp Gln Asp Gly
465                 470                 475                 480

Glu Gly Glu Gly Ser Lys Thr Ala Leu Gln Pro Leu Lys His Ser Asp
                485                 490                 495

Ser Lys Glu Asp Asp Gly Gln Glu Ile Ala Ala Ser Thr Lys Gly Pro
            500                 505                 510

Ser Val Phe Pro Leu Ala Pro Ser Ser Lys Ser Thr Ser Gly Gly Thr
        515                 520                 525

Ala Ala Leu Gly Cys Leu Val Lys Asp Tyr Phe Pro Glu Pro Val Thr
    530                 535                 540

Val Ser Trp Asn Ser Gly Ala Leu Thr Ser Gly Val His Thr Phe Pro
545                 550                 555                 560

Ala Val Leu Gln Ser Ser Gly Leu Tyr Ser Leu Ser Ser Val Val Thr
                565                 570                 575

Val Pro Ser Ser Ser Leu Gly Thr Gln Thr Tyr Ile Cys Asn Val Asn
            580                 585                 590

His Lys Pro Ser Asn Thr Lys Val Asp Lys Lys Val Glu Pro Lys Ser
        595                 600                 605

Cys Asp Lys Thr His Thr Cys Pro Pro Cys Pro Ala Pro Glu Leu Leu
    610                 615                 620

Gly Gly Pro Ser Val Phe Leu Phe Pro Pro Lys Pro Lys Asp Thr Leu
625                 630                 635                 640

Met Ile Ser Arg Thr Pro Glu Val Thr Cys Val Val Val Asp Val Ser
                645                 650                 655

His Glu Asp Pro Glu Val Lys Phe Asn Trp Tyr Val Asp Gly Val Glu
            660                 665                 670

Val His Asn Ala Lys Thr Lys Pro Arg Glu Glu Gln Tyr Asn Ser Thr
        675                 680                 685

Tyr Arg Val Val Ser Val Leu Thr Val Leu His Gln Asp Trp Leu Asn
    690                 695                 700

Gly Lys Glu Tyr Lys Cys Lys Val Ser Asn Lys Ala Leu Pro Ala Pro
705                 710                 715                 720

Ile Glu Lys Thr Ile Ser Lys Ala Lys Gly Gln Pro Arg Glu Pro Gln
                725                 730                 735

Val Tyr Thr Leu Pro Pro Ser Arg Asp Glu Leu Thr Lys Asn Gln Val
            740                 745                 750

Ser Leu Thr Cys Leu Val Lys Gly Phe Tyr Pro Ser Asp Ile Ala Val
        755                 760                 765

Glu Trp Glu Ser Asn Gly Gln Pro Glu Asn Asn Tyr Lys Thr Thr Pro
    770                 775                 780

Pro Val Leu Asp Ser Asp Gly Ser Phe Phe Leu Tyr Ser Lys Leu Thr
785                 790                 795                 800

Val Asp Lys Ser Arg Trp Gln Gln Gly Asn Val Phe Ser Cys Ser Val
                805                 810                 815

Met His Glu Ala Leu His Asn His Tyr Thr Gln Lys Ser Leu Ser Leu
            820                 825                 830

Ser Pro Gly Lys
        835

<210>  92
<211>  522
<212>  PRT
<213>  Artificial sequence

<220>
<223>  4Ig-B7-H3 His fusion protein

<400>  92

Leu Glu Val Gln Val Pro Glu Asp Pro Val Val Ala Leu Val Gly Thr
1               5                   10                  15

Asp Ala Thr Leu Cys Cys Ser Phe Ser Pro Glu Pro Gly Phe Ser Leu
            20                  25                  30

Ala Gln Leu Asn Leu Ile Trp Gln Leu Thr Asp Thr Lys Gln Leu Val
        35                  40                  45

His Ser Phe Ala Glu Gly Gln Asp Gln Gly Ser Ala Tyr Ala Asn Arg
    50                  55                  60

Thr Ala Leu Phe Pro Asp Leu Leu Ala Gln Gly Asn Ala Ser Leu Arg
65                  70                  75                  80

Leu Gln Arg Val Arg Val Ala Asp Glu Gly Ser Phe Thr Cys Phe Val
                85                  90                  95

Ser Ile Arg Asp Phe Gly Ser Ala Ala Val Ser Leu Gln Val Ala Ala
            100                 105                 110

Pro Tyr Ser Lys Pro Ser Met Thr Leu Glu Pro Asn Lys Asp Leu Arg
        115                 120                 125

Pro Gly Asp Thr Val Thr Ile Thr Cys Ser Ser Tyr Gln Gly Tyr Pro
    130                 135                 140

Glu Ala Glu Val Phe Trp Gln Asp Gly Gln Gly Val Pro Leu Thr Gly
145                 150                 155                 160

Asn Val Thr Thr Ser Gln Met Ala Asn Glu Gln Gly Leu Phe Asp Val
                165                 170                 175

His Ser Ile Leu Arg Val Val Leu Gly Ala Asn Gly Thr Tyr Ser Cys
            180                 185                 190

Leu Val Arg Asn Pro Val Leu Gln Gln Asp Ala His Ser Ser Val Thr
        195                 200                 205

Ile Thr Pro Gln Arg Ser Pro Thr Gly Ala Val Glu Val Gln Val Pro
    210                 215                 220

Glu Asp Pro Val Val Ala Leu Val Gly Thr Asp Ala Thr Leu Arg Cys
225                 230                 235                 240

Ser Phe Ser Pro Glu Pro Gly Phe Ser Leu Ala Gln Leu Asn Leu Ile
                245                 250                 255

Trp Gln Leu Thr Asp Thr Lys Gln Leu Val His Ser Phe Thr Glu Gly
            260                 265                 270

Arg Asp Gln Gly Ser Ala Tyr Ala Asn Arg Thr Ala Leu Phe Pro Asp
        275                 280                 285

Leu Leu Ala Gln Gly Asn Ala Ser Leu Arg Leu Gln Arg Val Arg Val
    290                 295                 300

Ala Asp Glu Gly Ser Phe Thr Cys Phe Val Ser Ile Arg Asp Phe Gly
305                 310                 315                 320

Ser Ala Ala Val Ser Leu Gln Val Ala Ala Pro Tyr Ser Lys Pro Ser
                325                 330                 335

Met Thr Leu Glu Pro Asn Lys Asp Leu Arg Pro Gly Asp Thr Val Thr
            340                 345                 350

Ile Thr Cys Ser Ser Tyr Arg Gly Tyr Pro Glu Ala Glu Val Phe Trp
        355                 360                 365

Gln Asp Gly Gln Gly Val Pro Leu Thr Gly Asn Val Thr Thr Ser Gln
    370                 375                 380

Met Ala Asn Glu Gln Gly Leu Phe Asp Val His Ser Val Leu Arg Val
385                 390                 395                 400

Val Leu Gly Ala Asn Gly Thr Tyr Ser Cys Leu Val Arg Asn Pro Val
                405                 410                 415

Leu Gln Gln Asp Ala His Gly Ser Val Thr Ile Thr Gly Gln Pro Met
            420                 425                 430

Thr Phe Pro Pro Glu Ala Leu Trp Val Thr Val Gly Leu Ser Val Cys
        435                 440                 445

Leu Ile Ala Leu Leu Val Ala Leu Ala Phe Val Cys Trp Arg Lys Ile
    450                 455                 460

Lys Gln Ser Cys Glu Glu Glu Asn Ala Gly Ala Glu Asp Gln Asp Gly
465                 470                 475                 480

Glu Gly Glu Gly Ser Lys Thr Ala Leu Gln Pro Leu Lys His Ser Asp
                485                 490                 495

Ser Lys Glu Asp Asp Gly Gln Glu Ile Ala Glu Gln Lys Leu Ile Ser
            500                 505                 510

Glu Glu Asp Leu His His His His His His
        515                 520

<210> 93
<211> 547
<212> PRT
<213> Artificial sequence

<220>
<223> 2Ig-B7-H3 Fc fusion protein

<400> 93

Leu Glu Val Gln Val Pro Glu Asp Pro Val Val Ala Leu Val Gly Thr
1               5                   10                  15

Asp Ala Thr Leu Cys Cys Ser Phe Ser Pro Glu Pro Gly Phe Ser Leu
            20                  25                  30

Ala Gln Leu Asn Leu Ile Trp Gln Leu Thr Asp Thr Lys Gln Leu Val
        35                  40                  45

His Ser Phe Ala Glu Gly Gln Asp Gln Gly Ser Ala Tyr Ala Asn Arg
    50                  55                  60

Thr Ala Leu Phe Pro Asp Leu Leu Ala Gln Gly Asn Ala Ser Leu Arg
65                  70                  75                  80

Leu Gln Arg Val Arg Val Ala Asp Glu Gly Ser Phe Thr Cys Phe Val
                85                  90                  95

Ser Ile Arg Asp Phe Gly Ser Ala Ala Val Ser Leu Gln Val Ala Ala
            100                 105                 110

Pro Tyr Ser Lys Pro Ser Met Thr Leu Glu Pro Asn Lys Asp Leu Arg
        115                 120                 125

Pro Gly Asp Thr Val Thr Ile Thr Cys Ser Ser Tyr Arg Gly Tyr Pro
    130                 135                 140

Glu Ala Glu Val Phe Trp Gln Asp Gly Gln Gly Val Pro Leu Thr Gly
145                 150                 155                 160

Asn Val Thr Thr Ser Gln Met Ala Asn Glu Gln Gly Leu Phe Asp Val
                165                 170                 175

His Ser Val Leu Arg Val Val Leu Gly Ala Asn Gly Thr Tyr Ser Cys
            180                 185                 190

Leu Val Arg Asn Pro Val Leu Gln Gln Asp Ala His Gly Ser Val Thr
        195                 200                 205

Ile Thr Gly Gln Pro Met Thr Phe Pro Ala Ser Thr Lys Gly Pro Ser
    210                 215                 220

Val Phe Pro Leu Ala Pro Ser Ser Lys Ser Thr Ser Gly Gly Thr Ala
225                 230                 235                 240

Ala Leu Gly Cys Leu Val Lys Asp Tyr Phe Pro Glu Pro Val Thr Val
                245                 250                 255

Ser Trp Asn Ser Gly Ala Leu Thr Ser Gly Val His Thr Phe Pro Ala
            260                 265                 270

Val Leu Gln Ser Ser Gly Leu Tyr Ser Leu Ser Ser Val Val Thr Val
        275                 280                 285

Pro Ser Ser Ser Leu Gly Thr Gln Thr Tyr Ile Cys Asn Val Asn His
    290                 295                 300

Lys Pro Ser Asn Thr Lys Val Asp Lys Lys Val Glu Pro Lys Ser Cys
305                 310                 315                 320

Asp Lys Thr His Thr Cys Pro Pro Cys Pro Ala Pro Glu Leu Leu Gly
                325                 330                 335

Gly Pro Ser Val Phe Leu Phe Pro Pro Lys Pro Lys Asp Thr Leu Met
            340                 345                 350

Ile Ser Arg Thr Pro Glu Val Thr Cys Val Val Val Asp Val Ser His
        355                 360                 365

Glu Asp Pro Glu Val Lys Phe Asn Trp Tyr Val Asp Gly Val Glu Val
    370                 375                 380

His Asn Ala Lys Thr Lys Pro Arg Glu Glu Gln Tyr Asn Ser Thr Tyr
385                 390                 395                 400

Arg Val Val Ser Val Leu Thr Val Leu His Gln Asp Trp Leu Asn Gly
                405                 410                 415

Lys Glu Tyr Lys Cys Lys Val Ser Asn Lys Ala Leu Pro Ala Pro Ile
            420                 425                 430

Glu Lys Thr Ile Ser Lys Ala Lys Gly Gln Pro Arg Glu Pro Gln Val
        435                 440                 445

Tyr Thr Leu Pro Pro Ser Arg Asp Glu Leu Thr Lys Asn Gln Val Ser
    450                 455                 460

Leu Thr Cys Leu Val Lys Gly Phe Tyr Pro Ser Asp Ile Ala Val Glu
465                 470                 475                 480

Trp Glu Ser Asn Gly Gln Pro Glu Asn Asn Tyr Lys Thr Thr Pro Pro
                485                 490                 495

Val Leu Asp Ser Asp Gly Ser Phe Phe Leu Tyr Ser Lys Leu Thr Val
            500                 505                 510

Asp Lys Ser Arg Trp Gln Gln Gly Asn Val Phe Ser Cys Ser Val Met
        515                 520                 525

His Glu Ala Leu His Asn His Tyr Thr Gln Lys Ser Leu Ser Leu Ser
530                 535                 540

Pro Gly Lys
545

<210> 94
<211> 233
<212> PRT
<213> Artificial sequence

<220>
<223> 2Ig-B7-H3 His fusion protein

<400> 94

Leu Glu Val Gln Val Pro Glu Asp Pro Val Val Ala Leu Val Gly Thr
1               5                   10                  15

Asp Ala Thr Leu Cys Cys Ser Phe Ser Pro Glu Pro Gly Phe Ser Leu
            20                  25                  30

Ala Gln Leu Asn Leu Ile Trp Gln Leu Thr Asp Thr Lys Gln Leu Val
        35                  40                  45

His Ser Phe Ala Glu Gly Gln Asp Gln Gly Ser Ala Tyr Ala Asn Arg
    50                  55                  60

Thr Ala Leu Phe Pro Asp Leu Leu Ala Gln Gly Asn Ala Ser Leu Arg
65                  70                  75                  80

Leu Gln Arg Val Arg Val Ala Asp Glu Gly Ser Phe Thr Cys Phe Val
                85                  90                  95

Ser Ile Arg Asp Phe Gly Ser Ala Ala Val Ser Leu Gln Val Ala Ala
            100                 105                 110

Pro Tyr Ser Lys Pro Ser Met Thr Leu Glu Pro Asn Lys Asp Leu Arg
        115                 120                 125

Pro Gly Asp Thr Val Thr Ile Thr Cys Ser Ser Tyr Arg Gly Tyr Pro
    130                 135                 140

Glu Ala Glu Val Phe Trp Gln Asp Gly Gln Gly Val Pro Leu Thr Gly
145                 150                 155                 160

Asn Val Thr Thr Ser Gln Met Ala Asn Glu Gln Gly Leu Phe Asp Val
                165                 170                 175

His Ser Val Leu Arg Val Val Leu Gly Ala Asn Gly Thr Tyr Ser Cys
                180                 185                 190

Leu Val Arg Asn Pro Val Leu Gln Gln Asp Ala His Gly Ser Val Thr
            195                 200                 205

Ile Thr Gly Gln Pro Met Thr Phe Pro Glu Gln Lys Leu Ile Ser Glu
    210                 215                 220

Glu Asp Leu His His His His His His
225                 230

<210>  95
<211>  452
<212>  PRT
<213>  Artificial sequence

<220>
<223>  hBRCA84D heavy chain

<400>  95

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Gly Met His Trp Val Arg Gln Ala Pro Gly Lys Gly Leu Glu Trp Val
        35                  40                  45

Ala Tyr Ile Ser Ser Asp Ser Ser Ala Ile Tyr Tyr Ala Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Asp Glu Asp Thr Ala Val Tyr Tyr Cys
                85                  90                  95

Gly Arg Gly Arg Glu Asn Ile Tyr Tyr Gly Ser Arg Leu Asp Tyr Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Arg Val Glu Pro Lys Ser
    210                 215                 220

Cys Asp Lys Thr His Thr Cys Pro Pro Cys Pro Ala Pro Glu Leu Val
225                 230                 235                 240

Gly Gly Pro Ser Val Phe Leu Leu Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Pro Glu Glu Gln Tyr Asn Ser Thr
    290                 295                 300

Leu Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Leu Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  96
<211>  214
<212>  PRT
<213>  Artificial sequence

<220>
<223>  hBRCA84D light chain

<400>  96

Asp Ile Gln Leu Thr Gln Ser Pro Ser Phe Leu Ser Ala Ser Val Gly
1               5                   10                  15

Asp Arg Val Thr Ile Thr Cys Lys Ala Ser Gln Asn Val Asp Thr Asn
            20                  25                  30

Val Ala Trp Tyr Gln Gln Lys Pro Gly Lys Ala Pro Lys Ala Leu Ile
        35                  40                  45

Tyr Ser Ala Ser Tyr Arg Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Asn Tyr Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210